# EUROPEAN PATENT SPECIFICATION

(11) **EP 3 271 468 B1**
(45) Date of publication and mention of the grant of the patent: **14.12.2022**
(21) Application number: 16712777.8
(22) Date of filing: 18.03.2016
(51) Int. Cl.: C12P 19/30, C12P 9/00, C12P 17/16, C07H 19/06, C07H 19/10, C07H 19/12

(54) **DEAMINATION OF ORGANOPHOSPHORUS-NUCLEOSIDES**
DESAMINIERUNG VON ORGANOPHOSPHOR-NUKLEOSIDEN
DÉSAMINATION DE NUCLÉOSIDES ORGANOPHOSPHORÉS

(30) Priority: 19.03.2015 EP 15382131; 07.08.2015 EP 15382419
(43) Date of publication of application: 24.01.2018
(73) Proprietor: Laboratorio Tecnico de Seguridad y Estandarizacion, SL, 08225 Terrassa, Barcelona (ES)
(72) Inventor: PÉREZ OZCÁRIZ, Sergio, 08100 Mollet Del Valles (ES); PASCUAL GILABERT, Marta, 08100 Mollet Del Vallès (ES); ALONSO FERNÁNDEZ, Javier, 08100 Mollet Del Vallès (ES); LÓPEZ GÓMEZ, Cristina, 08100 Mollet Del Vallès (ES); FERNÁNDEZ FERNÁNDEZ, Carmen María, 08100 Mollet Del Valles (ES); CASTELLS BOLIART, Josep, 08100 Mollet Del Valles (ES)
(74) Representative: Ponti & Partners, S.L.P
(86) International application number: PCT/EP2016/055939
(87) International publication number: WO 2016/146808

(56) References cited:
- WO-A2-2012/158811
- S. EMMANUELLE FAIVRE-NITSCHKE ET AL: "A prokaryotic-type cytidine deaminase from Arabidopsis thaliana . Gene expression and functional characterization", EUROPEAN JOURNAL OF BIOCHEMISTRY, vol. 263, no. 3, August 1999 (1999-08), pages 896-903, XP055243283, GB ISSN: 0014-2956, DOI: 10.1046/j.1432-1327.1999.00591.x cited in the application
- A VITA ET AL: "Cytidine deaminase from Escherichia coli B. Purification and enzymatic and molecular properties", BIOCHEMISTRY, vol. 24, no. 21, 8 October 1985 (1985-10-08), pages 6020-6024, XP055242599, US ISSN: 0006-2960 cited in the application
- TOHORU KATSURAGI ET AL: "Cytosine Deaminase from Escherichia coli-Production, Purification, and Some Characteristics", AGRIC. BIOL. CHEM,, vol. 50, no. 7, 1986, pages 1721-1730, XP001317698, cited in the application

## Description

### Field of the invention

The present invention relates to a novel enzymatic process for nucleoside deamination, in particular, for the deamination of cytidinic organophosphorus nucleoside analogues (NAs) bearing bulky substituents, as well as intermediates of the process.

### Background of the invention

Nucleoside analogues (NAs) are synthetic compounds structurally related to natural nucleosides. In terms of their structure, nucleosides are constituted by three key elements: (i) the hydroxymethyl group, (ii) the heterocyclic nitrogenous base moiety, and (iii) the furanose ring, which in several instances seems to act as a spacer presenting the hydroxymethyl group and the base in the correct orientation.

NAs are extensively used as antiviral and antitumor agents. These molecules have been traditionally synthesized by different chemical methods which often require time-consuming multistep processes including protection-deprotection reactions on the heterocycle base and/or the pentose moiety to allow the modification of naturally occurring nucleosides (Boryski J. 2008. Reactions of transglycosylation in the nucleoside chemistry. Curr Org Chem 12:309-325). This time consuming multistep processes often lead to low yields and increased costs. Indeed, chemical methods usually increase the difficulty of obtaining products with correct stereo- and regioselectivity, generating by-products as impurities (Condezo, L. A., et al. 2007. Enzymatic synthesis of modified nucleosides, p. 401-423. Biocatalysis in the pharmaceutical and biotechnology industries. CRC Press, Boca Raton, FL, Mikhailopulo, I. A. 2007; Sinisterra, J.V. et al. 2010. Enzyme-catalyzed synthesis of nonnatural or modified nucleosides, p. 1-25. Encyclopedia of Industrial Biotechnology: Bioprocess, Bioseparation, and Cell Technology, John Wiley & sons, Ed. By M. C. Flickinger, 2010). Moreover, the chemical methods include the use of chemical reagents and organic solvents that are expensive and environmentally harmful.

Therefore, enzymatic approaches have special interest because they can solve some of these problems. In particular, the deamination of amino-containing nucleosides is an interesting way to synthesize their corresponding keto-counterparts. Deamination reactions occurring in natural nucleosides, either ribo- or 2'-deoxyribonucleosides, take place at the nucleobase moiety, including cytosine, 5-methylcytosine, guanine and adenine nucleosides, that are transformed into their corresponding nucleoside analogues containing, respectively, uracil, thymine, xanthine and hypoxanthine as the nucleobases, and ammonia as by-product.

Although deaminase enzymes are broadly distributed, usually they are very specific for their corresponding substrates (Katsiragi, T. et al. 1986. Cytosine Deaminase from Escherichia coli - Production, Purification, and Some characteristics, Agric. Biol. Chem. 50(7), 1721-1730; Vita, A. et al. 1985. Cytidine Deaminase from Eschericia coli B. Purification and Enzymatic Molecular Properties, Biochemistry, 24, 6020-6024).

According to enzyme databases (http://www.brenda-enzymes.info/search_result.php?quicksearch=1&noOfResults=10&a=9&W[2]=deaminase&T[ 2]=2), among deaminating enzymes, only a short group is disclosed as being able to deaminate nucleobase containing substrates. According to this specificity, deaminases can be divided into: 1) nucleobase deaminases (such as cytosine deaminase, EC 3.5.4.1; adenine deaminase, EC 3.5.4.2; guanine deaminase, EC 3.5.4.3; 8-oxoguanine deaminase, EC 3.5.4.32; *i.e.* natural substrate are the nucleobases cytosine, adenine, guanine and 8-oxoguanine, respectively); 2) nucleoside deaminases (such as cytidine deaminase, EC 3.5.4.5; adenosine deaminase, EC 3.5.4.4; guanosine deaminase, EC 3.5.4.15; S-methyl-5'-thioadenosine deaminase, EC 3.5.4.31; 5'-deoxyadenosine deaminase, EC 3.5.4.41; *i.e.* natural substrate are the nucleosides cytidine, adenosine, guanosine, S-methyl-5'-thioadenosine and 5'-deoxyadenosine deaminase, respectively); and 3) nucleotide deaminases (such as 2'-deoxycytidine triphosphate deaminase, EC 3.5.4.13; 2'-deoxycytidine triphosphate deaminase (dUMP forming), EC 3.5.4.30; adenosine monophosphate deaminase, EC 3.5.4.6; adenosine diphosphate deaminase, EC 3.5.4.7; adenosin-phosphate deaminase, EC 3.5.4.17; adenosine triphosphate deaminase, EC 3.5.4.18; *i.e.* natural substrate are the nucleotides 2'-deoxycytidine triphosphate, adenosine monophosphate, adenosine diphosphate and adenosine triphosphate, respectively).

Accordingly, nucleoside deaminases are able to deaminate nucleosides but not nucleotides, whereas nucleotide deaminases are able to deaminate nucleotides but not nucleosides.

Taking the structure of the substrates into consideration, organophosphorus nucleosides, *i.e.* those nucleosides bearing a substituted phosphor atom connected to the oxygen at nucleosidic position C-5', such as organic phosphates, phosphinates, phosphonates, phosphoramidates, and the like, should exhibit a substrate behavior and specificity similar to natural nucleotides (*i.e.* a nucleoside bearing at least one PO4²⁻group and the like). Therefore, for those skilled in the art, the enzymes of choice for catalyzing their corresponding deamination would be nucleotide deaminases.

Furthermore, the deamination of certain nucleosidic substrates incorporating bulky substituents remains an unresolved problem because of their difficult fitting into the active site of the enzymes. In particular, those NA containing mono-, di- or triphosphate groups bounded to the sugar ring are known to usually act as inhibitors of these enzymes (Faivre-Nitschke, S.E. et al. 1999, A prokaryotic-type cytidine deaminase from Arabidopsis thaliana, Eur. J. Biochem. 263, 896-903).

WO 2012/158811 A2 disclose a deaminase assay for nucleosides and monophosphate prodrugs performed by adenosine deaminase, using commercially available purified enzymes under analytical conditions not suitable for synthetic preparative industrial purposes. Authors disclose a 59% deamination yield for deoxyadenosine, the natural substrate of adenosine deaminase, *i.e.* a natural nucleoside without any substitution at position C-5', therefore, without bulky substitution in there. No reference or data are made to the deamination of any of the purine monophosphate compounds disclosed therein.

Surprisingly, it was found that the drawbacks of previous cited biocatalytic synthesis on organophosphorus cytidine nucleosides can be avoided by applying an enzymatic method based on the use of nucleoside deaminase enzymes, wherein said nucleoside deaminase is a cytidine deaminase enzyme. The referred enzymes, surprisingly, can recognize proper modified phosphor-containing cytidine nucleoside analogs substrates and are able to perform the deamination reaction in spite of bearing bulky substituents at position C-5'. The inventors have demonstrated that the same biocatalytic reaction but using cytidine nucleotides as substrates does not render the corresponding deaminated product.

Therefore, as shown below, the present invention contributes to a highly efficient synthesis and production method of such compounds of formula I, by means of a biocatalytic deamination of compounds of formula II.

### Description of the invention

The present invention relates to a process for preparing a compound of formula I according to the following reaction catalyzed by a nucleoside deaminase, wherein said nucleoside deaminase is a cytidine deaminase, (hereinafter, simply referred as Reaction II-I) 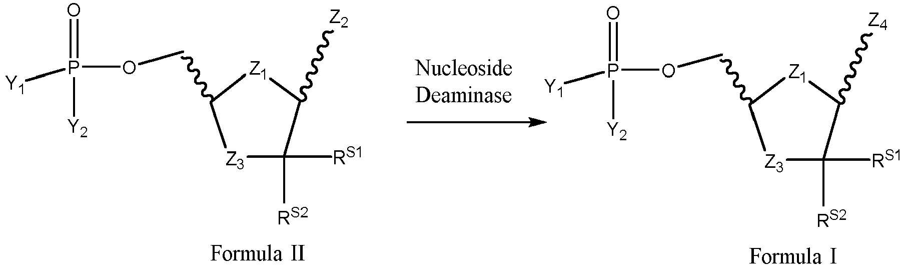 wherein
Z₁ is O;
Z₂ is selected from 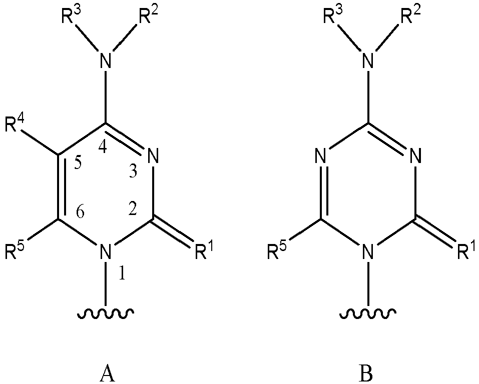
Z3 is selected, independently of Z₁ from O, C(R^{S3}R^{S4}); Z₄ is selected from 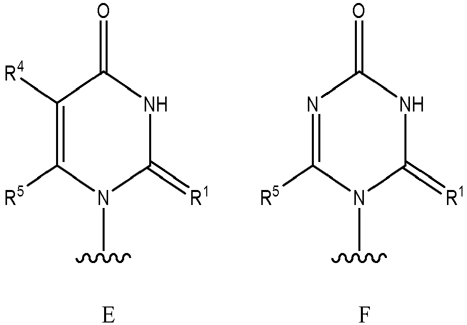
R¹ is selected from O, CH₂, alkyl, S and NH;
R² is hydrogen;
R³ is hydrogen;
R⁴ is selected from hydrogen; OH; NH₂; SH; halogen, preferably F, Cl or I; methyl; an optionally substituted alkyl chain; an optionally substituted alkenyl chain; an optionally substituted alkynyl chain; trihaloalkyl; OR⁶; NR⁶R⁷; CN; COR⁶; CONR⁶R⁷; CO₂R⁶; C(S)OR⁶; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; SO₂NR⁶R⁷; an optionally substituted aryl linked to C-5 by an optionally substituted alkyl, alkenyl or alkynyl chain; and an optionally substituted heterocycle linked to C-5 by an optionally substituted alkyl, alkenyl or alkynyl chain;
R⁵ is selected from hydrogen; OH; NH₂; SH; halogen, preferably F, Cl or I; methyl; an optionally substituted alkyl chain; an optionally substituted alkenyl chain; an optionally substituted alkynyl chain; trihaloalkyl; OR⁶; NR⁶R⁷; CN; COR⁶; CONR⁶R⁷; CO₂R⁶; C(S)OR⁶; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; SO₂NR⁶R⁷; an optionally substituted aryl linked to C-6 by an optionally substituted alkyl, alkenyl or alkynyl chain; and an optionally substituted heterocycle linked to C-6 by an optionally substituted alkyl, alkenyl or alkynyl chain;
R⁶ and R⁷ are selected, independently of each other, from hydrogen; an optionally substituted alkyl chain; an optionally substituted alkenyl chain; an optionally substituted alkynyl chain; an optionally substituted heterocycle; and an optionally substituted aryl, preferably phenyl or naphtyl;
R^{S1} is selected, independently of R^{S2}, from hydrogen; halogen, preferably F; methyl; OH; NH₂; SH; N₃; an optionally substituted alkyl chain; an optionally substituted alkenyl chain; an optionally substituted alkynyl chain; trihaloalkyl, OR⁶; NR⁶R⁷; CN; COR⁶; CONR⁶R⁷; CO₂R⁶; C(S)OR⁶; OCONR⁶R⁷; OCOR⁶; OCO₂R⁶; OSO₂R⁶; OC(S)OR⁶; O-Ketal; O-Si-alkyl; O-Si-aryl; NHCONR⁶R⁷; NHCOR⁶; NR⁶CO₂R⁷; NHCO₂R⁶; NHC(S)OR⁶; SO₂NR⁶R⁷; an optionally substituted aryl linked to C-2' by an optionally substituted alkyl, alkenyl or alkynyl chain; and an optionally substituted heterocycle linked to C-2' by an optionally substituted alkyl, alkenyl or alkynyl chain;
R^{S2} is selected, independently of R^{S1}, from hydrogen; halogen, preferably F; methyl; OH; NH₂; SH; N₃; an optionally substituted alkyl chain; an optionally substituted alkenyl chain; an optionally substituted alkynyl chain; trihaloalkyl; OR⁶; NR⁶R⁷; CN; COR⁶; CONR⁶R⁷; CO₂R⁶; C(S)OR⁶; OCONR⁶R⁷; OCOR⁶; OCO₂R⁶; OSO₂R⁶; OC(S)OR⁶; O-Ketal; O-Si-alkyl; O-Si-aryl; NHCONR⁶R⁷; NHCOR⁶; NR⁶CO₂R⁷; NHCO₂R⁶; NHC(S)OR⁶; SO₂NR⁶R⁷; an optionally substituted aryl linked to C-2' by an optionally substituted alkyl, alkenyl or alkynyl chain; and an optionally substituted heterocycle linked to C-2' by an optionally substituted alkyl, alkenyl or alkynyl chain;
R^{S3} is selected, independently of R^{S4}, from hydrogen; OH; halogen, preferably F; methyl; CN; NH₂; SH; C≡CH; N₃; an optionally substituted alkyl chain; an optionally substituted alkenyl chain; an optionally substituted alkynyl chain; and an optionally substituted aryl; an optionally substituted heterocycle; OR⁶; OCONR⁶R⁷; OCOR⁶; OCO₂R⁶; OSO₂R⁶; OC(S)OR⁶; O-Ketal; O-Si-alkyl; and O-Si-aryl;
R^{S4} is selected, independently of R^{S3}, from hydrogen; OH; halogen, preferably F; methyl; CN; NH₂; SH; C≡CH; N₃; an optionally substituted alkyl chain; an optionally substituted alkenyl chain; an optionally substituted alkynyl chain; an optionally substituted aryl; an optionally substituted heterocycle; OR⁶; OCONR⁶R⁷; OCOR⁶; OCO₂R⁶; OSO₂R⁶; OC(S)OR⁶; O-Ketal; O-Si-alkyl; and O-Si-aryl;
Y₁ is selected, independently of Y₂, from OR⁸; NR⁶R⁷; COR⁶; CONR⁶R⁷; CO₂R⁶; C(S)OR⁶; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; SO₂NR⁶R⁷; an optionally substituted alkyl chain; an optionally substituted alkenyl chain; an optionally substituted alkynyl chain; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; an optionally substituted heterocycle optionally linked to P through O or N atoms; an ether of an optionally substituted alkyl chain; an ether of an optionally substituted alkenyl chain; an ether of an optionally substituted alkynyl chain; an ether of an optionally substituted aryl, preferably *O*-phenyl or *O*-naphtyl; an ether of an optionally substituted heterocycle; and an amino acid, preferably alanine, valine, leucine or isoleucine, either in the free form or protected by a suitable functional group;
Y₂ is selected, independently of Y₁, from OR⁸; NR⁶R⁷; COR⁶; CONR⁶R⁷; CO₂R⁶; C(S)OR⁶; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; SO₂NR⁶R⁷; an optionally substituted alkyl chain; an optionally substituted alkenyl chain; an optionally substituted alkynyl chain; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; an optionally substituted heterocycle optionally linked to P through O or N atoms; an ether of an optionally substituted alkyl chain; an ether of an optionally substituted alkenyl chain; an ether of an optionally substituted alkynyl chain; an ether of an optionally substituted aryl, preferably *O*-phenyl or *O-*naphtyl; an ether of an optionally substituted heterocycle; an amino acid, preferably alanine, valine, leucine or isoleucine, either in the free form or protected by a suitable functional group; R⁸ is selected from an optionally substituted alkyl chain; an optionally substituted alkenyl chain; an optionally substituted alkynyl chain; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; and an optionally substituted heterocycle optionally linked to P through O or N atoms; aryl, preferably phenyl or naphthyl;
wherein when Z₂ is A, Z₄ is E; and when Z₂ is B, Z₄ is F.

In the context of the present invention, when reference is made to a compound of formula I or a compound of formula II, the pharmaceutically acceptable salts thereof are also included.

Applicants have surprisingly found that cytosine containing organophosphorus-nucleoside analogues, represented by formula II, are recognized as substrates by cytidine deaminases at a conversion rate and yields equivalent to their natural substrates, i.e. nucleoside analogues, instead of being recognized as nucleotide analogues, which are, in fact, non-reactive under the same reaction conditions.

Accordingly, cytosine containing organophosphorus-nucleoside analogues described herein allow the preparation/production of uridinic nucleoside analogues at high conversions and yields (more than 70%, usually quantitative, *i.e.* 99-100%).

No evidences have been found so far pointing at the fact that this sort of bulky chemical modification at position C-5' in cytidine derivatives chemical backbone, would have been able to modify the substrate specificity for cytidine deaminase.

In the context of the present invention, the term uridine or uridinic derivatives, nucleosides, intermediates, they all should be understood as chemical compounds derived from uridine backbone. In particular, the uridine or uridinic derivatives are uridine containing organophosphorus-nucleoside analogues, represented by formula I.

In the context of the present invention, the term cytidine or cytidinic derivatives, nucleosides, intermediates, they all should be understood as chemical compounds derived from cytidine backbone. In particular, cytidine or cytidinic derivatives are cytosine containing organophosphorus-nucleoside analogues, represented by formula II.

In a preferred embodiment for the process for preparing a compound of formula I as defined above in Reaction II-I:
Z₁ is O;
Z₃ is selected, independently of Z₁, from O and C(R^{S3}R^{S4}), more preferably C(R^{S3}R^{S4});
Z₂ is selected from 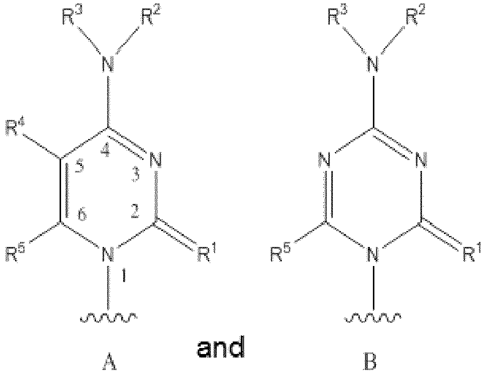
Z₄ is selected from 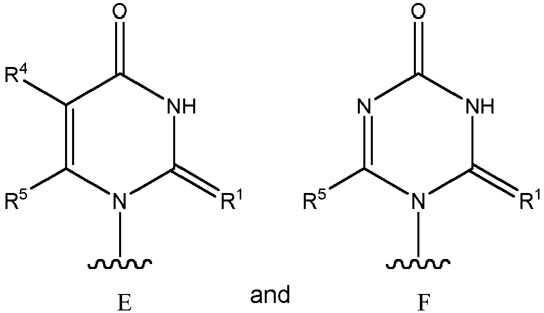
R1 is O;
R² is H;
R³ is H;
R⁴ is selected from H; OH; halogen, preferably F, Cl or I, more preferably F; methyl; trihaloalkyl; OR⁶; COR⁶; CONR⁶R⁷; CO₂R⁶; OCONR⁶R⁷; OCOR⁶; and OCO₂R⁶;
R⁵ is selected from H; OH; halogen, preferably F, Cl or I, more preferably F; methyl; trihaloalkyl; OR⁶; COR⁶; CONR⁶R⁷; CO₂R⁶; OCONR⁶R⁷; OCOR⁶; and OCO₂R⁶;
R^{S1} is selected, independently of R^{S2}, from hydrogen; halogen, preferably F, methyl; OH; OR⁶; NR⁶R⁷; OCONR⁶R⁷; OCOR⁶; OCO₂R⁶; OSO₂R⁶; OC(S)OR⁶; O-Ketal; O-Si-alkyl; and O-Si-aryl; R^{S2} is selected, independently of R^{S1}, from hydrogen; halogen, preferably F, methyl; OH; OR⁶; NR⁶R⁷; OCONR⁶R⁷; OCOR⁶; OCO₂R⁶; OSO₂R⁶; OC(S)OR⁶; O-Ketal; O-Si-alkyl; and O-Si-aryl; R^{S3} is selected, independently of R^{S4}, from hydrogen; methyl; OH; OR⁶; OCONR⁶R⁷; OCOR⁶; OCO₂R⁶; OSO₂R⁶; OC(S)OR⁶; O-Ketal; O-Si-alkyl; O-Si-aryl; and halogen, preferably F;
R^{S4} is selected, independently of R^{S3}, from hydrogen; methyl; OH; OR⁶; OCONR⁶R⁷; OCOR⁶; OCO₂R⁶; OSO₂R⁶; OC(S)OR⁶; O-Ketal; O-Si-alkyl; O-Si-aryl; and halogen, preferably F;
Y₁ is selected, independently of Y₂, from OR⁸; NR⁶R⁷; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; an optionally substituted alkyl chain; an optionally substituted alkenyl chain; an optionally substituted alkynyl chain; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; an optionally substituted heterocycle optionally linked to P through O or N atoms; an ether of an optionally substituted alkyl chain; an ether of an optionally substituted alkenyl chain; an ether of an optionally substituted alkynyl chain; an ether of an optionally substituted aryl, preferably *O*-phenyl or *O*-naphtyl; an ether of an optionally substituted heterocycle; and an amino acid, preferably alanine, valine, leucine or isoleucine, either in the free form or protected by a suitable functional group;
Y₂ is selected, independently of Y₁, from OR⁸; NR⁶R⁷; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; an optionally substituted alkyl chain; an optionally substituted alkenyl chain; an optionally substituted alkynyl chain; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; an optionally substituted heterocycle optionally linked to P through O or N atoms; an ether of an optionally substituted alkyl chain; an ether of an optionally substituted alkenyl chain; an ether of an optionally substituted alkynyl chain; an ether of an optionally substituted aryl, preferably *O*-phenyl or *O*-naphtyl; an ether of an optionally substituted heterocycle; and an amino acid, preferably alanine, valine, leucine or isoleucine, either in the free form or protected by a suitable functional group;
R⁸ is selected from an optionally substituted alkyl chain; an optionally substituted alkenyl chain; an optionally substituted alkynyl chain; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; an optionally substituted heterocycle optionally linked to P through O or N atoms; and aryl, preferably phenyl and naphthyl;
wherein when Z₂ is A, Z₄ is E; and when Z₂ is B, Z₄ is F.

In a more preferred embodiment for the process for preparing a compound of formula I as defined above in Reaction II-I:
Z₁ is O;
Z₃ is C(R^{S3}R^{S4});
Z₂ is selected from 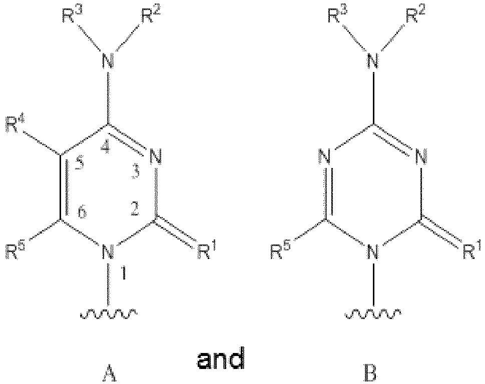
Z₄ is selected from 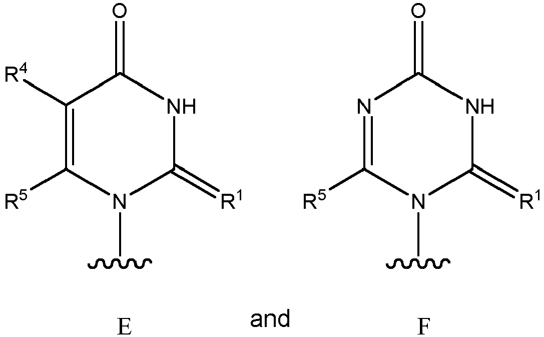
R¹ is O;
R² is H;
R³ is H;
R⁴ is selected from H; OH; halogen, preferably F; methyl and trihaloalkyl;
R⁵ is selected from H; OH; halogen; OR⁶; COR⁶; CONR⁶R⁷; CO₂R⁶; OCONR⁶R⁷; OCOR⁶; and OCO₂R⁶;
R^{S1} is selected, independently of R^{S2}, from hydrogen; halogen, preferably F; methyl; OH; OR⁶; OCONR⁶R⁷; OCOR⁶; OSO₂R⁶; O-Ketal; O-Si-alkyl; O-Si-aryl; and OCO₂R⁶;
R^{S2} is selected, independently of R^{S1}, from hydrogen; halogen preferably F; methyl; OH; OR⁶; OCONR⁶R⁷; OCOR⁶; OSO₂R⁶; O-Ketal; O-Si-alkyl; O-Si-aryl; and OCO₂R⁶;
R^{S3} is selected, independently of R^{S4}, from hydrogen; methyl; OH; OR⁶; OCONR⁶R⁷; OCOR⁶; OCO₂R⁶; OC(S)OR⁶; OSO₂R⁶; O-Ketal; O-Si-alkyl; O-Si-aryl; and halogen, preferably F;
R^{S4} is selected, independently of R^{S3}, from hydrogen; methyl; OH; OR⁶; OCONR⁶R⁷; OCOR⁶; OCO₂R⁶; OC(S)OR⁶; OSO₂R⁶; O-Ketal; O-Si-alkyl; O-Si-aryl;and halogen, preferably F;
Y₁ is selected, independently of Y₂, from OR⁸; NR⁶R⁷; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; an optionally substituted alkyl chain; an optionally substituted alkenyl chain; an optionally substituted alkynyl chain; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; an optionally substituted heterocycle optionally linked to P through O or N atoms; an ether of an optionally substituted alkyl chain; an ether of an optionally substituted alkenyl chain; an ether of an optionally substituted alkynyl chain; an ether of an optionally substituted aryl, preferably *O*-phenyl or *O-*naphtyl; an ether of an optionally substituted heterocycle; and an amino acid, preferably alanine, valine, leucine or isoleucine, either in the free form or protected by a suitable functional group;
Y₂ is selected, independently of Y₁, from OR⁸; NR⁶R⁷; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; an optionally substituted alkyl chain; an optionally substituted alkenyl chain; an optionally substituted alkynyl chain; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; an optionally substituted heterocycle optionally linked to P through O or N atoms; an ether of an optionally substituted alkyl chain; an ether of an optionally substituted alkenyl chain; an ether of an optionally substituted alkynyl chain; an ether of an optionally substituted aryl, preferably O-phenyl or O-naphtyl; an ether of an optionally substituted heterocycle; and an amino acid, preferably alanine, valine, leucine or isoleucine, either in the free form or protected by a suitable functional group;
R⁸ is selected from an optionally substituted alkyl chain; an optionally substituted alkenyl chain; an optionally substituted alkynyl chain; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; an optionally substituted heterocycle optionally linked to P through O or N atoms; and aryl, preferably phenyl and naphthyl;
wherein when Z₂ is A, Z₄ is E; and when Z₂ is B, Z₄ is F.

In an even more preferred embodiment for the process for preparing a compound of formula I as defined above in Reaction II-I:
Z₁ is O;
Z₃ is C(R^{S3}R^{S4}) wherein R^{S3} is H or OH and wherein R^{S4} is, independently of R^{S3}, H or OH;
Z₂ is selected from 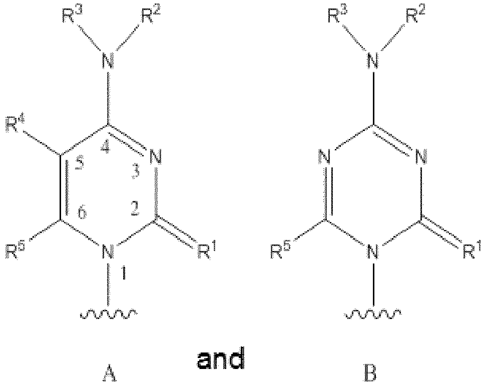
Z₄ is selected from 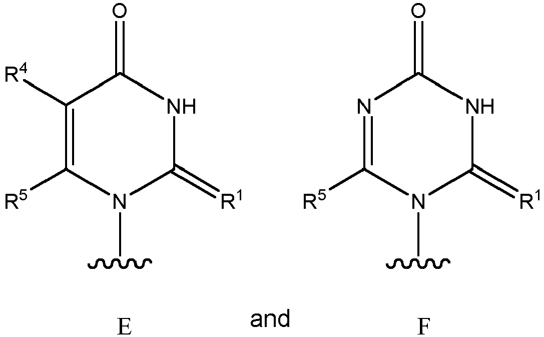
R1 is O;
R² is H;
R³ is H;
R⁴ is H, methyl or halogen, preferably F;
R⁵ is H;
R^{S1} is selected, independently of R^{S2}, from hydrogen; halogen, preferably F; methyl; and OH; R^{S2} is selected, independently of R^{S1}, from hydrogen; halogen, preferably F; methyl; and OH; Y₁ is selected, independently of Y₂, from OR⁸; an ether of an optionally substituted aryl, preferably O-phenyl or O-naphtyl; an ether of an optionally substituted heterocycle; and an amino acid, preferably alanine, valine, leucine or isoleucine, either in the free form or protected by a suitable functional group;
Y₂ is selected, independently of Y₁, from OR⁸; NR⁶R⁷; NHCONR⁶R⁷; NHCOR⁶; NR⁶CO₂R⁷; NHCO₂R⁶; NHC(S)OR⁶; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; an optionally substituted heterocycle optionally linked to P through O or N atoms; an ether of an optionally substituted alkyl chain; an ether of an optionally substituted alkenyl chain; an ether of an optionally substituted alkynyl chain; an ether of an optionally substituted aryl, preferably *O-*phenyl or O-naphtyl; an ether of an optionally substituted heterocycle; and an amino acid, preferably alanine, valine, leucine or isoleucine, either in the free form or protected by a suitable functional group;
R⁸ is selected from an optionally substituted alkyl chain; an optionally substituted alkenyl chain; an optionally substituted alkynyl chain; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; an optionally substituted heterocycle optionally linked to P through O or N atoms; and aryl, preferably phenyl and naphthyl;
wherein when Z₂ is A, Z₄ is E; and when Z₂ is B, Z₄ is F.

In an even more preferred embodiment for the process for preparing a compound of formula I as defined above in Reaction II-I,
Z₁ is O;
Z₃ is C(R^{S3}R^{S4}) wherein R^{S3} is H or OH and wherein R^{S4} is, independently of R^{S3}, H or OH;
Z₂ is selected from 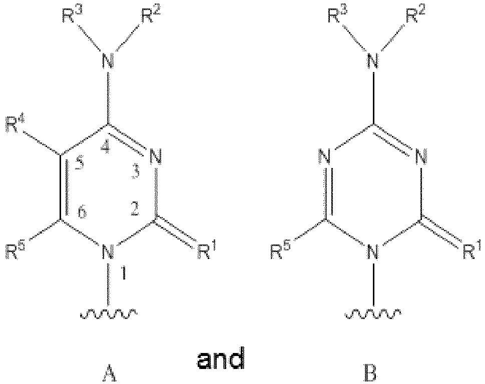
Z₄ is selected from 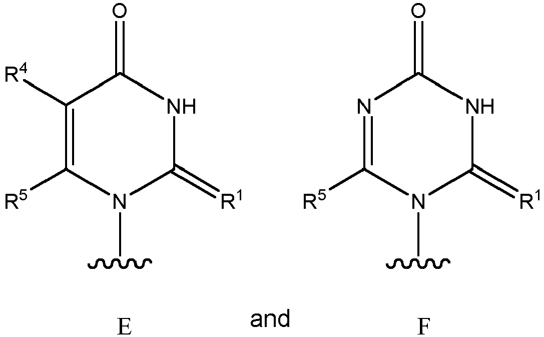
R¹ is O;
R² is H;
R³ is H;
R⁴ is H;
R⁵ is H;
R^{S1} is selected, independently of R^{S2}, from hydrogen; halogen, preferably F; methyl; and OH; R^{S2} is selected, independently of R^{S1}, from hydrogen; halogen, preferably F; methyl; and OH; Y₁ is selected, independently of Y₂, from OR⁸; an ether of an optionally substituted aryl, preferably *O*-phenyl or *O*-naphtyl; an ether of an optionally substituted heterocycle; and an amino acid, preferably alanine, valine, leucine or isoleucine, either in the free form or protected by a suitable functional group;
more preferably Y₁ is selected, independently of Y₂, from an ether of an optionally substituted aryl, preferably *O*-phenyl; and an amino acid, preferably alanine, valine, leucine or isoleucine, either in the free form or protected by a suitable functional group;
Y₂ is selected, independently of Y₁, from an ether of an optionally substituted aryl, preferably *O*-phenyl or *O*-naphtyl; and an amino acid, preferably alanine, valine, leucine or isoleucine, either in the free form or protected by a suitable functional group;
more preferably Y₂ is selected, independently of Y₁, from an ether of an optionally substituted aryl, preferably *O*-phenyl; and an amino acid, preferably alanine, valine, leucine or isoleucine, either in the free form or protected by a suitable functional group;
R⁸ is selected from an optionally substituted alkyl chain; an optionally substituted alkenyl chain; an optionally substituted alkynyl chain; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; an optionally substituted heterocycle optionally linked to P through O or N atoms; and aryl, preferably phenyl and naphthyl;
wherein when Z₂ is A, Z₄ is E; and when Z₂ is B, Z₄ is F.

The suitable chemical modifications that are the object of the present invention provide a more convenient, efficient and easier process for the one-step production of uridine organophosphorus nucleoside analogues bearing bulky substituent groups at position C-5' from their corresponding cytidine counterparts, that fully avoids the inhibition problems disclosed in the prior art. Particularly, preferred phosphor substitution is in the form of phosphoramidate derivatives, more preferably phosphoramidate groups including an aromatic group such as phenyl and an amino acid such as alanine, preferably protected at the carbon in the terminal end as isopropyl ester, being this then a suitable functional group for protection. Hence, the invention provides improved alternative synthesis methods of nucleoside analogues, useful as anticancer and/or antiviral products, by shortening conventional multistep synthesis, increasing overall yield, reducing side reactions and by-product content and, therefore, improving product purity and quality.

For the purposes of present description, the following terms are further defined as follows.

The term "cytidine deaminase" refers to any protein showing cytidine deaminase activity and accordingly, it includes any catalytic presentation of this protein, either in the form of purified protein or in the form of an extract with any formulation additive. This protein can be a naturally occurring enzyme, such as the cytidine deaminase present, but not limited thereto, in *Arabidopsis thaliana, Bacillus caldolyticus, Bacillus cereus, Bacillus subtilis, Bos taurus, Brugia pahangi, Caenorhabditis elegans, Canis lupus, Cavia porcellus, Columba* spp., any genus of the Cricetinae family, *Crithidia fasciculata, Escherichia coli, Felis catus, Gallus gallus, Geobacillus stearothermophilus, Haemophilus influenzae, Haliotis deversicolor, Homo sapiens, Macaca mulatta, Mus musculus, Mycobacterium tuberculosis, Mycoplasma pneumoniae, Nocardioides* spp., *Oryctolagus cuniculus, Ovis aries, Penicillium palitans, Rana* spp., *Rattus norvegicus, Saccharomyces cerevisiae, Salmonella enterica, Sporosarcina psychrophila, Sus scrofa, Trypanosoma cruzi, Zea mays,* extracted from its natural source or obtained by recombination techniques, as well as any mutation or recombination of these proteins that displays cytidine deaminase activity.

The term "activity unit (AU)" refers to the amount of enzyme capable to convert 1 µmol of substrate into product per minute under standard controlled conditions.

The term "nucleoside" refers to all compounds in which a heterocyclic base is covalently coupled to a sugar, and especially preferred coupling of the nucleoside to the sugar includes a C1'-(glycosidic) bond of a carbon atom in a sugar to a carbon- or heteroatom (typically nitrogen) in the heterocyclic base. Therefore, in the present context the term "nucleoside" means the glycoside of a heterocyclic base.

The term "nucleoside" used herein is used broadly as to include, naturally occurring nucleosides and non-naturally occurring nucleosides. Illustrative examples of nucleosides are ribonucleosides comprising a ribose moiety as well as deoxyribonucleosides comprising a deoxyribose moiety. With respect to the bases of such nucleosides, it should be understood that this may be any of the naturally occurring bases, e.g. adenine, guanine, cytosine, thymine, and uracil, as well as any modified variants thereof or any possible unnatural bases.

The term "nucleoside analogue", "nucleoside analog", "NA" or "NAs" as used herein refers to all nucleosides in which at least one atom of the structure is different from those present in natural nucleosides (*i.e.,* adenosine, cytidine, uridine, thymidine, inosine, guanosine, among others).

The term "organophosphorus nucleoside" refers to those nucleosides bearing a substituted phosphor atom connected to the oxygen at position C-5' and represented as compounds of formula I and compounds of formula II. The organophosphorus nucleoside analogues describe herein are intended to include, but not limited to organic phosphates, phosphinates, phosphonates, phosphoramidates, and the like, but excluding nucleotides (*i.e.* compounds wherein the substitution at OH-5' is either mono-, di- or triphosphate).

For the purposes of the present invention, the term "bulky" when referring to substituents at position C-5', means any group containing a higher number of atoms and/or a larger accessible surface area than that corresponding to a monophosphate PO₄²⁻ group.

The term "nucleotide" refers to a nucleoside wherein at least one phosphate group is coupled to the sugar through oxygen at C-5' position. Natural nucleotides bear one, two or three phosphate groups.

As further used herein, the term "sugar" refers to all carbohydrates and derivatives thereof, wherein particularly contemplated derivatives include deletion, substitution or addition or a chemical group or atom in the sugar. For example, especially contemplated deletions include 2'-deoxy, 3'-deoxy, 5'-deoxy and/or 2',3'-dideoxy-sugars. Especially contemplated substitutions include replacement of the ring-oxygen with sulphur or methylene, or replacement of a hydroxyl group with a halogen, azido, amino-, cyano, sulfhydryl-, or methyl group, and especially contemplated additions include methylene phosphonate groups. Further contemplated sugars also include sugar analogues (i.e., not naturally occurring sugars), and particularly carbocyclic ring systems. The term "carbocyclic ring system" as used herein refers to any molecule in which a plurality or carbon atoms form a ring, and in especially contemplated carbocyclic ring systems the ring is formed from 3, 4, 5, or 6 carbon atoms.

The term "enzymatic synthesis" refers to a method of synthesis of chemical compounds by means of a process which only comprises biocatalytic steps, carried out by the appropriate enzyme. Accordingly, other preferred embodiment of the synthesis process described herein is a full biocatalytic process which departs from cytosine derivatives, as the ones previously mentioned as represented by general formula II, already prepared or available in the market as cytosine derivatives as such.

The term "chemo-enzymatic synthesis" refers to a method of synthesis of chemical compounds through a combination of chemical and biocatalytic steps.

For the purposes of the present application, the term "process" is intented to include both enzymatic and chemo-enzymatic synthesis, i.e. wherein at least one of the steps in the process employs an enzyme.

The terms "heterocyclic ring" or "heterocyclic base" or "base" or "nucleobase" are used interchangeably herein and refer to any compound in which plurality of atoms form a ring via a plurality of covalent bonds, wherein the ring includes at least one atom other than a carbon atom. Particularly contemplated heterocyclic bases include 5- and 6-membered rings containing at least 1 to 4 heteroatoms each independently selected from nitrogen, oxygen and sulphur as the non-carbon atom (e.g., imidazole, pyrrole, triazole, dihydropyrimidine). Further contemplated heterocycles may be fused (i.e., covalently bound) to another ring or heterocycle, and are thus termed "fused heterocycle" or "fused heterocyclic base" as used herein. Especially contemplated fused heterocycles include a 5-membered ring fused to a 6-membered ring (e.g., purine, pyrrolo[2,3-*d*]pyrimidine), and a 6-membered ring fused to another 6-membered or higher ring (e.g., pyrido[4,5-*d*]pyrimidine, benzodiazepine). Still further contemplated heterocyclic bases may be aromatic, or may include one or more double or triple bonds. Moreover, contemplated heterocyclic bases and fused heterocycles may further be substituted in one or more positions. And any one of the rings being optionally substituted with one, two or three substituents each independently selected from the group consisting of halogen, hydroxy, nitro, cyano, carboxyl, C₁₋₆alkyl, C₁₋₆alkoxy, C₁₋₆alkoxyC₁₋₆alkyl, C₁₋₆alkylcarbonyl, amino, mono- or diC₁₋₆alkylamino, azido, mercapto, polyhaloC₁₋₆alkyl, polyhaloC₁₋₆alkoxy, and C₃₋₇cycloalkyl.

The term "nucleobase" covers naturally occurring nucleobases as well as non-naturally occurring nucleobases. It should be clear to the person skilled in the art that various nucleobases which previously have been considered "non-naturally occurring" have subsequently found in nature. Thus, "nucleobase" includes not only the known purine and pyrimidine heterocycles, but also heterocyclic analogues (such as *N*-substituted heterocycles) and tautomers thereof. Illustrative examples of nucleobases are adenine, guanine, thymine, cytosine, uracil, purine, xanthine, 2-chloroadenine, 2-fluoroadenine, pentyl (5-fluoro-2-oxo-1,2, dihydropyrimidin-4-yl)carbamate, cytosine *N*-alkyl carbamates, cytosine *N*-alkylesters, 5-azacytosine, 5-bromovinyluracil, 5-fluorouracil, 5-trifluromethyluracil, 6-methoxy-9*H*-purin-2-amine and (*R*)-3,6,7,8-tetrahydroimidazo[4,5-d][1,3]diazepin-8-ol.

The term "nucleobase" is intended to cover every and all of these examples as well as analogues and tautomers, and regioisomers thereof. In order to differentiate these "nucleobases" from other heterocyclic bases also present in this specification, for the purposes of present specification, the term "nucleobase" mainly refers to cytosinic bases represented as Z₂ in formula II and as uridinic bases represented by Z₄ in formula I.

The term "tautomer" or "tautomeric form" refers to structural isomer of different energies which are interconvertible via a low energy barrier. For example, proton tautomers (also known as prototropic tautomers) include interconversion via migration of a proton, such as keto-enol and imine-enamine isomerizations. Valence tautomers include interconversions by reorganization of some of the bonding electrons.

The term "regioisomer" refers to structural isomer, or constitutional isomer in the sense that refers to molecules with the same molecular formula that whose atoms are bonded in different order of connectivity.

The term "conversion" refers to is the percentage of starting material that is transformed into products, either the expected final product, byproducts, or even into products of degradation.

The term "yield" is the number of synthesized molecules of product per number of starting molecules. In a multistep synthesis, the yield can be calculated by multiplication of the yields of all the single steps.

The term "anomeric purity" refers to the amount of a particular anomer of a compound divided by the total amount of all anomers of that compound present in the mixture multiplied by 100.

The term "intermediate" or "intermediates" refer to any nucleoside analogue type compounds which may be transformed into the final product, the final product being preferably an active pharmaceutical ingredient (API) of nucleosidic structure, by means of suitable additional chemical reactions. Therefore, intermediates are molecules that may be considered as API precursors. The compounds of the present invention can also be considered as intermediate compounds and as such are also included in the scope of the present invention.

An *in vivo* hydrolysable ester of a compound of the formula I containing a hydroxy group includes inorganic esters such as phosphate esters and α-acyloxyalkyl ethers and related compounds which as a result of the *in vivo* hydrolysis of the ester breakdown to give the parent hydroxy group. Examples of α-acyloxyalkyl ethers include acetoxymethoxy and 2,2-dimethylpropionyloxy-methoxy. A selection of *in vivo* hydrolysable ester forming groups for hydroxy include alkanoyl, benzoyl, phenylacetyl and substituted benzoyl and phenylacetyl, alkoxycarbonyl (to give alkyl carbonate esters), dialkylcarbamoyl and N-(dialkylaminoethyl)-N-alkylcarbamoyl (to give carbamates), dialkylaminoacetyl and carboxyacetyl. Examples of substituents on benzoyl include morpholino and piperazino linked from a ring nitrogen atom via a methylene group to the 3- or 4-position of the benzoyl ring.

For therapeutic use, salts of either the compounds of formula I or the compounds of formula II are those wherein the counter-ion is pharmaceutically acceptable. However, salts of acids and bases which are non-pharmaceutically acceptable may also find use, for example, in the preparation or purification of a pharmaceutically acceptable compound. All salts, whether pharmaceutically acceptable or not are included within the scope of the present invention.

The pharmaceutically acceptable acid and base addition salts as mentioned above are meant to comprise the therapeutically active non-toxic acid and base addition salt forms which either the compounds of formula I or the compounds of formula II are able to form. The pharmaceutically acceptable acid addition salts can conveniently be obtained by treating the base form with such appropriate acid. Appropriate acids comprise, for example, inorganic acids such as hydrohalic acids, e.g. hydrochloric or hydrobromic acid, sulfuric, nitric, phosphoric and the like acids; or organic acids such as, for example, acetic, propanoic, hydroxyacetic, lactic, pyruvic, oxalic (i.e. ethanedioic), malonic, succinic (i.e. butanedioic acid), maleic, fumaric, malic (i.e. hydroxybutanedioic acid), tartaric, citric, methanesulfonic, ethanesulfonic, benzenesulfonic, p-toluenesulfonic, cyclamic, salicylic, p-aminosalicylic, pamoic and the like acids.

Conversely said salt forms can be converted by treatment with an appropriate base into the free base form.

Either the compounds of formula I or the compounds of formula II containing an acidic proton may also be converted into their non-toxic metal or amine addition salt forms by treatment with appropriate organic and inorganic bases. Appropriate base salt forms comprise, for example, the ammonium salts, the alkali and earth alkaline metal salts, e.g. the lithium, sodium, potassium, magnesium, calcium salts and the like, salts with organic bases, e.g. the benzathine, N-methyl-D-glucamine, hydrabamine salts, and salts with amino acids such as, for example, arginine, lysine and the like.

The term "addition salt" as used hereinabove also comprises the solvates which either the compounds of formula I or the compounds of formula II as well as the salts thereof, are able to form. Such solvates are for example hydrates, alcoholates and the like.

The term "quaternary amine" as used hereinbefore defines the quaternary ammonium salts which either the compounds of formula I or the compounds of formula II are able to form by reaction between a basic nitrogen of either the compounds of formula I or the compounds of formula II and an appropriate quaternizing agent, such as, for example, an optionally substituted alkylhalide, arylhalide or arylalkylhalide, e.g. methyliodide or benzyliodide. Other reactants with good leaving groups may also be used, such as alkyl trifluoromethanesulfonates, alkyl methanesulfonates, and alkyl p-toluenesulfonates. A quaternary amine has a positively charged nitrogen.Pharmaceutically acceptable counterions include chloro, bromo, iodo, trifluoroacetate and acetate. The counterion of choice can be introduced using ion exchange resins.

The N-oxide forms of the present compounds are meant to comprise either the compounds of formula I or the compounds of formula II wherein one or several nitrogen atoms are oxidized to the so-called N-oxide.

It will be appreciated that either the compounds of formula I or the compounds of formula II may have metal binding, chelating, complex forming properties and therefore may exist as metal complexes or metal chelates. Such metalated derivatives of the compounds of formula I are intended to be included within the scope of the present invention.

Some of the compounds of either the compounds of formula I or the compounds of formula II may also exist in their tautomeric form. Such forms although not explicitly indicated in the above formula are intended to be included within the scope of the present invention.

The compounds described herein may have asymmetric centers and occur as racemates, racemic mixtures, individual diastereomers or enantiomers, with all isomeric forms being included in the present invention. Compounds of the present invention having a chiral center can exist in and be isolated in optically active and racemic forms. Some compounds can exhibit polymorphism.

The term "alkyl" as used herein it does refer to any linear, branched, or cyclic hydrocarbon in which all carbon-carbon bonds are single bonds. Alkyl chains may optionally be substituted by heteroatoms.

The term "alkenyl" and "unsubstituted alkenyl" are used interchangeably herein and refer to any linear, branched, or cyclic alkyl with at least one carbon-carbon double bond.

Furthermore, the term "alkynyl" as used herein it does refer to any linear, branched, or cyclic alkyl or alkenyl with at least one carbon-carbon triple bond.

The term "aryl" as used herein it does refer to any aromatic cyclic alkenyl or alkynyl, being as a group or part of a group is phenyl or naphthalenyl, each optionally substituted with one, two or three substituents selected from halo, hydroxy, nitro, cyano, carboxyl, C₁₋₆alkyl, C₁₋₆alkoxy, C₁₋₆alkoxyC₁₋₆alkyl, C₁₋₆alkylcarbonyl, amino, mono- or diC₁₋₆alkylamino, azido, mercapto, polyhaloC₁₋₆alkyl, and polyhaloC₁₋₆alkoxy. Preferred aryl groups are phenyl and naphtyl. The term "alkaryl" is employed where an aryl is covalently bound to an alkyl, alkenyl, or alkynyl.

The term "substituted" as used herein refers to a replacement of an atom or chemical group (e.g., H, NH₂, or OH) with a functional group, and particularly contemplated functional groups include nucleophilic groups (e.g., -NH₂, -OH, -SH, -NC, etc.), electrophilic groups (e.g., C(O)OR, C(X)OH, etc.), polar groups (e.g., -OH), non-polar groups (e.g., aryl, alkyl, alkenyl, alkynyl, etc.), ionic groups (e.g., -NH₃⁺), and halogens (e.g., -F, -CI), and all chemically reasonable combinations thereof. Thus, the term "functional group" and the term "substituent" are used interchangeably herein and refer to nucleophilic groups (e.g., -NH₂, -OH, -SH, -NC, - CN, etc.), electrophilic groups (e.g., C(O)OR, C(X)OH, C(Halogen)OR, etc.), polar groups (e.g., -OH), non-polar groups (e.g., aryl, alkyl, alkenyl, alkynyl, etc.), ionic groups (e.g., -NH₃⁺), and halogens.

Functional groups such as -OH, -NH₂, and the like, can incorporate protecting groups (abbreviated as PG) such as those known for those skilled in the art (GREENE'S PROTECTIVE. GROUPS IN ORGANIC. SYNTHESIS. Fourth Edition. PETER G. M. WUTS. and. THEODORA W. GREENE. 2007. Wiley-Interscience). By way of example, hydroxyl protection (Greene's vide supra, pages 16-366), including 1,2-diols could be in the form of ethers, esters, cyclic acetals, cyclic ketals, and silyl derivatives, such as, but not limited to, methyl ether, methoxymethyl ether, methylthiomethyl ether, *t*-butylthiomethyl ether, (phenyldimethylsislymethoxymethyl) ether, benzyloxymethyl ether, *p*-methoxybenzyloxymethyl ether, *p*-nitrobenzyloxymethyl ether, o-nitrobenzyzloxymethyl ether, (4-methoxyphenoxy)methyl ether, guaiacolmethyl ether, *t-*butoxymethyl ether, 4-pentenyloxymethyl ether, siloxymethyl ether, 2-methoxethoxymethyl ether, 2,2,2-trichloroethoxymethyl ether, bis(2-chloroethoxy)methyl ether, 2-(trimethylsilyl)ethoxymethyl ether, menthoxymethyl ether, tetrahydropyranyl ether, 3-bromotetrahydropyranyl ether, tetrahydrothiopyranyl ether, 1-methoxycyclohexyl ether, 4-methoxytetrahydropyranyl ether, 4-methoxytetrahydrothiopyranyl ether, 4-methoxytetrahydrothiopyranyl *S*, *S*-Dioxido ether, 1-[(2-chloro-4-methyl)phenyl]-4-methoxypiperidin-4-yl ether, 1,4-dioxan-2-yl ether, 1,4-dioxan-2-yl ether, tetrahydrothiofuranyl ether, 2,3,3a,4,5,6,7,7a-octahydro-7,8,8-trimethyl-4,7-methanobenzofuran-2-yl ether, 1-ethoxyethyl ether, 1-(2-chloroethoxy)ethyl ether, 1-hydroxyethyl ether, 2-bromoethyl ether, 1-[2-(trimethylsilyl)ethoxy]ethyl ether, 1-(2-cyanoethoxy)ethyl ether, prenyl ether, cynnamyl ether, propargyl ether, p-nitrophenyl ether, 1-methyl-1-methoxyethyl ether, 1-methyl-1-benzyloxyethyl ether, 1-methyl-1-1-benzyloxy-2-fluoroethyl ether, 2,2,2-trichloroethyl ether, 2-trimethylsilylethyl ether, 2-(phenylselenyl)ethyl ether, *t*-butyl ether, allyl ether, *p*-chlorophenyl ether, *p*-methoxyphenyl ether, 2,4-dinitrophenyl ether, benzyl ether, *p*-methoxylbenzyl ether, 3,4-dimethoxybenzyl ether, 2,6-dimethoxybenyzl, *o*-nitrobenzyl ether, *p*-nitrobenzyl ether, *p-*bromobenzyl ether, *p*-chlorobenzyl ether, 2,6-dichlorobenzyl ether, 2,4-dinitrobenzyl ether, fluorous benzyl ether, trimethylsilylxylyl ether, *p*-phenylbenzyl ether, cumyl ether, *p-*azidobenzyl ether, 2,6-difluorobenzyl ether, *p*-cyanobenzyl ether, p-phenylbenzyl ether, 2-picolyl ether, 4-picolyl ether, 3-methyl-2-picolyl *N*-oxido ether, diphenylmethyl ether, *p,p'-*dinitrobenzhydryl ether, 5-dibenzosuberyl ether, triphenylmethyl ether, α-naphtyldiphenylmethyl ether, *p*-methoxyphenyldiphenylmethyl ether, di(*p-*methoxyphenyl)phenylmethyl ether, tri(*p*-methoxyphenyl)phenylmethyl ether, 4-(4'-bromophenacyloxyphenyl)diphenylmethyl ether, 4,4',4"-tris(4,5-dichlorophthalimidophenyl)methyl ether, pentadienylnitrobenzyl, p-azidobenzyl ether, *p-*(methylsulfinyl)benzyl ether, 2-naphthylmethyl ether, 2-quinolinylmethyl ether, 1-pyrenylmethyl ether, 4-methoxydiphenylmethyl ether, 4-phenyldiphenylmethyl ether, α-naphthyldiphenylmethyl ether, *p*-methoxyphenyldiphenylmethyl ether, anthryl ether, 9-phenylthioxanthyl ether and the like; Silyl ethers such as trimethylsilyl ether, triethylsilyl ether, triisopropylsilyl ether, dimethylhexylsilyl ether, 2-norbornyldimethylsilyl ether, *t-*butyldimethylsilyl ether, *t*-butyldiphenylsilyl ether, tribenzylsilyl ether, tri-*p*-xylylsilyl ether, triphenylsilyl ether, diphenylmethylsilyl ether, di-*t*-butylmethylsilyl ether, bis(t-butyl)-1-pyrenylmethoxysilyl ether, tris(trimethylsilyl)silyl ether, (2-hidroxystyryl)dimethylsilyl ether, *t-*butoxydiphenylsilyl ether, 1,1,3,3-tetraisopropyl-3-[2-(tripheynlmethoxy)ethoxy]disiloxane-1-yl ether, fluorous silyl ether, and the like; Esters such as formate, benzoylformate, acetate, chloroacetate, dichloroacetate, trichloroacetate, trichloroacetimidate, trilfuoroacetate, methoxyacetate, triphenylmethoxyacetate, phenoxyacetate, *p*-chloropheynyacetate, phenylacetate, diphenylacetate, 3-phenylpropionate, bisfluorous chain type propanoyl ester, 4-pentenoate, levulinate, pivaloate, adamantoate, crotonate, 4-methoxylcrotonate, benzoate, p-phenylbenzoate, mesitoate, 4-bromobenzoate, 2,5-diflourobenzoate, p-nitrobenzoate, picolinate, nicotinate, 2-(azidomethyl)benzoate, 4-azidobutirate, (2-azidomethyl)phenylacetate, 2-{[(tritylthio)oxy]methyl}benzoate, 2-(allyloxy)phenylacetate, 2-(prenyloxymethyl)benzoate, 4-benzyloxybutyrate, 4-trialkylsiloxybutyrate, 4-acetoxy-2,2-dimethylbutyrate, 2,2-dimethyl-4-pentanoate, 2-iodobenzoate, 4-nitro-4-methylpentanoate, o-(dibromomethyl)benzoate, 2-formylbenzenesulfonate, 4-(methylthiomethoxy)butyrate, 2-(methylthiomethoxymethyl)benzoate, 2-(chloroacetoxymethyl)benzoate, 2-[(2-chloracetoxy)ethyl]benzoate, 2-[2-(benzyloxy)ethyl]benzoate, 2-[2-(4-methoxybenzyloxy)ethyl]benzoate, 2,6-dichloro-4-methylphenoxyacetate, 2,6-dichloro-4-(1,1,3,3-tetramethylbutyl)phenoxyacetate, chlorodiphenylacetate, isobutyrate, monosuccionate, tigloate, *o*-(methoxycarbonyl)benzoate, *p*-benzoate, α-napthoate, nitrate, alkyl *N,N,N',N'*-tetramethylphosphorodiamidate, 2-chlorobenzoate, and the like; Sulfonates such as sulfate, allylsulfonate, methanesulfonate, benzylsulfonate, tosylate, 2-trifluoromethylsulfonate and the like; Carbonates such as alkyl methyl carbonate, methoxymethyl carbonate, 9-fluoromethyl carbonate, ethyl carbonate, bromoethyl carbonate, 2-(trimethylsilyl)ethyl carbonate, isobutyl carbonate, *t*-butyl carbonate, vinyl carbonate, allyl carbonate, propargyl carbonate, *p*-nitrophenyl carbonate, benzyl carbonate, 2-dansylethyl carbonate, phenacyl carbonate, methyl dithiocarbonate, S-benzyl thiocarbonate and the like; Carbamates such as dimethylthiocarbamate, *N*-phenylcarbamate, and the like; Cyclic acetals and ketals such as methylene acetal, ethylidene acetal, *t*-butylmethylidene acetal, 1-*t-*butylethylidine ketal, 1-phenyethylidene ketal, 2-(methoxycarbonyl)ethylidene acetal, 2-(*t-*butylcarbonyl)ethylidene acetal, phenylsulfonylethylidene acetal, 3-(benzyloxy)propylidene acetal, isopropylidene acetal or acetonide, cyclopentylidene acetal, benzylidene acetal, *p-*methoxybenzylidene acetal, mesitylene acetal, naphthaldehyde acetal, 9-anthracene acetal, benzophenone ketal and the like; Chiral ketones such as camphor ketal, menthone ketal and the like; Cyclic ortho esters such as methoxymethylene acetal, ethoxymethylene acetal, dimethoxymethylene orto ester, methylidene orto ester, phthalide orto ester, 1,2-dimethoxyethylidene orto ester, 2-oxacyclopentylidene orto ester, butane 2,3-bisacetal, cyclohexane-1,2-diacetal, dispiroketals and the like; Silyl derivatives such as di-*t*-butylsilylene group, diakkylsilylene group, 1,3-(1,1,3,3-tetraisopropyldisiloxanylidene) derivative, 1,1,3,3-tetra-*t*-butoxydisiloxanylidene derivative, methylene-bis-(diisopropylsilanoxanylidene, 1,1,4,4-tetrapheynyl-1,4-disilanylidene, *o*-xylyl ether, 3,3'-oxybis(dimethoxytrityl)ether, and the like; cyclic carbonates; cyclic borate such as methyl boronate, ethyl boronate, and the like.

The term "optionally substituted" when referring to alkyl, alkenyl, alkynyl, cycloalkyl, cycloalkenyl, aryl and heterocycle is intended to cover groups having oxo, ethylenedioxy, alkanoyloxy, alkoxy, alkylthio, carboxyl, halogen, thienyl, acetyl, 1-oxopropyl, 2-oxopropyl, 2-oxobutyl, 3-oxobutyl, 3-oxopentyl, 4-oxopentyl, 4-oxohexyl, 5-oxohexyl, ethylenedioxymethyl, 1,1-ethylenedioxyethyl, 2,2-ethylenedioxyethyl, 1,1-ethylenedioxypropyl, 2,2-ethylenedioxypropyl, 3,3-ethylenedioxypropyl, 1,1-ethylenedioxybutyl, 2,2-ethylenedioxybutyl, 3,3-ethylenedioxybutyl, 4,4-ethylenedioxybutyl, 3,3-ethylenedioxypentyl, 4,4-ethylenedioxyhexyl, 5,5-ethylenedioxyhexyl, acetyloxymethyl, 2-acetyloxyethyl, 3-acetyloxypropyl, 3-acetyloxybutyl, 4-acetyloxybutyl, 3-propionyloxybutyl, 3-butyryloxybutyl, 3-valeryloxypentyl, 3-hexanoyloxyhexyl, 4-acetyloxypentyl, 5-acetyloxypentyl, 4-acetyloxyhexyl, 5-acetyloxyhexyl, 6-acetyloxyhexyl, methoxymethyl, ethoxymethyl, propoxymethyl, butoxymethyl, pentyloxymethyl, hexyloxymethyl, 1-methoxyethyl, 2-methoxyethyl, 2-ethoxyethyl, 2-propoxyethyl, 2-butoxyethyl, 2-pentyloxyethyl, 2-hexyloxyethyl, 3-methoxypropyl, 3-ethoxypropyl, 2-methoxybutyl, 4-ethoxybutyl, 3-methoxypentyl, 5-ethoxypentyl, 4-methoxyhexyl, 6-ethoxyhexyl, methylthiomethyl, ethylthiomethyl, propylthiomethyl, butylthiomethyl, pentylthiomethyl, hexylthiomethyl, 1-methylthioethyl, 2-methylthioethyl, 2-ethylthioethyl, 2-methylthiopropyl, 3-methylthiopropyl, 3-ethylthiobutyl, 4-butylthiobutyl, 5-methylthiopentyl, 6-ethylthiohexyl, carboxymethyl, 1-carboxyethyl, 2-carboxyethyl, 2-carboxypropyl, 3-carboxypropyl, 4-carboxybutyl, 5-carboxypentyl, 6-carboxyhexyl, fluoromethyl, bromomethyl, chloromethyl, iodomethyl, 2-chloroethyl, 2-bromopropyl, 3-iodopropyl, 4-fluorobutyl, 5-chloropentyl, 6-bromohexyl, 2-thienylmethyl, 1-(2-thienyl)ethyl, 2-(2-thienyl)ethyl and the like.

The term "amino acid" refers to any of a class of organic compounds that contains at least one amino group, -NH-, and one carboxyl group, -COOH. These compounds can be the natural amino acids present in peptides or can contain any substitution in the amino group, in the carboxyl group or in the side chain. They can also present different chirality of the peptidic natural amino acids or can have different backbone, linear or cyclic, but must present, as said, at least one amino group and one carboxyl group. Amino acids can incorporate functional or protecting groups, such as those known for those skilled in the art (T.W.Greene, *vide supra*)*.* Preferred amino acids include, but are not limited to, alanine, valine, leucine and isoleucine.

As for the reaction conditions for the Reaction II-I, this process is preferably carried out at the following conditions, independently one of each other:
- the temperature ranges from 18 to 100 °C;
- the reaction time ranges from 1 minute to 600 h;
- pH ranges from 3 to 12;
- the concentration of compound of formula II or a pharmaceutically acceptable salt thereof as defined above ranges from 0.1 mM to 500 M;
- the amount of enzyme having cytidine deaminase activity ranges from 0.001 to 10000 mg/ml, preferably from 0.001 to 1000 mg/ml, in terms of concentration, or alternatively, the amount of enzyme having deaminase activity ranges from 0.001 to 10000 AU/micromol substrate, preferably from 0.001 to 100 AU/micromol substrate.

The reaction medium is an aqueous optionally buffered solution containing organic or inorganic salts such as, but not limited to, phosphate, carbonate, citrate, acetate. In a further embodiment, the reaction medium optionally also contains up to 50%, preferably up to 30% and more preferably up to 15% of a suitable organic solvent. Preferably, said organic solvent is selected from methanol, ethanol, propanol, isopropanol, t-butanol, n-butanol, ethyl acetate, isopropyl acetate, butyl acetate, dichloromethane, toluene, tetrahydrofuran, 2-methyltetrahydrofuran, acetonitrile, acetone, cyclopentyl methyl ether, methyl ethyl ketone, methyl isobutyl ketone, dimethylamide, dimethylformamide and dimethylsulfoxide.

The process according to present invention may also include isolation and/or purification steps of the NA produced by standard operation means selected from chromatography, precipitation, filtration, concentration and crystallization.

The present invention also relates to novel compounds represented by formula I and formula II (see Table 1). Table 1.

| **Compound** | **Z₁** | **Z₂ or Z₄** | **Z₃** | **R^{S1}** | **R^{S2}** | **Y₁** | **Y₂** | **CAS nr** |
|---|---|---|---|---|---|---|---|---|
| **9** | O | A | CH-OPG | H | OPG | O-Ph | Leu-methyl ester | NO |
| **10** | O | A | CHOH | H | OH | O-Ph | Leu-methyl ester | NO |
| **11** | O | A | CH-OPG | H | OPG | O-Ph | Val-methyl ester | NO |
| **12** | O | A | CHOH | H | OH | O-Ph | Val-methyl ester | NO |
| **13** | O | A | CHOPG | H | OPG | O-Ph | Ala-isopropyl ester | NO |
| **14** | O | A | CHOH | H | OH | O-Ph | Ala-isopropyl ester | NO |
| **14-Me** | O | A | CHOH | H | OH | O-Ph | Ala-methyl ester | NO |
| **16** | O | A | CHOH | F | F | O-Ph | Leu-methyl ester | NO |
| **17** | O | A | CHOH | F | F | O-Ph | Val-methyl ester | NO |
| **18** | O | A | CHOH | F | F | O-Ph | Ala-isopropyl ester | 1627888-08-7; 1562406-06-7 & 1562406-07-8 |
| **21** | O | B | CHOH | H | H | O-Ph | Val-methyl ester | NO |
| **22** | O | B | CHOPOY₁Y₂ | H | H | O-Ph | Val-methyl ester | NO |
| **10B** | O | E | CHOH | H | OH | O-Ph | Leu-methyl ester | NO |
| **12B** | O | E | CHOH | H | OH | O-Ph | Val-methyl ester | NO |
| **14B** | O | E | CHOH | H | OH | O-Ph | Ala-isopropyl ester | NO |
| **14B-Me** | O | E | CHOH | H | OH | O-Ph | Ala-methyl ester | NO |
| **16B** | O | E | CHOH | F | F | O-Ph | Leu-methyl ester | NO |
| **17B** | O | E | CHOH | F | F | O-Ph | Val-methyl ester | NO |
| **18B** | O | E | CHOH | F | F | O-Ph | Ala-isopropyl ester | NO |
| **21B** | O | F | CHOH | H | H | O-Ph | Val-methyl ester | NO |

wherein
A= Cytosine, wherein R¹=O, R²=R³=R⁴=R⁵=H
B= 5-Azacytosine, wherein R¹=O, R²=R³=R⁵=H
E= Uracil, wherein R¹=O, R⁴=R⁵=H
F= 5-Azauracil, wherein R¹=O, R⁵=H
PG= protecting group, as defined above

The present invention will be further described by means of examples which do not intend to limit the scope of the instant invention. Comparative examples are also provided. The reaction schemes provided below only intend to illustrate how to obtain various compounds as disclosed herein. As it is well known by a person skilled in the art, different conditions can be applied, when necessary, providing that the the general process as disclosed herein is performed.

### EXAMPLES

### Comparative Example 1: Deamination of cytidine (compound 1) to uridine

A 100 mM solution of the cytidine (495 µl) in 100 mM phosphate buffer at pH 7 was mixed with 50 µL of cytidine deaminase enzyme solution containing >300 AU in phosphate buffer. The reaction was performed at 37°C during 5 minutes and stopped with HCI. Then, the crude reaction was filtered through a 10 KDa membrane, and a portion was diluted and analyzed by HPLC under UV-DAD (ultraviolet-diode array detection). Product identification was performed by comparison to a standard sample. Uridine was obtained in quantitative yield (>99%).

### Comparative Example 2: Deamination of cytidine 5'-monophosphate to uridine 5'-monophosphate

A 100 mM solution of cytidine 5'-monophosphate (495 µl) in 100 mM phosphate buffer at pH 7 was mixed with 50 µL of cytidine deaminase enzyme solution containing >300 AU in phosphate buffer. The reaction was performed at 37°C during 5 minutes and stopped with HCI. Then, the crude reaction was filtered through a 10 KDa membrane, and a portion was diluted and analyzed by HPLC-UV-DAD. The expected uridine 5'-monophosphate product was not detected, by comparison to a standard sample. Therefore, no conversion of the substrate into the final product was obtained (0 %).

### Comparative Example 3: Deamination of 2'-deoxycytidine to 2'-deoxyuridine

A 100 mM solution of 2'-deoxycytidine (495 µL) in 100 mM phosphate buffer at pH 7 was mixed with 50 µL of cytidine deaminase enzyme solution containing >300 AU in phosphate buffer. The reaction was performed at 37°C during 5 minutes and stopped with HCI. Then, the crude reaction was filtered through a 10 KDa membrane, and a portion was diluted and analyzed by HPLC-UV-DAD. Product identification was performed by comparison to a standard sample. 2'-Deoxyuridine was obtained in quantitative yield (>99%).

### Comparative Example 4: Deamination of 2'-deoxycytidine 5'-monophosphate to 2'-deoxyuridine 5'-monophosphate

A 100 mM solution of 2'-deoxycytidine 5'-monophosphate (495 µL) in 100 mM phosphate buffer at pH 7.0 was mixed with 50 µL of cytidine deaminase enzyme solution containing >300 AU in phosphate buffer. The reaction was performed at 37°C during 5 minutes and stopped with HCI. Then, the crude reaction was filtered through a 10 KDa membrane, and a portion was diluted and analyzed by HPLC-UV-DAD. The expected 2'-deoxyuridine 5'-monophosphate product was not detected by comparison to a standard sample. Therefore, no conversion of the substrate into the final product was obtained (0 %).

### Comparative Example 5: Deamination of cytidine 5'-triphosphate to uridine 5'-triphosphate

A 100 mM solution of cytidine 5'-triphosphate (495 µL) in 100 mM phosphate buffer at pH 7 was mixed with 50 µL of cytidine deaminase enzyme solution containing >300 AU in phosphate buffer. The reaction was performed at 37°C during 5 minutes and stopped with HCI. Then, the crude reaction was filtered through a 10 KDa membrane, and a portion was diluted and analyzed by HPLC-UV-DAD. The expected uridine 5'-triphosphate product was not detected by comparison to a standard sample. Therefore, no conversion of the substrate into the final product was obtained (0 %).

### Comparative Example 6: Deamination of cytarabine (cytosine arabinoside) to uridine arabinoside

A 100 mM solution of cytarabine (495 µL) in 100 mM phosphate buffer at pH 7 was mixed with 50 µL of cytidine deaminase enzyme solution containing >300 AU in phosphate buffer. The reaction was performed at 37°C during 5 minutes and stopped with HCI. Then, the crude reaction was filtered through a 10 KDa membrane, and a portion was diluted and analyzed by HPLC-UV-DAD. The expected uridine arabinoside was identified by comparison to a standard sample, and formed in quantitative yield (>99%).

### Comparative Example 7: Deamination of cytarabine 5'-monophosphate (cytosine arabinoside 5'-monophosphate) to uridine arabinoside 5'-monophosphate

A 100 mM solution of cytarabine 5'-monophosphate (495 µL) in 100 mM phosphate buffer at pH 7 was mixed with 50 µL of cytidine deaminase enzyme solution containing >300 AU in phosphate buffer. The reaction was performed at 37°C during 5 minutes and stopped with HCI. Then, the crude reaction was filtered through a 10 KDa membrane, and a portion was diluted and analyzed by HPLC-UV-DAD. The expected product was not detected. No conversion of the substrate into the final product was obtained (0 %).

### Comparative Example 8: Deamination of gemcitabine (compound 2) to uridine arabinoside

A 70 mM solution of gemcitabine (495 µL) in 100 mM phosphate buffer at pH 7 was mixed with 5 µL of cytidine deaminase enzyme solution containing >30 AU in phosphate buffer. The reaction was performed at 37°C during 5 minutes and stopped with HCI. Then, the crude reaction was filtered through a 10 KDa membrane, and a portion was diluted and analyzed by HPLC under UV-DAD (ultraviolet-diode array detection). The expected product was obtained in quantitative yield (>99% from the crude reaction).

### Example 9: Preparation of methyl ((((2R,3S,4R,5R)-5-(4-amino-2-oxopyrimidin-1(2H)-yl)-3,4-dihydroxytetrahydrofuran-2-yl)methoxy)(phenoxy)phosphoryl)-L-leucinate hydrochloride (compound 10):

### -Step 1: Protection of cytidine (compound 1) to furnish 2',3'-protected cytidine (compound 8):

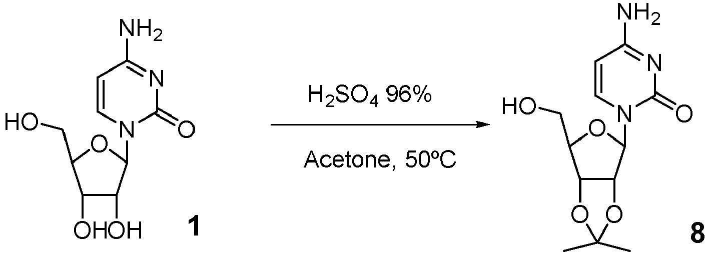

To a 100 mL round bottom flask fitted with a reflux condenser, cytidine (4.41 g, 18.1 mmol) suspended in dry acetone (44 mL) was added. To the stirring suspension, activated 4 Å molecular sieves and H₂SO₄ 98% (0.145 mL, 2.7 mmol, 0.15 equivalents) were added. The suspension was left stirring at 50°C. After 16 hours, at r.t., NaHCO₃ (2 g) was added and the mixture was stirred for 0.5 hours. Then, the crude mixture was filtered *in vacuo.* The solid obtained was washed with MeOH/EtOH 1/1 (2 × 20 mL). The organic phases were combined and evaporated to furnish the 2',3'-protected cytidine (compound 8,1.33 g, 25.8 %), as a white solid.

¹H-NMR (δ, ppm): 8.19 - 8.32 (m, 1 H), 6.21 (dd, J=8.07, 2.93 Hz, 1 H), 5.93 (s, 1 H), 4.95 - 5.14 (m, 1 H), 4.88 (dd, J=6.24, 2.57 Hz, 1 H), 4.38 (d, J=2.93 Hz, 1 H), 3.66 - 3.83 (m, 2 H), 1.36 - 1.75 (m, 6 H).

### -Step 2: Preparation of methyl ((4-nitrophenoxy)(phenoxy)phosphoryl)-L-leucinate (compound 4):

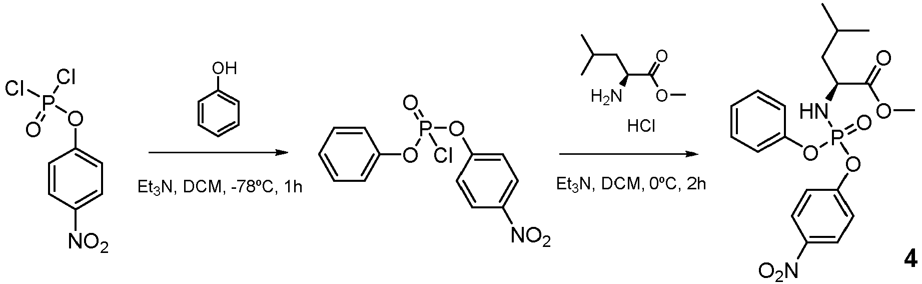

To a 50 mL three neck round bottom flask, under inert atmosphere, 4-nitrophenyl phosphorodichloridate (0.5 g, 1.95 mmol) dissolved in dry DCM (8 mL) was added. To the stirring solution, at -78°C, a solution containing phenol (0.184 g, 1.95 mmol, 1 equivalent), triethylamine (0.30 mL, 2.14 mmol, 1.1 equivalent) in dry DCM (3 mL), was added. The solution was stirred for 20 minutes at -78°C, and then it was transferred via cannula to a 100 mL three neck round bottom flask, under inert atmosphere and at 0°C, containing a solution of L-leucine methyl ester hydrochloride (0.355 g, 1.95 mmol, 1 equivalent), triethylamine (0.95 mL, 6.83 mmol, 3.5 equivalents) in dry DCM (5.5 mL). After 2 hours stirring at 0°C, the reaction was allowed to reach room temperature and was stirred for another 16 hours. The reaction crude was purified by column chromatography using hexane/AcOEt 1/1 as the solvent on SiO₂. 509 mg (62 % yield) of methyl ((4-nitrophenoxy)(phenoxy)phosphoryl)-L-leucinate were obtained, as yellowish oil.

¹H-NMR (δ, ppm): 8.29 (m, 2 H), 7.43 (m, 4 H), 7.26 (m, 3 H), 3.97 (m, 1 H), 3.60 (s, 3 H), 3.35 (m, 1 H), 1.55 (m, 3 H), 0.84 (m, 6 H).

### - Step 3: Coupling of compound 8 and compound 4 to furnish methyl ((((3aR,4R,6R,6aR)-6-(4-amino-2-oxopyrimidin-1(2H)-yl)-2,2-dimethyltetrahydrofuro[3,4-d][1,3]dioxol-4-yl)methoxy)(phenoxy)phosphoryl)-L-leucinate (compound 9)

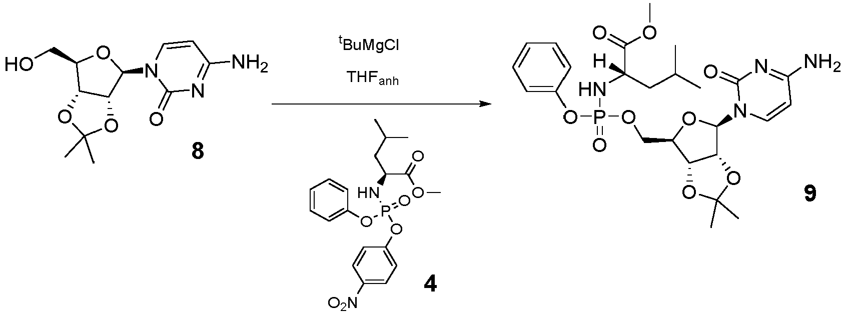

To a 25 mL round bottom flask, under inert atmosphere, 4-amino-1-((3aR,4R,6R,6aR)-6-(hydroxymethyl)-2,2-dimethyltetrahydrofuro[3,4-d][1,3]dioxol-4-yl)pyrimidin-2(1H)-one (50 mg, 0.177 mmol) dissolved in dry THF/DMF (1.5/1 mL) was added. To the stirring solution, at room temperature, 1 M *tert*-butylmagnessium chloride (0.265 mL, 0.265 mmol, 1.5 equivalents) was added, producing a white solid. After 30 minutes, methyl ((4-nitrophenoxy)(phenoxy)phosphoryl)-L-leucinate (97 mg, 0.229, 1.3 equivalents) dissolved in dry THF (1 mL) was added, forming a yellowish suspension. After 16 hours, the solvents were evaporated, and the crude was purified by column chromatography using CH₂Cl₂/MeOH 9/1 as the solvent on SiO₂. 49 mg (49 % yield) of methyl ((((3aR,4R,6R,6aR)-6-(4-amino-2-oxopyrimidin-1(2H)-yl)-2,2-dimethyltetrahydrofuro[3,4-d][1,3]dioxol-4-yl)methoxy)(phenoxy)phosphoryl)-L-leucinate were obtained, as yellowish semisolid. ¹H-NMR (δ, ppm): 7.62 (dd, J = 16.1, 7.3 Hz, 1 H), 7.35 (t, J = 7.7 Hz, 2 H), 7.21 (m, 3 H), 5.84 (m, 2 H), 4.33 (m, 3 H), 3.87 (m, 1 H), 3.67 (s, 3 H), 2.65 (m, 2 H), 1.54 (s, 6 H), 1.35 (m, 3 H), 0.87 (m, 6 H).

MS-ESI (+): (m/z): [M+Na]⁺ =589

### - Step 4: Deprotection of compound 9 to furnish compound 10:

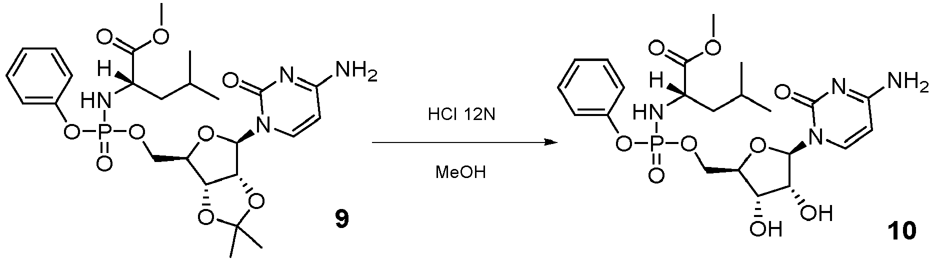

To a 10 mL round bottom flask, under inert atmosphere, methyl ((((3aR,4R,6R,6aR)-6-(4-amino-2-oxopyrimidin-1(2H)-yl)-2,2-dimethyltetrahydrofuro[3,4-d][1,3]dioxol-4-yl)methoxy)(phenoxy)phosphoryl)-L-leucinate (40 mg, 0.071 mmol) was dissolved in MeOH (0.8 mL). To the stirring solution, at room temperature, HCI 12 N (45 uL, 3.5 mmol, 50 equivalents) was added. After 48 hours, the solvent was evaporated *in vacuo.* 40 mg (quantitative yield) of methyl ((((2R,3S,4R,5R)-5-(4-amino-2-oxopyrimidin-1(2H)-yl)-3,4-dihydroxytetrahydrofuran-2-yl)methoxy)(phenoxy)phosphoryl)-L-leucinate hydrochloride were obtained, as yellowish oil.

MS (m/z): [M+H]⁺=527, [M+Na]⁺ =549.

### Example 10: Preparation of methyl ((((2R,3S,4R,5R)-5-(4-amino-2-oxopyrimidin-1(2H)-yl)-3,4-dihydroxytetrahydrofuran-2-yl)methoxy)(phenoxy)phosphoryl)-L-valinate hydrochloride (compound 12):

### -Step 1: Preparation of methyl ((4-nitrophenoxy)(phenoxy)phosphoryl)-L-valinate (compound 5):

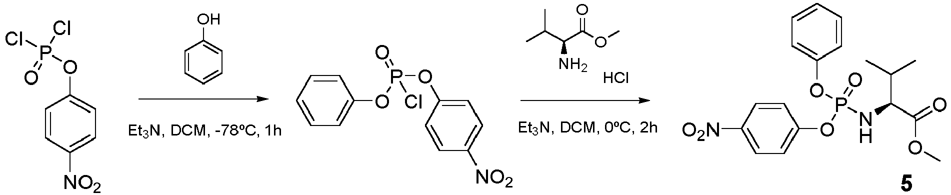

Following the procedure described for compound 4, using 4-nitrophenyl phosphorodichloridate (0.5 g, 1.95 mmol) and L-valine methyl ester hydrochloride (0.327 g, 1.95 mmol), methyl ((4-nitrophenoxy)(phenoxy)phosphoryl)-L-valinate (654 mg, 81.9 % yield) was obtained as colourless oil.

¹H-NMR (δ, ppm): 8.28 (d, J = 8.8 Hz, 2 H), 7.46 (dd, J = 8.9, 3.4, 2 H), 7.38 (t, J = 7.6 Hz, 2 H), 7.26 (m, 3 H), 3.75 (dt, J = 10.4, 6.3, 1 H), 3.60 (s, 3 H), 2.02 (m, 1 H), 0.87 (t, J = 7.8 Hz, 6 H).

### - Step 2: Coupling of compound (8) to compound (5) to furnish methyl ((((3aR,4R,6R,6aR)-6-(4-amino-2-oxopyrimidin-1(2H)-yl)-2,2-dimethyltetrahydrofuro[3,4-d][1,3]dioxol-4-yl)methoxy)(phenoxy)phosphoryl)-L-valinate (compound 11):

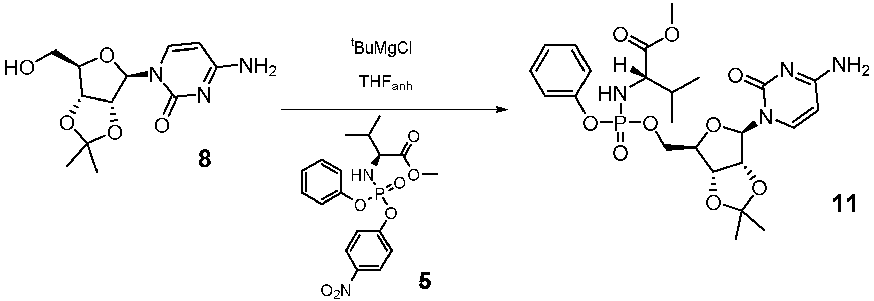

Following the procedure described for compound 9, using 4-amino-1-((3aR,4R,6R,6aR)-6-(hydroxymethyl)-2,2-dimethyltetrahydrofuro[3,4-d][1,3]dioxol-4-yl)pyrimidin-2(1H)-one (50 mg, 0.177 mmol) and methyl ((4-nitrophenoxy)(phenoxy)phosphoryl)-L-valinate (94 mg, 229 mmol), methyl ((((3aR,4R,6R,6aR)-6-(4-amino-2-oxopyrimidin-1(2H)-yl)-2,2-dimethyltetrahydrofuro[3,4-d][1,3]dioxol-4-yl)methoxy)(phenoxy)phosphoryl)-L-valinate (55 mg, 56 % yield) was obtained.

MS-ESI (+): (m/z): [M+Na]⁺ =575

### - Step 3: Deprotection of compound 11 to furnish methyl ((((2R,3S,4R,5R)-5-(4-amino-2-oxopyrimidin-1(2H)-yl)-3,4-dihydroxytetrahydrofuran-2-yl)methoxy)(phenoxy)phosphoryl)-L-valinate hydrochloride (compound 12):

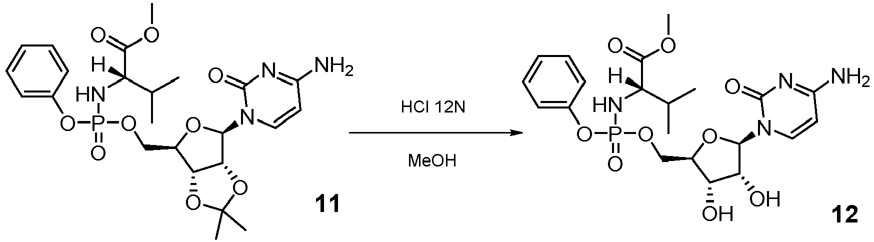

Following the procedure described for compound 9, using methyl ((((3aR,4R,6R,6aR)-6-(4-amino-2-oxopyrimidin-1(2H)-yl)-2,2-dimethyltetrahydrofuro[3,4-d][1,3]dioxol-4-yl)methoxy)(phenoxy)phosphoryl)-L-valinate (55 mg, 0.1 mmol), methyl ((((2R,3S,4R,5R)-5-(4-amino-2-oxopyrimidin-1(2H)-yl)-3,4-dihydroxytetrahydrofuran-2-yl)methoxy)(phenoxy)phosphoryl)-L-valinate hydrochloride (50 mg, quantitative yield) was obtained.

MS-ESI (+): (m/z): [M+Na]⁺ =535

### Example 11: Preparation of isopropyl ((((2R,3S,4R,5R)-5-(4-amino-2-oxopyrimidin-1(2H)-yl)-3,4-dihydroxytetrahydrofuran-2-yl)methoxy)(phenoxy)phosphoryl)-L-alaninate hydrochloride (compound 14):

### - Step 1: Preparation of isopropyl ((4-nitrophenoxy)(phenoxy)phosphoryl)-L-alaninate (compound 6):

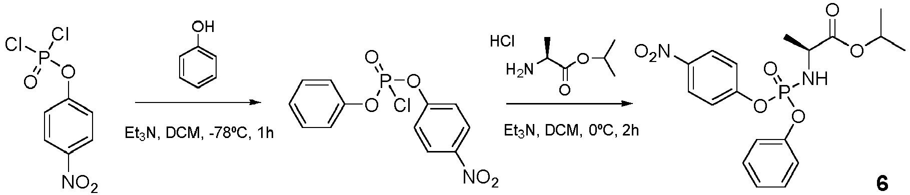

Following the procedure described for compound 4, using 4-nitrophenyl phosphorodichloridate (1.0 g, 3.90 mmol) and L-alanine isopropyl ester hydrochloride (0.655 g, 3.90 mmol), isopropyl ((4-nitrophenoxy)(phenoxy)phosphoryl)-L-alaninate (1.11 g, 70 % yield) was obtained as colourless oil.

¹H-NMR (δ, ppm): 8.28 (d, J = 8.8 Hz, 2 H), 7.46 (dd, J = 15.8, 8.4, 2 H), 7.39 (m, 2 H), 7.26 (m, 3 H), 4.93 (dt, J = 12.5, 6.2, 1 H), 4.01 (m, 1 H), 1.32 (m, 3 H), 1.19 (t, J = 5.9 Hz, 6 H).

### - Step 2: Coupling of compound 8 to compound 6 to furnish isopropyl ((((3aR,4R,6R,6aR)-6-(4-amino-2-oxopyrimidin-1(2H)-yl)-2,2-dimethyltetrahydrofuro[3,4-d][1,3]dioxol-4-yl)methoxy)(phenoxy)phosphoryl)-L-alaninate (compound 13):

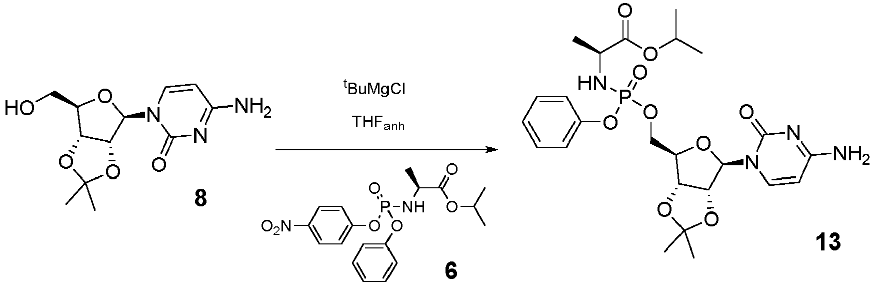

Following the procedure described for compound 9, using 4-amino-1-((3aR,4R,6R,6aR)-6-(hydroxymethyl)-2,2-dimethyltetrahydrofuro[3,4-d][1,3]dioxol-4-yl)pyrimidin-2(1H)-one (50 mg, 0.177 mmol) and isopropyl ((4-nitrophenoxy)(phenoxy)phosphoryl)-L-alaninate (94 mg, 0.229 mmol), isopropyl ((((3aR,4R,6R,6aR)-6-(4-amino-2-oxopyrimidin-1(2H)-yl)-2,2-dimethyltetrahydrofuro[3,4-d][1,3]dioxol-4-yl)methoxy)(phenoxy)phosphoryl)-L-alaninate (558 mg, 52 % yield) was obtained.

¹H-NMR (δ, ppm): 7.68 (dd, J = 12.8, 7.7 Hz, 1 H), 7.35 (m, 2 H), 7.21 (m, 3 H), 5.89 (dd, J = 17.6, 7.3 Hz, 1 H), 5.79 (d, J = 9.5 Hz, 1 H), 4.97 (m, 1 H), 4.80 (m, 2 H), 4.36 (m, 3 H), 3.89 (m, 1 H), 1.33 (m, 6 H), 1.31 (m, 7 H).

MS-ESI (+): (m/z): [M+Na]⁺ =575

### -Step 3: Deprotection of compound 13 to furnish isopropyl ((((2R,3S,4R,5R)-5-(4-amino-2-oxopyrimidin-1(2H)-yl)-3,4-dihydroxytetrahydrofuran-2-yl)methoxy)(phenoxy)phosphoryl)-L-alaninate hydrochloride (compound 14):

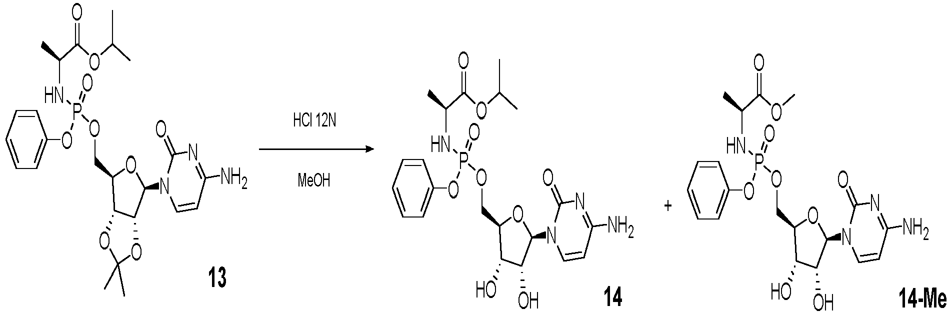

Following the procedure described for compound 9, using isopropyl ((((3aR,4R,6R,6aR)-6-(4-amino-2-oxopyrimidin-1(2H)-yl)-2,2-dimethyltetrahydrofuro[3,4-d][1,3]dioxol-4-yl)methoxy)(phenoxy)phosphoryl)-L-alaninate (113 mg, 0.205 mmol), a mixture of isopropyl ((((2R,3S,4R,5R)-5-(4-amino-2-oxopyrimidin-1(2H)-yl)-3,4-dihydroxytetrahydrofuran-2-yl)methoxy)(phenoxy)phosphoryl)-L-alaninate hydrochloride (compound 14) and methyl ((((2R,3S,4R,5R)-5-(4-amino-2-oxopyrimidin-1(2H)-yl)-3,4-dihydroxytetrahydrofuran-2-yl)methoxy)(phenoxy)phosphoryl)-L-alaninate hydrochloride (compound 14-Me) were obtained (115 mg). Compound 14-Me was obtained due to a transesterification in MeOH/HCI. Compound 14:
- MS-ESI (+): (m/z): [M+H]⁺=513, [M+Na]⁺ =535, [M+K]⁺ =551
- MS-ESI (-): (m/z): [M-H]⁻=511

Compound 14-Me:
- MS-ESI (+): (m/z): [M+H]⁺=485, [M+Na]⁺ =507, [M+K]⁺ =523
- MS-ESI (-): (m/z): [M-H] ⁻=483

### Example 12: Preparation of methyl ((((2R,3R,5R)-5-(4-amino-2-oxopyrimidin-1(2H)-yl)-4,4-difluoro-3-hydroxytetrahydrofuran-2-yl)methoxy)(phenoxy)phosphoryl)-L-leucinate (compound 16):

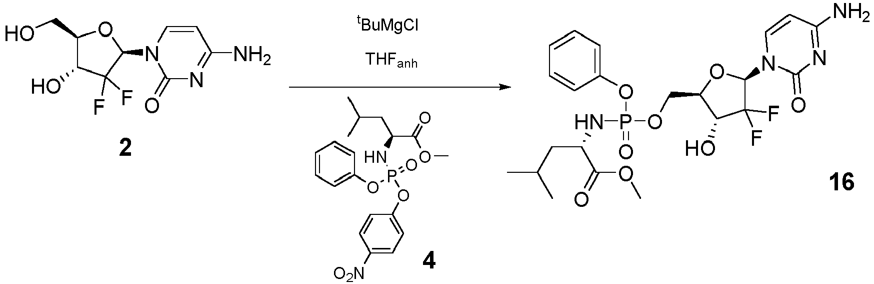

Following the procedure described for compound 9, using gemcitabine (compound 2), 50 mg, 0.190 mmol) and methyl ((4-nitrophenoxy)(phenoxy)phosphoryl)-L-leucinate (compound 4), 101 mg, 0.247 mmol), methyl ((((2R,3R,5R)-5-(4-amino-2-oxopyrimidin-1(2H)-yl)-4,4-difluoro-3-hydroxytetrahydrofuran-2-yl)methoxy)(phenoxy)phosphoryl)-L-leucinate (64 mg, 61 % yield) was obtained.

MS-ESI (+): (m/z): [M+H]⁺ =547, [M+Na]⁺ =569, [M+K]⁺ =585

### Example 13: Preparation of methyl ((((2R,3R,5R)-5-(4-amino-2-oxopyrimidin-1(2H)-yl)-4,4-difluoro-3-hydroxytetrahydrofuran-2-yl)methoxy)(phenoxy)phosphoryl)-L-valinate (compound 17):

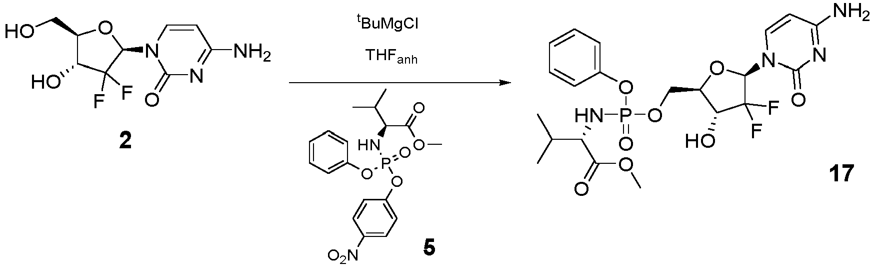

Following the procedure described for compound 9, using gemcitabine (compound 2), 50 mg, 0.190 mmol) and methyl ((4-nitrophenoxy)(phenoxy)phosphoryl)-L-valinate (compound 5), 101 mg, 0.247 mmol), methyl ((((2R,3R,5R)-5-(4-amino-2-oxopyrimidin-1(2H)-yl)-4,4-difluoro-3-hydroxytetrahydrofuran-2-yl)methoxy)(phenoxy)phosphoryl)-L-valinate (60 mg, 59 % yield) was obtained.

MS-ESI (+): (m/z): [M+H]⁺ =533, [M+Na]⁺ =555, [M+K]⁺ =571

MS-ESI (-): (m/z): [M-H]⁻ =530.9

### Example 14: Preparation of isopropyl ((((2R,3R,5R)-5-(4-amino-2-oxopyrimidin-1(2H)-yl)-4,4-difluoro-3-hydroxytetrahydrofuran-2-yl)methoxy)(phenoxy)phosphoryl)-L-alaninate (compound 18):

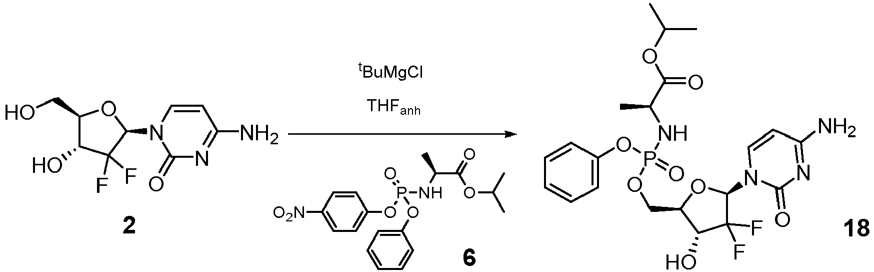

Following the procedure described for compound 9, using gemcitabine (compound (2), 50 mg, 0.190 mmol) and isopropyl ((4-nitrophenoxy)(phenoxy)phosphoryl)-L-alaninate (compound 6, 101 mg, 0.247 mmol), isopropyl ((((2R,3R,5R)-5-(4-amino-2-oxopyrimidin-1(2H)-yl)-4,4-difluoro-3-hydroxytetrahydrofuran-2-yl)methoxy)(phenoxy)phosphoryl)-L-alaninate (41 mg, 38 % yield) was obtained.

MS-ESI (+): (m/z): [M+H]⁺ =533, [M+Na]⁺ =555, [M+K]⁺ =571

### Example 15: Preparation of methyl ((((2R,3S,5R)-5-(4-amino-2-oxo-1,3,5-triazin-1(2H)-yl)-3-hydroxytetrahydrofuran-2-yl)methoxy)(phenoxy)phosphoryl)-L-valinate (compound 21):

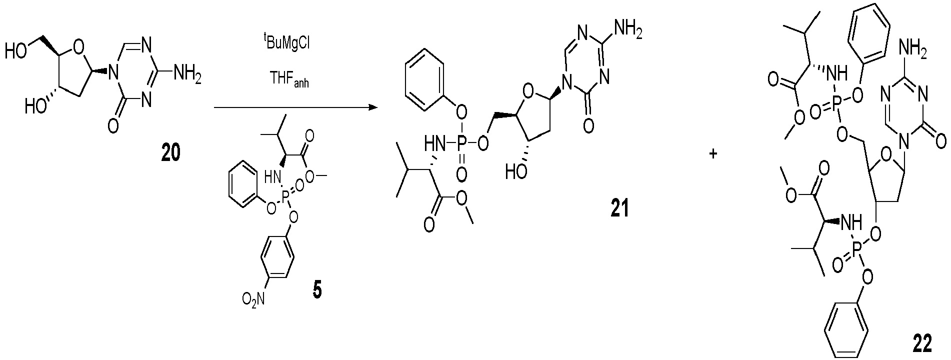

Following the procedure described for compound 9, using decitabine (compound 3), 20 mg, 0.088 mmol) and methyl ((4-nitrophenoxy)(phenoxy)phosphoryl)-L-valinate (compound 5, 47 mg, 0.114 mmol), methyl ((((2R,3S,5R)-5-(4-amino-2-oxo-1,3,5-triazin-1(2H)-yl)-3-hydroxytetrahydrofuran-2-yl)methoxy)(phenoxy)phosphoryl)-L-valinate (compound 21, 10 mg, 22.8 % yield) was obtained.

Simultaneously, methyl ((((2R,3S,5R)-5-(4-amino-2-oxo-1,3,5-triazin-1(2H)-yl)-2-((((((S)-1-methoxy-3-methyl-1-oxobutan-2-yl)amino)(phenoxy)phosphoryl)oxy)methyl)tetrahydrofuran-3-yl)oxy)(phenoxy)phosphoryl)-L-valinate (compound 22, 22 mg) was obtained.

### Compound 21:

- MS-ESI (+): (m/z): [M+Na]⁺ =520, [M+K]⁺ =536
- MS-ESI (-): (m/z): [M-H]⁻=496

### Compound 22:

- MS-ESI (+): (m/z): [M+Na]⁺ =789

### Example 16: Deamination of methyl ((((2R,3S,4R,5R)-5-(4-amino-2-oxopyrimidin-1(2H)-yl)-3,4-dihydroxytetrahydrofuran-2-yl)methoxy)(phenoxy)phosphoryl)-L-leucinate (compound 10) to furnish methyl ((((2R,3S,4R,5R)-5-(2,4-dioxo-3,4-dihydropyrimidin-1(2H)-yl)-3,4-dihydroxytetrahydrofuran-2-yl)methoxy)(phenoxy)phosphoryl)-L-leucinate (compound 10B)

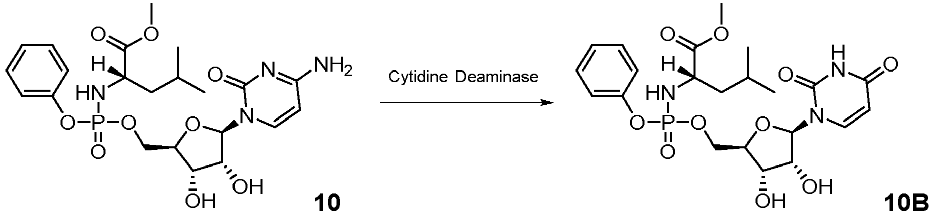

A 20 mM solution of compound 10 (150 µL) in KH₂PO₄ 100 mM pH: 7.0 was mixed with 15 µL of the cytidine deaminase solution containing 90 AU at 37°C. After 5 hours, the reaction was stopped with HCI and a portion of the reaction was diluted and filtered for HPLC and MS analysis. Methyl ((((2R,3S,4R,5R)-5-(2,4-dioxo-3,4-dihydropyrimidin-1(2H)-yl)-3,4-dihydroxytetrahydrofuran-2-yl)methoxy)(phenoxy)phosphoryl)-L-leucinate (compound 10B) was obtained in > 70% yield according to HPLC analysis from the crude mixture. MS-ESI (+): (m/z): [M+H]⁺=528, [M+Na]⁺ =550, [M+K]⁺ =567

MS-ESI (-): (m/z): [M-H]⁻=526

### Example 17: Deamination of methyl ((((2R,3S,4R,5R)-5-(4-amino-2-oxopyrimidin-1(2H)-yl)-3,4-dihydroxytetrahydrofuran-2-yl)methoxy)(phenoxy)phosphoryl)-L-valinate (compound 12) to furnish methyl ((((2R,3S,4R,5R)-5-(2,4-dioxo-3,4-dihydropyrimidin-1(2H)-yl)-3,4-dihydroxytetrahydrofuran-2-yl)methoxy)(phenoxy)phosphoryl)-L-valinate (compound 12B)

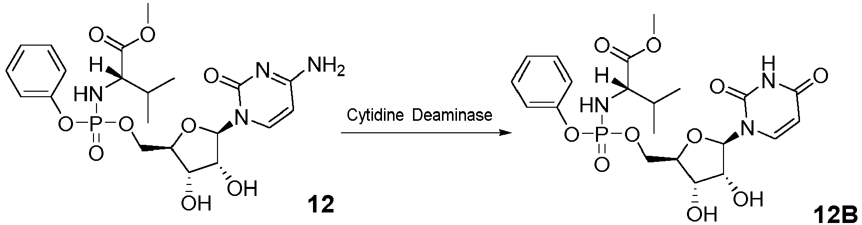

Following the procedure described in Example 16, compound 12, 150 µL of a 20 mM solution in KH₂PO₄ 100 mM pH: 7.0, was deaminated using 15 µL of the cytidine deaminase solution containing 90 AU at 37 °C. After 5 hours, the reaction was stopped with 150 µL of MeOH. An aliquot of the reaction was diluted and filtered for HPLC and MS analysis. Methyl ((((2R,3S,4R,5R)-5-(2,4-dioxo-3,4-dihydropyrimidin-1(2H)-yl)-3,4-dihydroxytetrahydrofuran-2-yl)methoxy)(phenoxy)phosphoryl)-L-valinate (compound 12B) was obtained in >70 % yield (HPLC analysis from crude reaction).

### Example 18: Deamination of isopropyl ((((2R,3S,4R,5R)-5-(4-amino-2-oxopyrimidin-1(2H)-yl)-3,4-dihydroxytetrahydrofuran-2-yl)methoxy)(phenoxy)phosphoryl)-L-alaninate hydrochloride (compound 14) to furnish isopropyl ((((2R,3S,4R,5R)-5-(2,4-dioxo-3,4-dihydropyrimidin-1(2H)-yl)-3,4-dihydroxytetrahydrofuran-2-yl)methoxy)(phenoxy)phosphoryl)-L-alaninate (compound 14B)

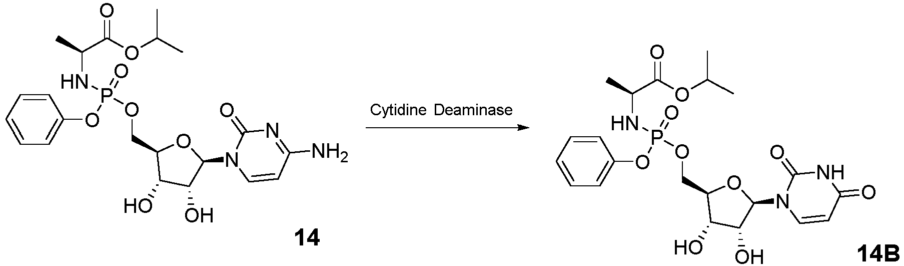

Following the procedure described in Example 16, compound 14, 150 µL of a 20 mM solution in KH₂PO₄ 100 mM pH:7.0 was deaminated using 15 µL of the cytidine deaminase solution containing 90 AU at 37 °C. After 5 hours, the reaction was stopped with 150 µL of MeOH and a portion of the reaction was diluted and filtered for HPLC and MS analysis. Isopropyl ((((2R,3S,4R,5R)-5-(2,4-dioxo-3,4-dihydropyrimidin-1(2H)-yl)-3,4-dihydroxytetrahydrofuran-2-yl)methoxy)(phenoxy)phosphoryl)-L-alaninate (compound 14B) was obtained quantitatively, according to HPLC analysis of the crude reaction.
- MS-ESI (+): (m/z): [M+Na]⁺ =536, [M+K]⁺ =552
- MS-ESI (-): (m/z): [M-H]⁻ =512

### Example 19: Deamination of methyl ((((2R,3S,4R,5R)-5-(4-amino-2-oxopyrimidin-1(2H)-yl)-3,4-dihydroxytetrahydrofuran-2-yl)methoxy)(phenoxy)phosphoryl)-L-alaninate hydrochloride (compound 14-Me) to furnish methyl ((((2R,3S,4R,5R)-5-(2,4-dioxo-3,4-dihydropyrimidin-1(2H)-yl)-3,4-dihydroxytetrahydrofuran-2-yl)methoxy)(phenoxy)phosphoryl)-L-alaninate (compound 14B-Me)

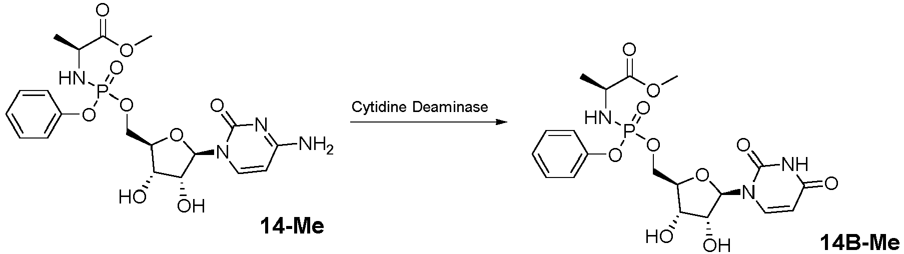

Following the procedure described in Example 16, the deamination process over compound 14-Me was carried out in 150 µL of a 20 mM solution in KH₂PO₄ 100 mM pH: 7.0 using 15 µL of the cytidine deaminase solution containing 90 AU at 37 °C. After 5 hours, the reaction was stopped with 150 µL of MeOH and a portion of the reaction was diluted and filtered for HPLC and MS analysis. Methyl ((((2R,3S,4R,5R)-5-(2,4-dioxo-3,4-dihydropyrimidin-1(2H)-yl)-3,4-dihydroxytetrahydrofuran-2-yl)methoxy)(phenoxy)phosphoryl)-L-alaninate (compound 14B-Me) was obtained in > 80% yield before purification.

MS-ESI (+): (m/z): [M+Na]⁺ =508, [M+K]⁺ =524

MS-ESI (-): (m/z): [M-H]⁻ =484

### Example 20 Deamination of methyl ((((2R,3R,5R)-5-(4-amino-2-oxopyrimidin-1(2H)-yl)-4,4-difluoro-3-hydroxytetrahydrofuran-2-yl)methoxy)(phenoxy)phosphoryl)-L-leucinate (compound 16) to furnish methyl ((((2R,3R,5R)-5-(2,4-dioxo-3,4-dihydropyrimidin-1(2H)-yl)-4,4-difluoro-3-hydroxytetrahydrofuran-2-yl)methoxy)(phenoxy)phosphoryl)-L-leucinate (compound 16B)

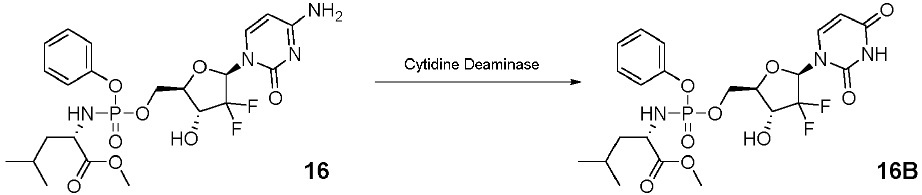

Following the procedure described in previous examples, the deamination process over compound 16 was carried out at 37°C in 250 µL of a 100 mM solution in KH₂PO₄ 100 mM pH: 7.0 using 25 µL of the cytidine deaminase solution containing 150 AU. After 5 hours, the reaction was stopped with HCI and a portion of the reaction was diluted and filtered for HPLC and MS analysis. Methyl ((((2R,3R,5R)-5-(2,4-dioxo-3,4-dihydropyrimidin-1(2H)-yl)-4,4-difluoro-3-hydroxytetrahydrofuran-2-yl)methoxy)(phenoxy)phosphoryl)-L-leucinate (compound 16B) was obtained quantitatively.

MS-ESI (+): (m/z): [M+Na]⁺ =570, [M+K]⁺ =586

MS-ESI (-): (m/z): [M-H]⁻ = 546

### Example 21: Deamination of methyl ((((2R,3R,5R)-5-(4-amino-2-oxopyrimidin-1(2H)-yl)-4,4-difluoro-3-hydroxytetrahydrofuran-2-yl)methoxy)(phenoxy)phosphoryl)-L-valinate (compound 17) to furnish methyl ((((2R,3R,5R)-5-(2,4-dioxo-3,4-dihydropyrimidin-1(2H)-yl)-4,4-difluoro-3-hydroxytetrahydrofuran-2-yl)methoxy)(phenoxy)phosphoryl)-L-valinate (compound 17B)

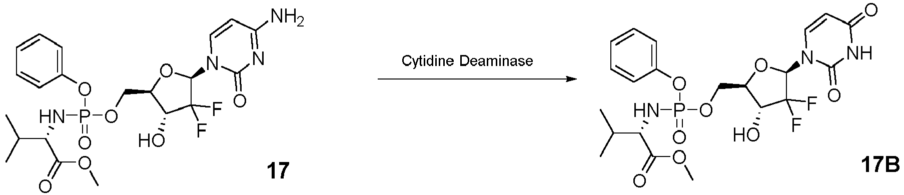

Following the procedure described in previous examples, the deamination process over compound 17 was carried out in 400 µL of a 100 mM solution in KH₂PO₄ 100 mM pH: 7.0 using 40 µL of the cytidine deaminase solution containing 240 AU at 37°C. After 5 hours, the reaction was stopped with HCI and a portion of the reaction was diluted and filtered for HPLC and MS analysis. Methyl ((((2R,3R,5R)-5-(2,4-dioxo-3,4-dihydropyrimidin-1(2H)-yl)-4,4-difluoro-3-hydroxytetrahydrofuran-2-yl)methoxy)(phenoxy)phosphoryl)-L-valinate (compound 17B) was obtained in quantitative yield (according to HPLC analysis of the crude mixture).

MS-ESI (+): (m/z): [M+Na]⁺ =556, [M+K]⁺ =572

MS-ESI (-): (m/z): [M-H] ⁻= 532

### Example 22: Deamination of isopropyl ((((2R,3R,5R)-5-(4-amino-2-oxopyrimidin-1(2H)-yl)-4,4-difluoro-3-hydroxytetrahydrofuran-2-yl)methoxy)(phenoxy)phosphoryl)-L-alaninate (compound 18) to furnish isopropyl ((((2R,3R,5R)-5-(2,4-dioxo-3,4-dihydropyrimidin-1(2H)-yl)-4,4-difluoro-3-hydroxytetrahydrofuran-2-yl)methoxy)(phenoxy)phosphoryl)-L-alaninate (compound 18B)

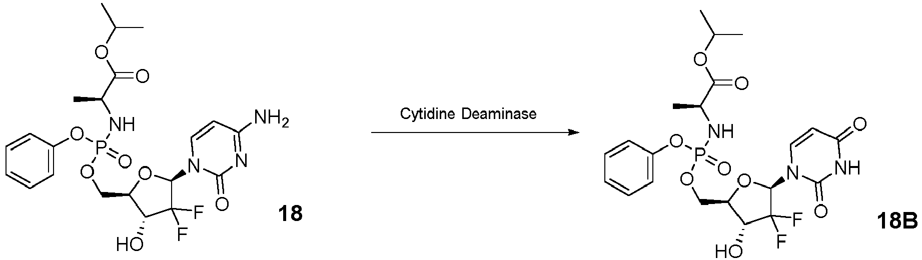

Following the procedure described in previous examples, the deamination process over the compound 18 was carried out in 60 µL of a 100 mM solution in KH₂PO₄ 100 mM pH: 7.0 using 6 µL of the cytidine deaminase solution containing 36 AU at 37°C. After 5 hours, the reaction is stopped with HCI and a portion of the reaction is diluted and filtered for HPLC and MS analysis. Isopropyl ((((2R,3R,5R)-5-(2,4-dioxo-3,4-dihydropyrimidin-1(2H)-yl)-4,4-difluoro-3-hydroxytetrahydrofuran-2-yl)methoxy)(phenoxy)phosphoryl)-L-alaninate (compound 18B) was obtained in quantitative yield.

MS-ESI (+): (m/z): [M+Na]⁺ =556, [M+K]⁺ =572

MS-ESI (-): (m/z): [M-H]⁻ = 532

### Example 23: Deamination of methyl ((((2R,3S,5R)-5-(4-amino-2-oxo-1,3,5-triazin-1(2H)-yl)-3-hydroxytetrahydrofuran-2-yl)methoxy)(phenoxy)phosphoryl)-L-valinate (compound 21) to furnish methyl ((((2R,3S,5R)-5-(2,4-dioxo-3,4-dihydro-1,3,5-triazin-1(2H)-yl)-3-hydroxytetrahydrofuran-2-yl)methoxy)(phenoxy)phosphoryl)-L-valinate (compound 21B)

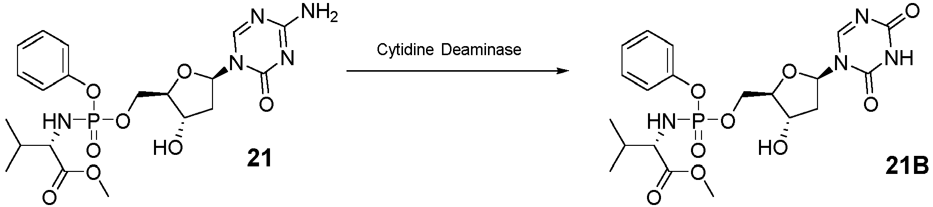

Following the procedure described in previous examples, the deamination process over compound 21 was carried out at pH 6, using 100 µL of a 13 mM solution in KH₂PO₄ 100 mM, and 11 µL of the cytidine deaminase solution containing 66 AU at 37°C. After 24 hours, the reaction was stopped with MeOH and a portion of the reaction was filtered and diluted for HPLC and MS analysis. Methyl ((((2R,3S,5R)-5-(2,4-dioxo-3,4-dihydro-1,3,5-triazin-1(2H)-yl)-3-hydroxytetrahydrofuran-2-yl)methoxy)(phenoxy)phosphoryl)-L-valinate (compound 21B) was obtained in yield higher than 90% (according to HPLC analysis), which was further hydrolyzed *in situ* to methyl ((((2R,3S,5R)-5-(3-carbamoylureido)-3-hydroxytetrahydrofuran-2-yl)methoxy)(phenoxy)phosphoryl)-L-valinate (compound 21D), according to its MS spectrum and characteristic ion fragmentation. Compound 21D:

MS-ESI (+): (m/z): [M+Na]⁺ =511, [M+K]⁺ =527

### Example 24: Deamination of methyl ((((2R,3S,5R)-5-(4-amino-2-oxo-1,3,5-triazin-1(2H)-yl)-3-hydroxytetrahydrofuran-2-yl)methoxy)(phenoxy)phosphoryl)-L-valinate (compound 21) to methyl ((((2R,3S,5R)-5-(2,4-dioxo-3,4-dihydro-1,3,5-triazin-1(2H)-yl)-3-hydroxytetrahydrofuran-2-yl)methoxy)(phenoxy)phosphoryl)-D-valinate (compound 21B)

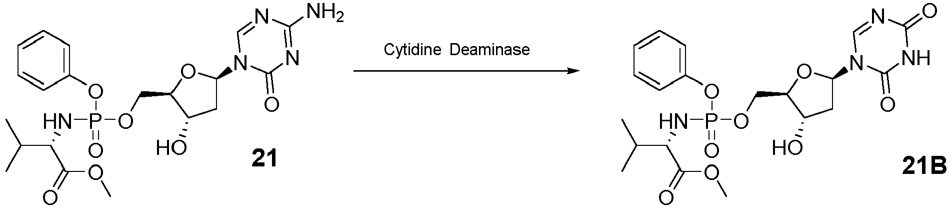

Following the procedure described in previous examples, the deamination process over compound 21 was carried out at pH 7, using 100 µL of a 13 mM solution in KH₂PO₄ 100 mM, and 11 µL of the cytidine deaminase solution containing 66 AU at 37°C. After 24 hours, the reaction was stopped with MeOH and a portion of the reaction was filtered and diluted for HPLC and MS analysis. Compound 21 quantitatively evolved into methyl ((((2R,3S,5R)-5-(2,4-dioxo-3,4-dihydro-1,3,5-triazin-1(2H)-yl)-3-hydroxytetrahydrofuran-2-yl)methoxy)(phenoxy)phosphoryl)-L-valinate (compound 21B), which was further hydrolyzed *in situ* to methyl ((((2R,3S,5R)-5-(3-carbamoylureido)-3-hydroxytetrahydrofuran-2-yl)methoxy)(phenoxy)phosphoryl)-L-valinate (compound 21D), according to its MS spectrum and characteristic ion fragmentation. Compound 21D:
MS-ESI (+): (m/z): [M+Na]⁺ =511, [M+K]⁺ =527

### Example 25: Deamination of methyl ((((2R,3S,5R)-5-(4-amino-2-oxo-1,3,5-triazin-1(2H)-yl)-3-hydroxytetrahydrofuran-2-yl)methoxy)(phenoxy)phosphoryl)-L-valinate (compound 21) to furnish methyl ((((2R,3S,5R)-5-(2,4-dioxo-3,4-dihydro-1,3,5-triazin-1(2H)-yl)-3-hydroxytetrahydrofuran-2-yl)methoxy)(phenoxy)phosphoryl)-L-valinate (compound 21B)

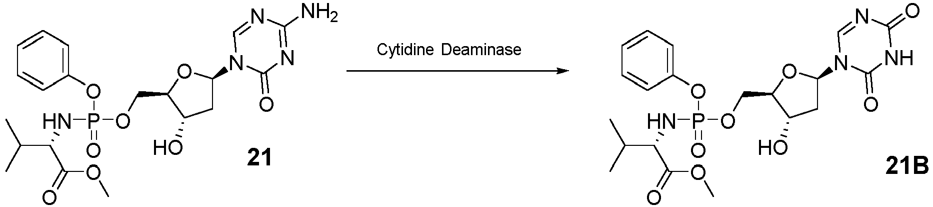

Following the procedure described in previous examples, the deamination process over compound 21 was carried out at pH 8, using 100 µL of a 13 mM solution in KH₂PO₄ 100 mM, and 11 µL of the cytidine deaminase solution containing 66 AU at 37°C. After 24 hours, the reaction was stopped with MeOH and a portion of the reaction was filtered and diluted for HPLC and MS analysis. Compound 21 quantitatively evolved into methyl ((((2R,3S,5R)-5-(2,4-dioxo-3,4-dihydro-1,3,5-triazin-1(2H)-yl)-3-hydroxytetrahydrofuran-2-yl)methoxy)(phenoxy)phosphoryl)-L-valinate (compound 21B), which was further hydrolyzed *in situ* to methyl ((((2R,3S,5R)-5-(3-carbamoylureido)-3-hydroxytetrahydrofuran-2-yl)methoxy)(phenoxy)phosphoryl)-L-valinate (compound 21D), according to its MS spectrum and characteristic ion fragmentation. Compound 21D:
MS-ESI (+): (m/z): [M+Na]⁺ =511, [M+K]⁺ =527

As it has been demonstrated in comparative examples, the deamination of nucleosides using cytidine deaminases works at quantitative conversion and yield (see cytidine (comparative example 1), 2'-deoxycytidine (comparative example 3), cytarabine (comparative example 6) and gemcitabine (comparative example 8)), due to the fact that cytosinic nucleosides bearing no substitution at OH-5' are the natural substrates for cytidine deaminases.

On the other hand, no transformation is obtained when the substrate is the corresponding nucleotide (cytidine 5'-monophosphate (comparative example 2), 2'-deoxycytidine 5'-monophosphate (comparative example 4), cytidine 5'-triphosphate (comparative example 5) and cytarabine 5'-monophosphate (comparative example 7), as it is expected, because the molecules bearing a phosphate substitution at OH-5' are not recognized by cytidine deaminases.

However, inventors have surprisingly found that when the functionality of the nucleoside at OH-5' is in the form of bulky substituted organophosphorus nucleoside, excluding natural nucleotides, deamination reaction takes place at conversions and yields similar to those obtained in the natural non-OH-5' substituted nucleosides (as it is observed in gemcitabine (comparative example 8) and its bulky substituted organophosphorus derivatives compounds 16, 17 and 18 in Examples 20, 21 and 22, respectively). This is a teaching away from what is reported in literature, since some authors have disclosed that nucleosidic substrates incorporating bulky substituents exhibit difficult fitting into the active site of the cytosine deaminase enzymes, and in some cases, they are even inhibitors of this type of enzymes. Therefore, the present invention contributes to a highly efficient synthesis and production method of such compounds of formula I, by means of a biocatalytic deamination of compounds of formula II.

## Claims

1. - Process for preparing a compound of formula I or a pharmaceutically acceptable salt thereof according to the following reaction catalyzed by a nucleoside deaminase, wherein said nucleoside deaminase is a cytidine deaminase: 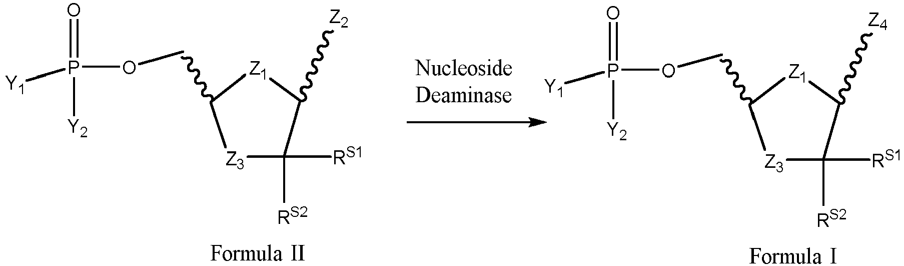 wherein
Z₁ is O;
Z₂ is selected from: 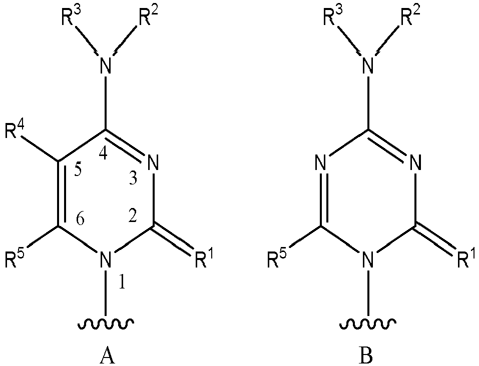
Z₃ is selected, independently of Z₁, from O and C(R^{S3}R^{S4});
Z₄ is selected from: 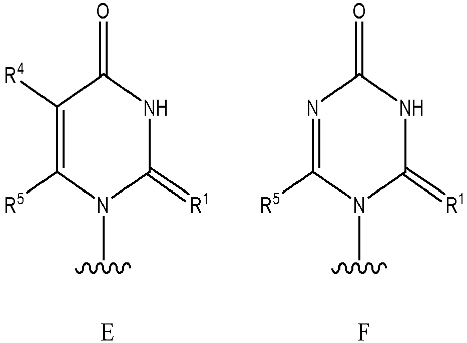 and
R¹ is selected from O, CH₂, alkyl, S and NH;
R² is hydrogen;
R³ is hydrogen;
R⁴ is selected from hydrogen; OH; NH₂; SH; halogen, preferably F, Cl or I; methyl; an optionally substituted alkyl chain; an optionally substituted alkenyl chain; an optionally substituted alkynyl chain; trihaloalkyl; OR⁶; NR⁶R⁷; CN; COR⁶; CONR⁶R⁷; CO₂R⁶; C(S)OR⁶; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; SO₂NR⁶R⁷; an optionally substituted aryl linked to C-5 by an optionally substituted alkyl, alkenyl or alkynyl chain; and an optionally substituted heterocycle linked to C-5 by an optionally substituted alkyl, alkenyl or alkynyl chain;
R⁵ is selected from hydrogen; OH; NH₂; SH; halogen, preferably F, Cl or I; methyl; an optionally substituted alkyl chain; an optionally substituted alkenyl chain; an optionally substituted alkynyl chain; trihaloalkyl; OR⁶; NR⁶R⁷; CN; COR⁶; CONR⁶R⁷; CO₂R⁶; C(S)OR⁶; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; SO₂NR⁶R⁷; an optionally substituted aryl linked to C-6 by an optionally substituted alkyl, alkenyl or alkynyl chain; and an optionally substituted heterocycle linked to C-6 by an optionally substituted alkyl, alkenyl or alkynyl chain;
R⁶ and R⁷ are selected, independently of each other, from hydrogen; an optionally substituted alkyl chain; an optionally substituted alkenyl chain; an optionally substituted alkynyl chain; an optionally substituted heterocycle; and an optionally substituted aryl, preferably phenyl or naphtyl;
R^{S1} is selected, independently of R^{S2}, from hydrogen; halogen, preferably F; methyl; OH; NH₂; SH; N₃; an optionally substituted alkyl chain; an optionally substituted alkenyl chain; an optionally substituted alkynyl chain; trihaloalkyl, OR⁶; NR⁶R⁷; CN; COR⁶; CONR⁶R⁷; CO₂R⁶; C(S)OR⁶; OCONR⁶R⁷; OCOR⁶; OCO₂R⁶; OSO₂R⁶; OC(S)OR⁶; O-Ketal; O-Si-alkyl; O-Si-aryl; NHCONR⁶R⁷; NHCOR⁶; NR⁶CO₂R⁷; NHCO₂R⁶; NHC(S)OR⁶; SO₂NR⁶R⁷; an optionally substituted aryl linked to C-2' by an optionally substituted alkyl, alkenyl or alkynyl chain; and an optionally substituted heterocycle linked to C-2' by an optionally substituted alkyl, alkenyl or alkynyl chain;
R^{S2} is selected, independently of R^{S1}, from hydrogen; halogen, preferably F; methyl; OH; NH₂; SH; N₃; an optionally substituted alkyl chain; an optionally substituted alkenyl chain; an optionally substituted alkynyl chain; trihaloalkyl; OR⁶; NR⁶R⁷; CN; COR⁶; CONR⁶R⁷; CO₂R⁶; C(S)OR⁶; OCONR⁶R⁷; OCOR⁶; OCO₂R⁶; OSO₂R⁶; OC(S)OR⁶; O-Ketal; O-Si-alkyl; O-Si-aryl; NHCONR⁶R⁷; NHCOR⁶; NR⁶CO₂R⁷; NHCO₂R⁶; NHC(S)OR⁶; SO₂NR⁶R⁷; an optionally substituted aryl linked to C-2' by an optionally substituted alkyl, alkenyl or alkynyl chain; and an optionally substituted heterocycle linked to C-2' by an optionally substituted alkyl, alkenyl or alkynyl chain;
R^{S3} is selected, independently of R^{S4}, from hydrogen; OH; halogen, preferably F; methyl; CN; NH₂; SH; C≡CH; N₃; an optionally substituted alkyl chain; an optionally substituted alkenyl chain; an optionally substituted alkynyl chain; and an optionally substituted aryl; an optionally substituted heterocycle; OR⁶; OCONR⁶R⁷; OCOR⁶; OCO₂R⁶; OSO₂R⁶; OC(S)OR⁶; O-Ketal; O-Si-alkyl; and O-Si-aryl;
R^{S4} is selected, independently of R^{S3}, from hydrogen; OH; halogen, preferably F; methyl; CN; NH₂; SH; C≡CH; N₃; an optionally substituted alkyl chain; an optionally substituted alkenyl chain; an optionally substituted alkynyl chain; an optionally substituted aryl; an optionally substituted heterocycle; OR⁶; OCONR⁶R⁷; OCOR⁶; OCO₂R⁶; OSO₂R⁶; OC(S)OR⁶; O-Ketal; O-Si-alkyl; and O-Si-aryl;
Y₁ is selected, independently of Y₂, from OR⁸; NR⁶R⁷; COR⁶; CONR⁶R⁷; CO₂R⁶; C(S)OR⁶; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; SO₂NR⁶R⁷; an optionally substituted alkyl chain; an optionally substituted alkenyl chain; an optionally substituted alkynyl chain; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; an optionally substituted heterocycle optionally linked to P through O or N atoms; an ether of an optionally substituted alkyl chain; an ether of an optionally substituted alkenyl chain; an ether of an optionally substituted alkynyl chain; an ether of an optionally substituted aryl, preferably O-phenyl or O-naphtyl; an ether of an optionally substituted heterocycle; and an amino acid, preferably alanine, valine, leucine or isoleucine, either in the free form or protected by a suitable functional group;
Y₂ is selected, independently of Y₁, from OR⁸; NR⁶R⁷; COR⁶; CONR⁶R⁷; CO₂R⁶; C(S)OR⁶; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; SO₂NR⁶R⁷; an optionally substituted alkyl chain; an optionally substituted alkenyl chain; an optionally substituted alkynyl chain; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; an optionally substituted heterocycle optionally linked to P through O or N atoms; an ether of an optionally substituted alkyl chain; an ether of an optionally substituted alkenyl chain; an ether of an optionally substituted alkynyl chain; an ether of an optionally substituted aryl, preferably O-phenyl or O-naphtyl; an ether of an optionally substituted heterocycle; an amino acid, preferably alanine, valine, leucine or isoleucine, either in the free form or protected by a suitable functional group;
R⁸ is selected from an optionally substituted alkyl chain; an optionally substituted alkenyl chain; an optionally substituted alkynyl chain; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; and an optionally substituted heterocycle optionally linked to P through O or N atoms; aryl, preferably phenyl or naphthyl;
wherein when Z₂ is A, Z₄ is E; and when Z₂ is B, Z₄ is F.

2. Process for preparing a compound of formula I, according to claim 1, wherein
Z₁ is O;
Z₂ is selected from 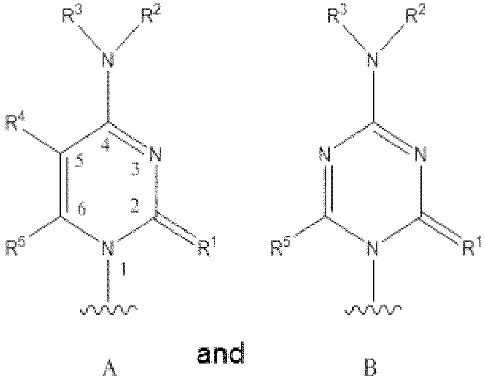
Z₃ is selected, independently of Z₁, from O and C(R^{S3}R^{S4}), more preferably C(R^{S3}R^{S4});
Z₄ is selected from: 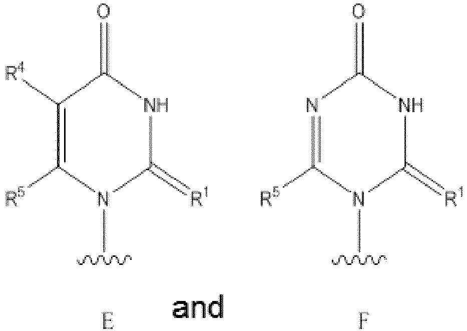
R1 is O;
R² is H;
R³ is H;
R⁴ is selected from H; OH; halogen, preferably F, Cl or I, more preferably F; methyl; trihaloalkyl; OR⁶; COR⁶; CONR⁶R⁷; CO₂R⁶; OCONR⁶R⁷; OCOR⁶; and OCO₂R⁶;
R⁵ is selected from H; OH; halogen, preferably F, Cl or I, more preferably F; methyl; trihaloalkyl; OR⁶; COR⁶; CONR⁶R⁷; CO₂R⁶; OCONR⁶R⁷; OCOR⁶; and OCO₂R⁶;
R^{S1} is selected, independently of R^{S2}, from hydrogen; halogen, preferably F, methyl; OH; OR⁶; NR⁶R⁷; OCONR⁶R⁷; OCOR⁶; OCO₂R⁶; OSO₂R⁶; OC(S)OR⁶; O-Ketal; O-Si-alkyl; and O-Si-aryl;
R^{S2} is selected, independently of R^{S1}, from hydrogen; halogen, preferably F, methyl; OH; OR⁶; NR⁶R⁷; OCONR⁶R⁷; OCOR⁶; OCO₂R⁶; OSO₂R⁶; OC(S)OR⁶; O-Ketal; O-Si-alkyl; and O-Si-aryl;
R^{S3} is selected, independently of R^{S4}, from hydrogen; methyl; OH; OR⁶; OCONR⁶R⁷; OCOR⁶; OCO₂R⁶; OSO₂R⁶; OC(S)OR⁶; O-Ketal; O-Si-alkyl; O-Si-aryl; and halogen, preferably F;
R^{S4} is selected, independently of R^{S3}, from hydrogen; methyl; OH; OR⁶; OCONR⁶R⁷; OCOR⁶; OCO₂R⁶; OSO₂R⁶; OC(S)OR⁶; O-Ketal; O-Si-alkyl; O-Si-aryl; and halogen, preferably F;
Y₁ is selected, independently of Y₂, from OR⁸; NR⁶R⁷; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; an optionally substituted alkyl chain; an optionally substituted alkenyl chain; an optionally substituted alkynyl chain; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; an optionally substituted heterocycle optionally linked to P through O or N atoms; an ether of an optionally substituted alkyl chain; an ether of an optionally substituted alkenyl chain; an ether of an optionally substituted alkynyl chain; an ether of an optionally substituted aryl, preferably O-phenyl or O-naphtyl; an ether of an optionally substituted heterocycle; and an amino acid, preferably alanine, valine, leucine or isoleucine, either in the free form or protected by a suitable functional group;
Y₂ is selected, independently of Y₁, from OR⁸; NR⁶R⁷; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; an optionally substituted alkyl chain; an optionally substituted alkenyl chain; an optionally substituted alkynyl chain; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; an optionally substituted heterocycle optionally linked to P through O or N atoms; an ether of an optionally substituted alkyl chain; an ether of an optionally substituted alkenyl chain; an ether of an optionally substituted alkynyl chain; an ether of an optionally substituted aryl, preferably O-phenyl or O-naphtyl; an ether of an optionally substituted heterocycle; and an amino acid, preferably alanine, valine, leucine or isoleucine, either in the free form or protected by a suitable functional group;
R⁸ is selected from an optionally substituted alkyl chain; an optionally substituted alkenyl chain; an optionally substituted alkynyl chain; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; an optionally substituted heterocycle optionally linked to P through O or N atoms; and aryl, preferably phenyl and naphthyl;
wherein when Z₂ is A, Z₄ is E; and when Z₂ is B, Z₄ is F.

3. Process for preparing a compound of formula I, according to claim 1, wherein
Z₁ is O;
Z₂ is selected from 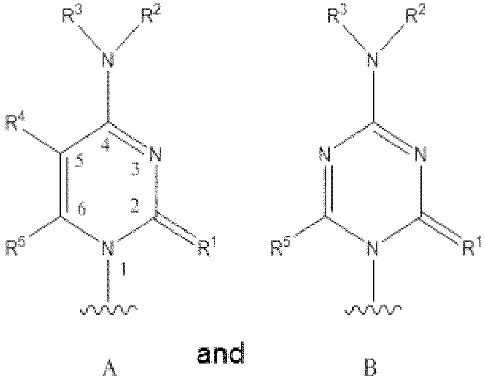
Z₃ is C(R^{S3}R^{S4});
Z₄ is selected from 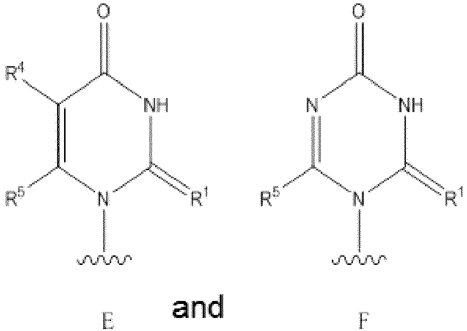
R¹ is O;
R² is H;
R³ is H;
R⁴ is selected from H; OH; halogen, preferably F; methyl and trihaloalkyl;
R⁵ is selected from H; OH; halogen; OR⁶; COR⁶; CONR⁶R⁷; CO₂R⁶; OCONR⁶R⁷; OCOR⁶; and OCO₂R⁶;
R^{S1} is selected, independently of R^{S2}, from hydrogen; halogen, preferably F; methyl; OH; OR⁶; OCONR⁶R⁷; OCOR⁶; OSO₂R⁶; O-Ketal; O-Si-alkyl; and O-Si-aryl;and OCO₂R⁶;
R^{S2} is selected, independently of R^{S1}, from hydrogen; halogen preferably F; methyl; OH; OR⁶; OCONR⁶R⁷; OCOR⁶; OSO₂R⁶; O-Ketal; O-Si-alkyl; and O-Si-aryl; and OCO₂R⁶;
R^{S3} is selected, independently of R^{S4}, from hydrogen; methyl; OH; OR⁶; OCONR⁶R⁷; OCOR⁶; OCO₂R⁶; OSO₂R⁶; OC(S)OR⁶; O-Ketal; O-Si-alkyl; O-Si-aryl; and halogen, preferably F;
R^{S4} is selected, independently of R^{S3}, from hydrogen; methyl; OH; OR⁶; OCONR⁶R⁷; OCOR⁶; OCO₂R⁶; OSO₂R⁶; OC(S)OR⁶; O-Ketal; O-Si-alkyl; O-Si-aryl; and halogen, preferably F;
Y₁ is selected, independently of Y₂, from OR⁸; NR⁶R⁷; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; an optionally substituted alkyl chain; an optionally substituted alkenyl chain; an optionally substituted alkynyl chain; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; an optionally substituted heterocycle optionally linked to P through O or N atoms; an ether of an optionally substituted alkyl chain; an ether of an optionally substituted alkenyl chain; an ether of an optionally substituted alkynyl chain; an ether of an optionally substituted aryl, preferably O-phenyl or O-naphtyl; an ether of an optionally substituted heterocycle; and an amino acid, preferably alanine, valine, leucine or isoleucine, either in the free form or protected by a suitable functional group;
Y₂ is selected, independently of Y₁, from OR⁸; NR⁶R⁷; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; an optionally substituted alkyl chain; an optionally substituted alkenyl chain; an optionally substituted alkynyl chain; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; an optionally substituted heterocycle optionally linked to P through O or N atoms; an ether of an optionally substituted alkyl chain; an ether of an optionally substituted alkenyl chain; an ether of an optionally substituted alkynyl chain; an ether of an optionally substituted aryl, preferably O-phenyl or O-naphtyl; an ether of an optionally substituted heterocycle; and an amino acid, preferably alanine, valine, leucine or isoleucine, either in the free form or protected by a suitable functional group;
R⁸ is selected from an optionally substituted alkyl chain; an optionally substituted alkenyl chain; an optionally substituted alkynyl chain; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; an optionally substituted heterocycle optionally linked to P through O or N atoms; and aryl, preferably phenyl and naphthyl;
wherein when Z₂ is A, Z₄ is E; and when Z₂ is B, Z₄ is F.

4. Process for preparing a compound of formula I, according to claim 1, wherein
Z₁ is O;
Z₂ is selected from 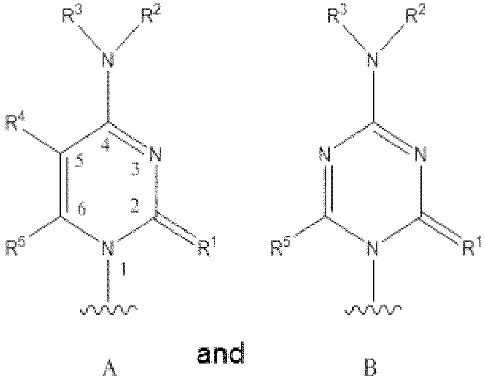
Z₃ is C(R^{S3}R^{S4}) wherein R^{S3} is H or OH and wherein R^{S4} is, independently of R^{S3}, H or OH;
Z₄ is selected from 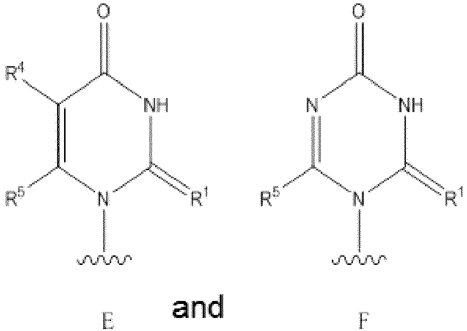
R1 is O;
R² is H;
R³ is H;
R⁴ is H; methyl or halogen, preferably F;
R⁵ is H;
R^{S1} is selected, independently of R^{S2}, from hydrogen; halogen, preferably F; methyl; and OH; R^{S2} is selected, independently of R^{S1}, from hydrogen; halogen, preferably F; methyl; and OH; Y₁ is selected, independently of Y₂, from OR⁸; an ether of an optionally substituted aryl, preferably O-phenyl or O-naphtyl; an ether of an optionally substituted heterocycle; and an amino acid, preferably alanine, valine, leucine or isoleucine, either in the free form or protected by a suitable functional group;
Y₂ is selected, independently of Y₁, from OR⁸; NR⁶R⁷; NHCONR⁶R⁷; NHCOR⁶; NR⁶CO₂R⁷; NHCO₂R⁶; NHC(S)OR⁶; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; an optionally substituted heterocycle optionally linked to P through O or N atoms; an ether of an optionally substituted alkyl chain; an ether of an optionally substituted alkenyl chain; an ether of an optionally substituted alkynyl chain; an ether of an optionally substituted aryl, preferably O-phenyl or O-naphtyl; an ether of an optionally substituted heterocycle; and an amino acid, preferably alanine, valine, leucine or isoleucine, either in the free form or protected by a suitable functional group;
R⁸ is selected from an optionally substituted alkyl chain; an optionally substituted alkenyl chain; an optionally substituted alkynyl chain; an optionally substituted cycloalkyl chain optionally linked to P through O or N atoms; an optionally substituted cycloalkenyl chain optionally linked to P through O or N atoms; an optionally substituted cycloalkynyl chain optionally linked to P through O or N atoms; an optionally substituted aryl optionally linked to P through O or N atoms; an optionally substituted heterocycle optionally linked to P through O or N atoms; and aryl, preferably phenyl and naphthyl;
wherein when Z₂ is A, Z₄ is E; and when Z₂ is B, Z₄ is F.

5. Process for preparing a compound of formula I, according to claim 4, wherein
R⁴ is H;
Y₁ is selected, independently of Y₂, from OR⁸; an ether of an optionally substituted aryl, preferably O-phenyl or O-naphtyl; an ether of an optionally substituted heterocycle; and an amino acid, preferably alanine, valine, leucine or isoleucine, either in the free form or protected by a suitable functional group; and
Y₂ is selected, independently of Y₁, from an ether of an optionally substituted aryl, preferably O-phenyl or O-naphtyl; and an amino acid, preferably alanine, valine, leucine or isoleucine, either in the free form or protected by a suitable functional group.

6. Process for preparing a compound of formula I, according to claim 4, wherein
Y₁ is selected, independently of Y₂, from an ether of an optionally substituted aryl, preferably O-phenyl; and an amino acid, preferably alanine, valine, leucine or isoleucine, either in the free form or protected by a suitable functional group; and
Y₂ is selected, independently of Y₁, from an ether of an optionally substituted aryl, preferably O-phenyl; and an amino acid, preferably alanine, valine, leucine or isoleucine, either in the free form or protected by a suitable group.

7. Process, according to any of the preceding claims, wherein said process is carried out at a temperature ranging from 18 to 100 °C.

8. Process, according to any of the preceding claims, wherein the medium pH ranges from 3 to 12.

9. Process, according to any of the preceding claims, wherein the concentration of compound of formula II or a pharmaceutically acceptable salt thereof ranges from 0.1 mM to 500 M.

10. Process, according to any of the preceding claims, wherein the amount of enzyme having cytidine deaminase activity ranges from 0.001 to 10000 mg/ml, preferably from 0.001 to 1000 mg/ml.

11. Process, according to any of the preceding claims, wherein the amount of enzyme having cytidine deaminase activity ranges from 0.001 to 10000 AU/micromol substrate, preferably from 0.001 to 100 AU/micromol substrate.

12. Process, according to any of the preceding claims, wherein the reaction medium is aqueous optionally also containing up to 50%, preferably up to 30% and more preferably up to 15% of a suitable organic solvent.

13. Process, according to claim 12, wherein said organic solvent is selected from methanol, ethanol, propanol, isopropanol, t-butanol, n-butanol, ethyl acetate, isopropyl acetate, butyl acetate, dichloromethane, toluene, tetrahydrofuran, 2-methyltetrahydrofuran, acetonitrile, acetone, cyclopentyl methyl ether, methyl ethyl ketone, methyl isobutyl ketone, dimethylamide, dimethylformamide and dimethylsulfoxide.

14. Compound of formula II: 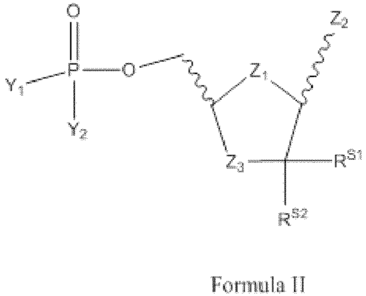 wherein:
Z₁ is O, Z₂ is A, Z₃ is CH-OPG, R^{S1} is H, R^{S2} is OPG, Y₁ is O-Ph and Y₂ is Leu-methyl ester;
or
Z₁ is O, Z₂ is A, Z₃ is CHOH, R^{S1} is H, R^{S2} is OH, Y₁ is O-Ph and Y₂ is Leu-methyl ester;
or
Z₁ is O, Z₂ is A, Z₃ is CH-OPG, R^{S1} is H, R^{S2} is OPG, Y₁ is O-Ph and Y₂ is Val-methyl ester;
or
Z₁ is O, Z₂ is A, Z₃ is CHOH, R^{S1} is H, R^{S2} is OH, Y₁ is O-Ph and Y₂ is Val-methyl ester;
or
Z₁ is O, Z₂ is A, Z₃ is CH-OPG, R^{S1} is H, R^{S2} is OPG, Y₁ is O-Ph and Y₂ is Ala-isopropyl ester;
or
Z₁ is O, Z₂ is A, Z₃ is CHOH, R^{S1} is H, R^{S2} is OH, Y₁ is O-Ph and Y₂ is Ala-isopropyl ester;
or
Z₁ is O, Z₂ is A, Z₃ is CHOH, R^{S1} is H, R^{S2} is OH, Y₁ is O-Ph and Y₂ is Ala-methyl ester;
or
Z₁ is O, Z₂ is A, Z₃ is CHOH, R^{S1} is F, R^{S2} is F, Y₁ is O-Ph and Y₂ is Leu-methyl ester;
or
Z₁ is O, Z₂ is A, Z₃ is CHOH, R^{S1} is F, R^{S2} is F, Y₁ is O-Ph and Y₂ is Val-methyl ester;
or
Z₁ is O, Z₂ is B, Z₃ is CHOH, R^{S1} is H, R^{S2} is H, Y₁ is O-Ph and Y₂ is Val-methyl ester;
or
Z₁ is O, Z₂ is B, Z₃ is CHOPOY₁Y₂, R^{S1} is H, R^{S2} is H, Y₁ is O-Ph and Y₂ is Val-methyl ester;
wherein:
A is 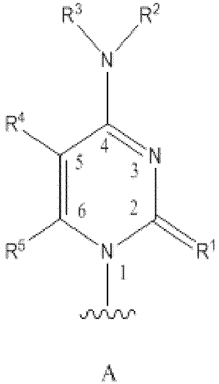
being R¹ = O, R² = R³ = R⁴ = R⁵ = H;
B is 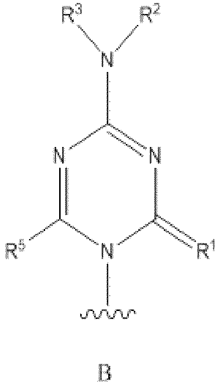
being R¹ = O, R² = R³ = R⁵ = H;
PG is a protecting group, preferably a cyclic acetal or ketal.

15. Compound of formula I: 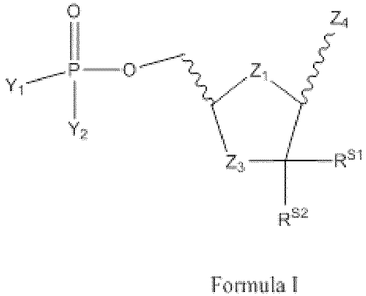 wherein:
Z₁ is O, Z₃ is CH-OH, Z₄ is E, R^{S1} is H, R^{S2} is OH, Y₁ is O-Ph and Y₂ is Leu-methyl ester;
or
Z₁ is O, Z₃ is CH-OH, Z₄ is E, R^{S1} is H, R^{S2} is OH, Y₁ is O-Ph and Y₂ is Val-methyl ester;
or
Z₁ is O, Z₃ is CH-OH, Z₄ is E, R^{S1} is H, R^{S2} is OH, Y₁ is O-Ph and Y₂ is Ala-isopropyl ester;
or
Z₁ is O, Z₃ is CH-OH, Z₄ is E, R^{S1} is H, R^{S2} is OH, Y₁ is O-Ph and Y₂ is Ala-methyl ester;
or
Z₁ is O, Z₃ is CH-OH, Z₄ is E, R^{S1} is F, R^{S2} is F, Y₁ is O-Ph and Y₂ is Leu-methyl ester;
or
Z₁ is O, Z₃ is CH-OH, Z₄ is E, R^{S1} is F, R^{S2} is F, Y₁ is O-Ph and Y₂ is Val-methyl ester;
or
Z₁ is O, Z₃ is CH-OH, Z₄ is E, R^{S1} is F, R^{S2} is F, Y₁ is O-Ph and Y₂ is Ala-isopropyl ester;
or
Z₁ is O, Z₃ is CH-OH, Z₄ is F, R^{S1} is H, R^{S2} is H, Y₁ is O-Ph and Y₂ is Val-methyl ester;
wherein:
E is 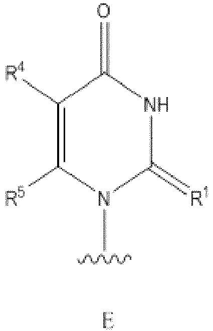
being R¹ = O, R⁴ = R⁵ = H; and
F is 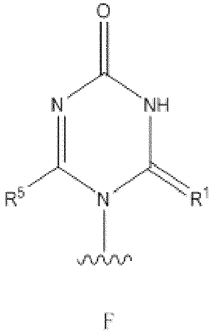
being R¹ = O, R⁵ = H.

## Patentansprüche

1. Verfahren zum Herstellen einer Verbindung einer Formel I oder eines pharmazeutisch akzeptablen Salzes derselben gemäß der folgenden Reaktion, die durch eine Nucleosid-Deaminase katalysiert wird, wobei die Nucleosid-Deaminase eine Cytidin-Deaminase ist: 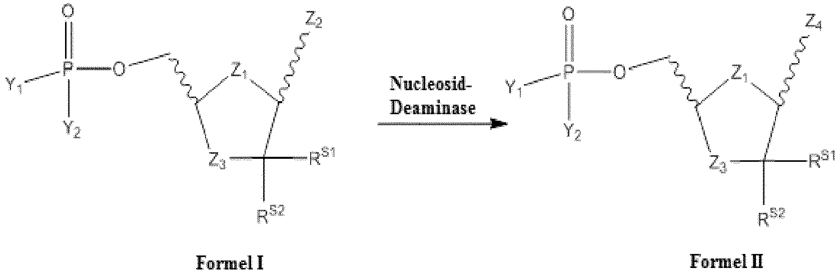 wobei
Z₁ O ist;
Z₂ aus Folgenden ausgewählt ist: 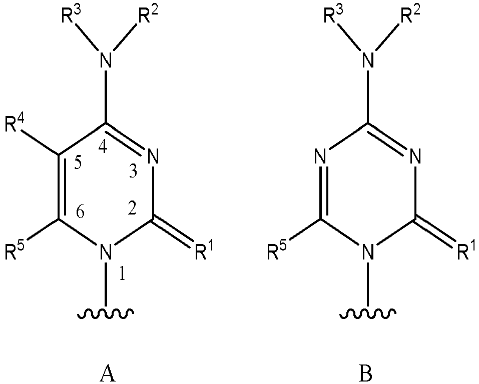
Z₃ unabhängig von Z₁ aus O und C(R^{S3}R^{S4}) ausgewählt ist;
Z₄ aus Folgenden ausgewählt ist: 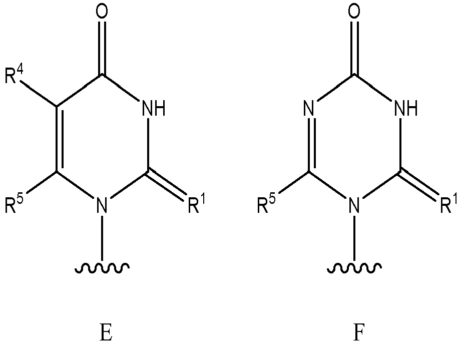 und
R¹ aus O, CH₂, Alkyl, S und NH ausgewählt ist;
R² Wasserstoff ist;
R³ Wasserstoff ist;
R⁴ aus Wasserstoff; OH; NH₂; SH; Halogen, vorzugsweise F, Cl oder I; Methyl; einer optional substituierten Alkylkette; einer optional substituierten Alkenylkette; einer optional substituierten Alkynylkette; Trihaloalkyl; OR⁶; NR⁶R⁷; CN; COR⁶; CONR⁶R⁷; CO₂R⁶; C(S)OR⁶; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; SO₂NR⁶R⁷; und einem optional substituierten Aryl, das durch eine optional substituierte Alkyl-, Alkenyl- oder Alkynylkette an C-5 gebunden ist; und einem optional substituierten Heterocyclus, der durch eine optional substituierte Alkyl-, Alkenyl- oder Alkynylkette an C-5 gebunden ist, ausgewählt ist;
R⁵ aus Wasserstoff; OH; NH₂; SH; Halogen, vorzugsweise F, Cl oder I; Methyl; einer optional substituierten Alkylkette; einer optional substituierten Alkenylkette; einer optional substituierten Alkynylkette; Trihaloalkyl; OR⁶; NR⁶R⁷; CN; COR⁶; CONR⁶R⁷; CO₂R⁶; C(S)OR⁶; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; SO₂NR⁶R⁷; und einem optional substituierten Aryl, das durch eine optional substituierte Alkyl-, Alkenyl- oder Alkynylkette an C-6 gebunden ist; und einem optional substituierten Heterocyclus, der durch eine optional substituierte Alkyl-, Alkenyl- oder Alkynylkette an C-6 gebunden ist, ausgewählt ist;
R⁶ und R⁷ unabhängig voneinander aus Wasserstoff; einer optional substituierten Alkylkette; einer optional substituierten Alkenylkette; einer optional substituierten Alkynylkette; einem optional substituierten Heterocyclus; und einem optional substituierten Aryl, vorzugsweise Phenyl oder Naphthyl, ausgewählt sind;
R^{S1} unabhängig von R^{S2} aus Wasserstoff; Halogen, vorzugsweise F; Methyl; OH; NH₂; SH; N₃; einer optional substituierten Alkylkette; einer optional substituierten Alkenylkette; einer optional substituierten Alkynylkette; Trihaloalkyl, OR⁶; NR⁶R⁷; CN; COR⁶; CONR⁶R⁷; CO₂R⁶; C(S)OR⁶; OCONR⁶R⁷; OCOR⁶; OCO₂R⁶; OSO₂R⁶; OC(S)OR⁶; O-Ketal; O-Si-Alkyl; O-Si-Aryl; NHCONR⁶R⁷; NHCOR⁶; NR⁶CO₂R⁷; NHCO₂R⁶; NHC(S)OR⁶; SO₂NR⁶R⁷; einem optional substituierten Aryl, das durch eine optional substituierte Alkyl-, Alkenyl- oder Alkynylkette an C-2' gebunden ist; und einem optional substituierten Heterocyclus, der durch eine optional substituierte Alkyl-, Alkenyl- oder Alkynylkette an C-2' gebunden ist, ausgewählt ist;
R^{S2} unabhängig von R^{S1} aus Wasserstoff; Halogen, vorzugsweise F; Methyl; OH; NH₂; SH; N₃; einer optional substituierten Alkylkette; einer optional substituierten Alkenylkette; einer optional substituierten Alkynylkette; Trihaloalkyl, OR⁶; NR⁶R⁷; CN; COR⁶; CONR⁶R⁷; CO₂R⁶; C(S)OR⁶; OCONR⁶R⁷; OCOR⁶; OCO₂R⁶; OSO₂R⁶; OC(S)OR⁶; O-Ketal; O-Si-Alkyl; O-Si-Aryl; NHCONR⁶R⁷; NHCOR⁶; NR⁶CO₂R⁷; NHCO₂R⁶; NHC(S)OR⁶; SO₂NR⁶R⁷; einem optional substituierten Aryl, das durch eine optional substituierte Alkyl-, Alkenyl- oder Alkynylkette an C-2' gebunden ist; und einem optional substituierten Heterocyclus, der durch eine optional substituierte Alkyl-, Alkenyl- oder Alkynylkette an C-2' gebunden ist, ausgewählt ist;
R^{S3} unabhängig von R^{S4} aus Wasserstoff; OH; Halogen, vorzugsweise F; Methyl; CN; NH₂; SH; C≡CH; N₃; einer optional substituierten Alkylkette; einer optional substituierten Alkenylkette; einer optional substituierten Alkynylkette; und einem optional substituierten Aryl; einem optional substituierten Heterocyclus; OR⁶; OCONR⁶R⁷; OCOR⁶; OCO₂R⁶; OSO₂R⁶; OC(S)OR⁶; O-Ketal; O-Si-Alkyl; und O-Si-Aryl ausgewählt ist;
R^{S4} unabhängig von R^{S3} aus Wasserstoff; OH; Halogen, vorzugsweise F; Methyl; CN; NH₂; SH; C≡CH; N₃; einer optional substituierten Alkylkette; einer optional substituierten Alkenylkette; einer optional substituierten Alkynylkette; einem optional substituierten Aryl; einem optional substituierten Heterocyclus; OR⁶; OCONR⁶R⁷; OCOR⁶; OCO₂R⁶; OSO₂R⁶; OC(S)OR⁶; O-Ketal; O-Si-Alkyl; und O-Si-Aryl ausgewählt ist;
Y₁ unabhängig von Y₂ aus OR⁸; NR⁶R⁷; COR⁶; CONR⁶R⁷; CO₂R⁶; C(S)OR⁶; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; SO₂NR⁶R⁷; einer optional substituierten Alkylkette; einer optional substituierten Alkenylkette; einer optional substituierten Alkynylkette; einer optional substituierten Cycloalkylkette, die durch O- oder N-Atome optional an P gebunden ist; einer optional substituierten Cycloalkenylkette, die durch O- oder N-Atome optional an P gebunden ist; einer optional substituierten Cycloalkynylkette, die durch O- oder N-Atome optional an P gebunden ist; einem optional substituierten Aryl, das durch O- oder N-Atome optional an P gebunden ist; einem optional substituierten Heterocyclus, der durch O- oder N-Atome optional an P gebunden ist; einem Ether einer optional substituierten Alkylkette; einem Ether einer optional substituierten Alkenylkette; einem Ether einer optional substituierten Alkynylkette; einem Ether eines optional substituierten Aryls, vorzugsweise O-Phenyl oder *O*-Naphthyl; einem Ether eines optional substituierten Heterocyclus; und einer Aminosäure, vorzugsweise Alanin, Valin, Leucin oder Isoleucin, entweder in der freien Form oder geschützt durch eine geeignete funktionelle Gruppe, ausgewählt ist;
Y₂ unabhängig von Y₁ aus OR⁸; NR⁶R⁷; COR⁶; CONR⁶R⁷; CO₂R⁶; C(S)OR⁶; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; SO₂NR⁶R⁷; einer optional substituierten Alkylkette; einer optional substituierten Alkenylkette; einer optional substituierten Alkynylkette; einer optional substituierten Cycloalkylkette, die durch O- oder N-Atome optional an P gebunden ist; einer optional substituierten Cycloalkenylkette, die durch O- oder N-Atome optional an P gebunden ist; einer optional substituierten Cycloalkynylkette, die durch O- oder N-Atome optional an P gebunden ist; einem optional substituierten Aryl, das durch O- oder N-Atome optional an P gebunden ist; einem optional substituierten Heterocyclus, der durch O- oder N-Atome optional an P gebunden ist; einem Ether einer optional substituierten Alkylkette; einem Ether einer optional substituierten Alkenylkette; einem Ether einer optional substituierten Alkynylkette; einem Ether eines optional substituierten Aryls, vorzugsweise O-Phenyl oder *O*-Naphthyl; einem Ether eines optional substituierten Heterocyclus; einer Aminosäure, vorzugsweise Alanin, Valin, Leucin oder Isoleucin, entweder in der freien Form oder geschützt durch eine geeignete funktionelle Gruppe, ausgewählt ist;
R⁸ aus einer optional substituierten Alkylkette; einer optional substituierten Alkenylkette; einer optional substituierten Alkynylkette; einer optional substituierten Cycloalkylkette, die durch O- oder N-Atome optional an P gebunden ist; einer optional substituierten Cycloalkenylkette, die durch O- oder N-Atome optional an P gebunden ist; einer optional substituierten Cycloalkynylkette, die durch O- oder N-Atome optional an P gebunden ist; einem optional substituierten Aryl, das durch O- oder N-Atome optional an P gebunden ist; und einem optional substituierten Heterocyclus, der durch O- oder N-Atome optional an P gebunden ist; Aryl, vorzugsweise Phenyl oder Naphthyl; ausgewählt ist;
wobei dann, wenn Z₂ A ist, Z₄ E ist; und dann, wenn Z₂ B ist, Z₄ F ist.

2. Verfahren zum Herstellen einer Verbindung der Formel I gemäß Anspruch 1, wobei
Z₁ O ist;
Z₂ aus 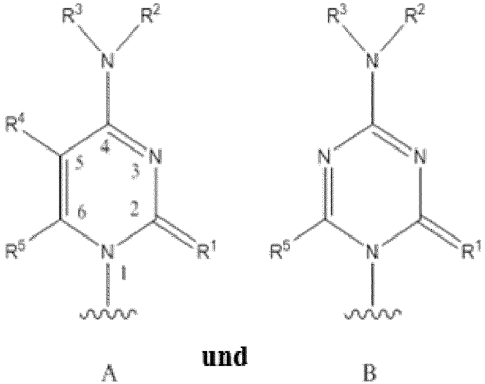
ausgewählt ist,
Z₃ unabhängig von Z₁ aus O und C(R^{S3}R^{S4}), stärker bevorzugt C(R^{S3}R^{S4}), ausgewählt ist;
Z₄ aus 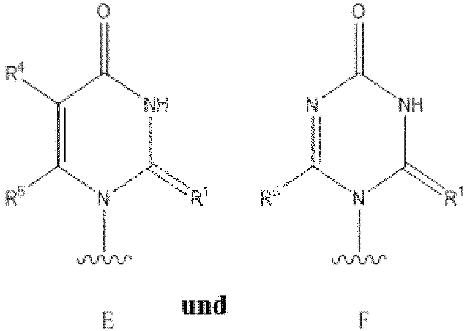
ausgewählt ist,
R¹ O ist;
R² H ist;
R³ H ist;
R⁴ aus H; OH; Halogen, vorzugsweise F, Cl oder I, stärker bevorzugt F; Methyl; Trihaloalkyl; OR⁶; COR⁶; CONR⁶R⁷; CO₂R⁶; OCONR⁶R⁷; OCOR⁶; und OCO₂R⁶ ausgewählt ist;
R⁵ aus H; OH; Halogen, vorzugsweise F, Cl oder I, stärker bevorzugt F; Methyl; Trihaloalkyl; OR⁶; COR⁶; CONR⁶R⁷; CO₂R⁶; OCONR⁶R⁷; OCOR⁶; und OCO₂R⁶ ausgewählt ist;
R^{S1} unabhängig von R^{S2} aus Wasserstoff; Halogen, vorzugsweise F, Methyl; OH; OR⁶; NR⁶R⁷; OCONR⁶R⁷; OCOR⁶; OCO₂R⁶; OSO₂R⁶; OC(S)OR⁶; O-Ketal; O-Si-Alkyl; und O-Si-Aryl ausgewählt ist;
R^{S2} unabhängig von R^{S1} aus Wasserstoff; Halogen, vorzugsweise F, Methyl; OH; OR⁶; NR⁶R⁷; OCONR⁶R⁷; OCOR⁶; OCO₂R⁶; OSO₂R⁶; OC(S)OR⁶; O-Ketal; O-Si-Alkyl; und O-Si-Aryl ausgewählt ist;
R^{S3} unabhängig von R^{S4} aus Wasserstoff; Methyl; OH; OR⁶; OCONR⁶R⁷; OCOR⁶; OCO₂R⁶; OSO₂R⁶; OC(S)OR⁶; O-Ketal; O-Si-Alkyl; O-Si-Aryl; und Halogen, vorzugsweise F, ausgewählt ist;
R^{S4} unabhängig von R^{S3} aus Wasserstoff; Methyl; OH; OR⁶; OCONR⁶R⁷; OCOR⁶; OCO₂R⁶; OSO₂R⁶; OC(S)OR⁶; O-Ketal; O-Si-Alkyl; O-Si-Aryl; und Halogen, vorzugsweise F, ausgewählt ist;
Y₁ unabhängig von Y₂ aus OR⁸; NR⁶R⁷; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; einer optional substituierten Alkylkette; einer optional substituierten Alkenylkette; einer optional substituierten Alkynylkette; einer optional substituierten Cycloalkylkette, die durch O- oder N-Atome optional an P gebunden ist; einer optional substituierten Cycloalkenylkette, die durch O- oder N-Atome optional an P gebunden ist; einer optional substituierten Cycloalkynylkette, die durch O- oder N-Atome optional an P gebunden ist; einem optional substituierten Aryl, das durch O- oder N-Atome optional an P gebunden ist; einem optional substituierten Heterocyclus, der durch O- oder N-Atome optional an P gebunden ist; einem Ether einer optional substituierten Alkylkette; einem Ether einer optional substituierten Alkenylkette; einem Ether einer optional substituierten Alkynylkette; einem Ether eines optional substituierten Aryls, vorzugsweise O-Phenyl oder *O*-Naphthyl; einem Ether eines optional substituierten Heterocyclus; und einer Aminosäure, vorzugsweise Alanin, Valin, Leucin oder Isoleucin, entweder in der freien Form oder geschützt durch eine geeignete funktionelle Gruppe, ausgewählt ist;
Y₂ unabhängig von Y₁ aus OR⁸; NR⁶R⁷; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; einer optional substituierten Alkylkette; einer optional substituierten Alkenylkette; einer optional substituierten Alkynylkette; einer optional substituierten Cycloalkylkette, die durch O- oder N-Atome optional an P gebunden ist; einer optional substituierten Cycloalkenylkette, die durch O- oder N-Atome optional an P gebunden ist; einer optional substituierten Cycloalkynylkette, die durch O- oder N-Atome optional an P gebunden ist; einem optional substituierten Aryl, das durch O- oder N-Atome optional an P gebunden ist; einem optional substituierten Heterocyclus, der durch O- oder N-Atome optional an P gebunden ist; einem Ether einer optional substituierten Alkylkette; einem Ether einer optional substituierten Alkenylkette; einem Ether einer optional substituierten Alkynylkette; einem Ether eines optional substituierten Aryls, vorzugsweise O-Phenyl oder *O*-Naphthyl; einem Ether eines optional substituierten Heterocyclus; und einer Aminosäure, vorzugsweise Alanin, Valin, Leucin oder Isoleucin, entweder in der freien Form oder geschützt durch eine geeignete funktionelle Gruppe, ausgewählt ist;
R⁸ aus einer optional substituierten Alkylkette; einer optional substituierten Alkenylkette; einer optional substituierten Alkynylkette; einer optional substituierten Cycloalkylkette, die durch O- oder N-Atome optional an P gebunden ist; einer optional substituierten Cycloalkenylkette, die durch O- oder N-Atome optional an P gebunden ist; einer optional substituierten Cycloalkynylkette, die durch O- oder N-Atome optional an P gebunden ist; einem optional substituierten Aryl, das durch O- oder N-Atome optional an P gebunden ist; und einem optional substituierten Heterocyclus, der durch O- oder N-Atome optional an P gebunden ist; und Aryl, vorzugsweise Phenyl und Naphthyl, ausgewählt ist;
wobei dann, wenn Z₂ A ist, Z₄ E ist; und dann, wenn Z₂ B ist, Z₄ F ist.

3. Verfahren zum Herstellen einer Verbindung der Formel I gemäß Anspruch 1, wobei
Z₁ O ist;
Z₂ aus 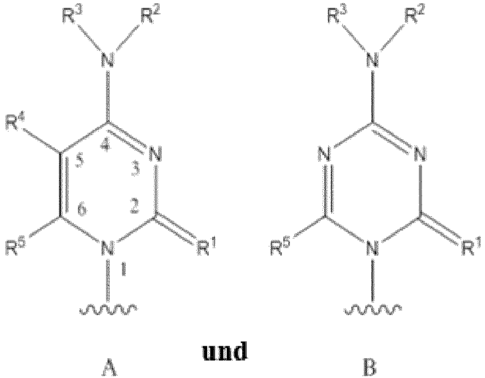
ausgewählt ist,
Z₃ C(R^{S3}R^{S4}) ist;
Z₄ aus 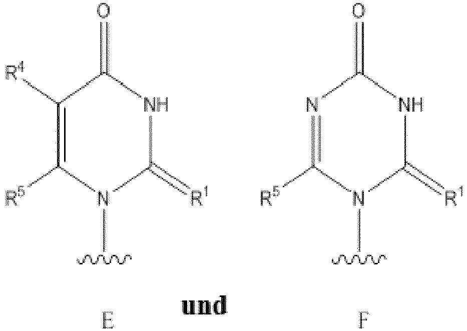
ausgewählt ist,
R¹ O ist;
R² H ist;
R³ H ist;
R⁴ aus H; OH; Halogen, vorzugsweise F; Methyl und Trihaloalkyl ausgewählt ist;
R⁵ aus H; OH; Halogen; OR⁶; COR⁶; CONR⁶R⁷; CO₂R⁶; OCONR⁶R⁷; OCOR⁶; und OCO₂R⁶ ausgewählt ist;
R^{S1} unabhängig von R^{S2} aus Wasserstoff; Halogen, vorzugsweise F; Methyl; OH; OR⁶; OCONR⁶R⁷; OCOR⁶; OSO₂R⁶; O-Ketal; O-Si-Alkyl; und O-Si-Aryl; und OCO₂R⁶ ausgewählt ist;
R^{S2} unabhängig von R^{S1} aus Wasserstoff; Halogen, vorzugsweise F; Methyl; OH; OR⁶; OCONR⁶R⁷; OCOR⁶; OSO₂R⁶; O-Ketal; O-Si-Alkyl; und O-Si-Aryl; und OCO₂R⁶ ausgewählt ist;
R^{S3} unabhängig von R^{S4} aus Wasserstoff; Methyl; OH; OR⁶; OCONR⁶R⁷; OCOR⁶; OCO₂R⁶; OSO₂R⁶; OC(S)OR⁶; O-Ketal; O-Si-Alkyl; O-Si-Aryl; und Halogen, vorzugsweise F, ausgewählt ist;
R^{S4} unabhängig von R^{S3} aus Wasserstoff; Methyl; OH; OR⁶; OCONR⁶R⁷; OCOR⁶; OCO₂R⁶; OSO₂R⁶; OC(S)OR⁶; O-Ketal; O-Si-Alkyl; O-Si-Aryl; und Halogen, vorzugsweise F, ausgewählt ist;
Y₁ unabhängig von Y₂ aus OR⁸; NR⁶R⁷; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; einer optional substituierten Alkylkette; einer optional substituierten Alkenylkette; einer optional substituierten Alkynylkette; einer optional substituierten Cycloalkylkette, die durch O- oder N-Atome optional an P gebunden ist; einer optional substituierten Cycloalkenylkette, die durch O- oder N-Atome optional an P gebunden ist; einer optional substituierten Cycloalkynylkette, die durch O- oder N-Atome optional an P gebunden ist; einem optional substituierten Aryl, das durch O- oder N-Atome optional an P gebunden ist; einem optional substituierten Heterocyclus, der durch O- oder N-Atome optional an P gebunden ist; einem Ether einer optional substituierten Alkylkette; einem Ether einer optional substituierten Alkenylkette; einem Ether einer optional substituierten Alkynylkette; einem Ether eines optional substituierten Aryls, vorzugsweise O-Phenyl oder *O*-Naphthyl; einem Ether eines optional substituierten Heterocyclus; und einer Aminosäure, vorzugsweise Alanin, Valin, Leucin oder Isoleucin, entweder in der freien Form oder geschützt durch eine geeignete funktionelle Gruppe, ausgewählt ist;
Y₂ unabhängig von Y₁ aus OR⁸; NR⁶R⁷; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; einer optional substituierten Alkylkette; einer optional substituierten Alkenylkette; einer optional substituierten Alkynylkette; einer optional substituierten Cycloalkylkette, die durch O- oder N-Atome optional an P gebunden ist; einer optional substituierten Cycloalkenylkette, die durch O- oder N-Atome optional an P gebunden ist; einer optional substituierten Cycloalkynylkette, die durch O- oder N-Atome optional an P gebunden ist; einem optional substituierten Aryl, das durch O- oder N-Atome optional an P gebunden ist; einem optional substituierten Heterocyclus, der durch O- oder N-Atome optional an P gebunden ist; einem Ether einer optional substituierten Alkylkette; einem Ether einer optional substituierten Alkenylkette; einem Ether einer optional substituierten Alkynylkette; einem Ether eines optional substituierten Aryls, vorzugsweise *O*-Phenyl oder *O*-Naphthyl; einem Ether eines optional substituierten Heterocyclus; und einer Aminosäure, vorzugsweise Alanin, Valin, Leucin oder Isoleucin, entweder in der freien Form oder geschützt durch eine geeignete funktionelle Gruppe, ausgewählt ist;
R⁸ aus einer optional substituierten Alkylkette; einer optional substituierten Alkenylkette; einer optional substituierten Alkynylkette; einer optional substituierten Cycloalkylkette, die durch O- oder N-Atome optional an P gebunden ist; einer optional substituierten Cycloalkenylkette, die durch O- oder N-Atome optional an P gebunden ist; einer optional substituierten Cycloalkynylkette, die durch O- oder N-Atome optional an P gebunden ist; einem optional substituierten Aryl, das durch O- oder N-Atome optional an P gebunden ist; und einem optional substituierten Heterocyclus, der durch O- oder N-Atome optional an P gebunden ist; und Aryl, vorzugsweise Phenyl und Naphthyl, ausgewählt ist;
wobei dann, wenn Z₂ A ist, Z₄ E ist; und dann, wenn Z₂ B ist, Z₄ F ist.

4. Verfahren zum Herstellen einer Verbindung der Formel I gemäß Anspruch 1, wobei
Z₁ O ist;
Z₂ aus 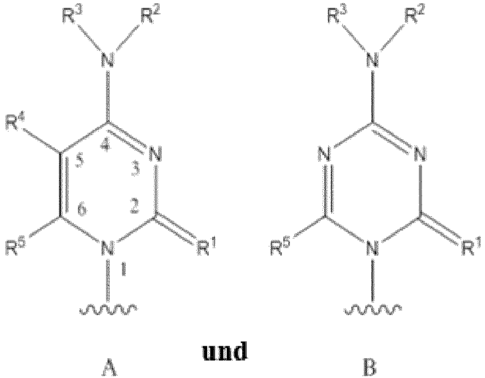
ausgewählt ist,
Z₃ C(R^{S3}R^{S4}) ist, wobei R^{S3} H oder OH ist und wobei R^{S4} unabhängig von R^{S3} H oder OH ist;
Z₄ aus 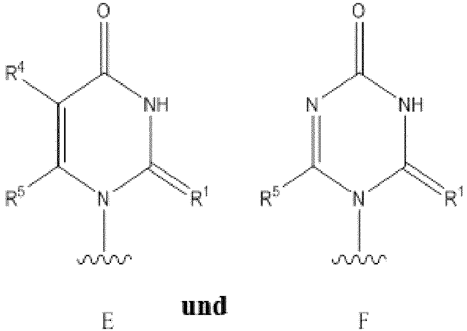
ausgewählt ist,
R¹ O ist;
R² H ist;
R³ H ist;
R⁴ H; Methyl oder Halogen, vorzugsweise F, ist;
R⁵ H ist;
R^{S1} unabhängig von R^{S2} aus Wasserstoff; Halogen, vorzugsweise F; Methyl und OH ausgewählt ist;
R^{S2} unabhängig von R^{S1} aus Wasserstoff; Halogen, vorzugsweise F; Methyl und OH ausgewählt ist;
Y₁ unabhängig von Y₂ aus OR⁸; einem Ether eines optional substituierten Aryls, vorzugsweise *O*-Phenyl oder *O*-Naphthyl; einem Ether eines optional substituierten Heterocyclus; und einer Aminosäure, vorzugsweise Alanin, Valin, Leucin oder Isoleucin, entweder in der freien Form oder geschützt durch eine geeignete funktionelle Gruppe, ausgewählt ist;
Y₂ unabhängig von Y₁ aus OR⁸; NR⁶R⁷; NHCONR⁶R⁷; NHCOR⁶; NR⁶CO₂R⁷; NHCO₂R⁶; NHC(S)OR⁶; einer optional substituierten Cycloalkylkette, die durch O- oder N-Atome optional an P gebunden ist; einer optional substituierten Cycloalkenylkette, die durch O- oder N-Atome optional an P gebunden ist; einer optional substituierten Cycloalkynylkette, die durch O- oder N-Atome optional an P gebunden ist; einem optional substituierten Aryl, das durch O- oder N-Atome optional an P gebunden ist; einem optional substituierten Heterocyclus, der durch O- oder N-Atome optional an P gebunden ist; einem Ether einer optional substituierten Alkylkette; einem Ether einer optional substituierten Alkenylkette; einem Ether einer optional substituierten Alkynylkette; einem Ether eines optional substituierten Aryls, vorzugsweise *O*-Phenyl oder *O*-Naphthyl; einem Ether eines optional substituierten Heterocyclus; und einer Aminosäure, vorzugsweise Alanin, Valin, Leucin oder Isoleucin, entweder in der freien Form oder geschützt durch eine geeignete funktionelle Gruppe, ausgewählt ist;
R⁸ aus einer optional substituierten Alkylkette; einer optional substituierten Alkenylkette; einer optional substituierten Alkynylkette; einer optional substituierten Cycloalkylkette, die durch O- oder N-Atome optional an P gebunden ist; einer optional substituierten Cycloalkenylkette, die durch O- oder N-Atome optional an P gebunden ist; einer optional substituierten Cycloalkynylkette, die durch O- oder N-Atome optional an P gebunden ist; einem optional substituierten Aryl, das durch O- oder N-Atome optional an P gebunden ist; und einem optional substituierten Heterocyclus, der durch O- oder N-Atome optional an P gebunden ist; und Aryl, vorzugsweise Phenyl und Naphthyl, ausgewählt ist;
wobei dann, wenn Z₂ A ist, Z₄ E ist; und dann, wenn Z₂ B ist, Z₄ F ist.

5. Verfahren zum Herstellen einer Verbindung der Formel I gemäß Anspruch 4, wobei
R⁴ H ist;
Y₁ unabhängig von Y₂ aus OR⁸; einem Ether eines optional substituierten Aryls, vorzugsweise *O*-Phenyl oder *O*-Naphthyl; einem Ether eines optional substituierten Heterocyclus; und einer Aminosäure, vorzugsweise Alanin, Valin, Leucin oder Isoleucin, entweder in der freien Form oder geschützt durch eine geeignete funktionelle Gruppe, ausgewählt ist; und
Y₂ unabhängig von Y₁ aus einem Ether eines optional substituierten Aryls, vorzugsweise *O*-Phenyl oder *O*-Naphthyl; und einer Aminosäure, vorzugsweise Alanin, Valin, Leucin oder Isoleucin, entweder in der freien Form oder geschützt durch eine geeignete funktionelle Gruppe, ausgewählt ist.

6. Verfahren zum Herstellen einer Verbindung der Formel I gemäß Anspruch 4, wobei
Y₁ unabhängig von Y₂ aus einem Ether eines optional substituierten Aryls, vorzugsweise *O*-Phenyl; und einer Aminosäure, vorzugsweise Alanin, Valin, Leucin oder Isoleucin, entweder in der freien Form oder geschützt durch eine geeignete funktionelle Gruppe, ausgewählt ist; und
Y₂ unabhängig von Y₁ aus einem Ether eines optional substituierten Aryls, vorzugsweise O-Phenyl; und einer Aminosäure, vorzugsweise Alanin, Valin, Leucin oder Isoleucin, entweder in der freien Form oder geschützt durch eine geeignete funktionelle Gruppe, ausgewählt ist.

7. Verfahren gemäß einem der vorhergehenden Ansprüche, wobei das Verfahren bei einer Temperatur zwischen 18 und 100 °C ausgeführt wird.

8. Verfahren gemäß einem der vorhergehenden Ansprüche, bei dem der mittlere pH-Wert zwischen 3 und 12 beträgt.

9. Verfahren gemäß einem der vorhergehenden Ansprüche, bei dem die Konzentration der Verbindung der Formel II oder eines pharmazeutisch akzeptablen Salzes derselben zwischen 0,1 mM und 500 M beträgt.

10. Verfahren gemäß einem der vorhergehenden Ansprüche, bei dem die Menge an Enzym, das eine Cytidin-Deaminase-Aktivität aufweist, zwischen 0,001 und 10.000 mg/ml, vorzugsweise zwischen 0,001 und 1.000 mg/ml, beträgt.

11. Verfahren gemäß einem der vorhergehenden Ansprüche, bei dem die Menge an Enzym, das eine Cytidin-Deaminase-Aktivität aufweist, zwischen 0,001 und 10.000 AU/Mikromol Substrat, vorzugsweise zwischen 0,001 und 100 AU/Mikromol Substrat, beträgt.

12. Verfahren gemäß einem der vorhergehenden Ansprüche, bei dem das Reaktionsmedium wässerig ist und optional auch bis zu 50%, vorzugsweise bis zu 30% und stärker bevorzugt bis zu 15% eines geeigneten organischen Lösungsmittels enthält.

13. Verfahren gemäß Anspruch 12, bei dem das organische Lösungsmittel aus Methanol, Ethanol, Propanol, Isopropanol, t-Butanol, n-Butanol, Ethylacetat, Isopro-pylacetat, Butylacetat, Dichlormethan, Toluol, Tetrahydrofuran, 2-Methyltetrahydrofuran, Acetonitril, Aceton, Cyclopentylmethylether, Methylethylketon, Methylisobutylketon, Dimethylamid, Dimethylformamid und Dimethylsulfoxide ausgewählt ist.

14. Verbindung der Formel II: 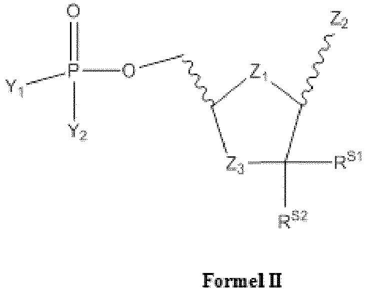 wobei
Z₁ O ist, Z₂ A ist, Z₃ CH-OPG ist, R^{S1} H ist, R^{S2} OPG ist, Y₁ O-Ph ist und Y₂ Leu-Methylester ist;
oder
Z₁ O ist, Z₂ A ist, Z₃ CHOH ist, R^{S1} H ist, R^{S2} OH ist, Y₁ O-Ph ist und Y₂ Leu-Methylester ist;
oder
Z₁ O ist, Z₂ A ist, Z₃ CH-OPG ist, R^{S1} H ist, R^{S2} OPG ist, Y₁ O-Ph ist und Y₂ Val-Methylester ist;
oder
Z₁ O ist, Z₂ A ist, Z₃ CHOH ist, R^{S1} H ist, R^{S2} OH ist, Y₁ O-Ph ist und Y₂ Val-Methylester ist;
oder
Z₁ O ist, Z₂ A ist, Z₃ CH-OPG ist, R^{S1} H ist, R^{S2} OPG ist, Y₁ O-Ph ist und Y₂ Ala-Isopropylester ist;
oder
Z₁ O ist, Z₂ A ist, Z₃ CHOH ist, R^{S1} H ist, R^{S2} OH ist, Y₁ O-Ph ist und Y₂ Ala-Isopropylester ist;
oder
Z₁ O ist, Z₂ A ist, Z₃ CHOH ist, R^{S1} H ist, R^{S2} OH ist, Y₁ O-Ph ist und Y₂ Ala-Methylester ist;
oder
Z₁ O ist, Z₂ A ist, Z₃ CHOH ist, R^{S1} F ist, R^{S2} F ist, Y₁ O-Ph ist und Y₂ Leu-Methylester ist;
oder
Z₁ O ist, Z₂ A ist, Z₃ CHOH ist, R^{S1} F ist, R^{S2} F ist, Y₁ O-Ph ist und Y₂ Val-Methylester ist;
oder
Z₁ O ist, Z₂ B ist, Z₃ CHOH ist, R^{S1} H ist, R^{S2} H ist, Y₁ O-Ph ist und Y₂ Val-Methylester ist;
oder
Z₁ O ist, Z₂ B ist, Z₃ CHOPOY₁Y₂ ist, R^{S1} H ist, R^{S2} H ist, Y₁ O-Ph ist und Y₂ Val-Methylester ist;
wobei: 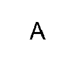 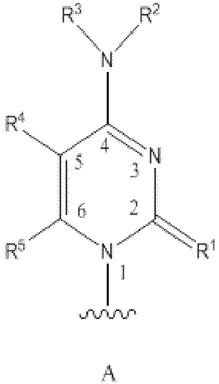 ist,
wobei R¹ = O, R² = R³ = R⁴ = R⁵ = H; 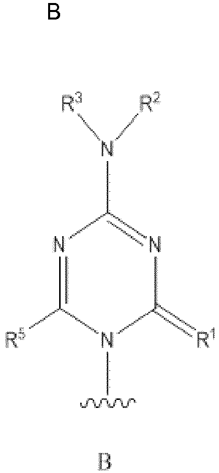 ist,
wobei R¹ = O, R² = R³ = R⁵ = H;
PG eine Schutzgruppe, vorzugsweise ein cyclisches Acetal oder Ketal, ist.

15. Verbindung der Formel I: 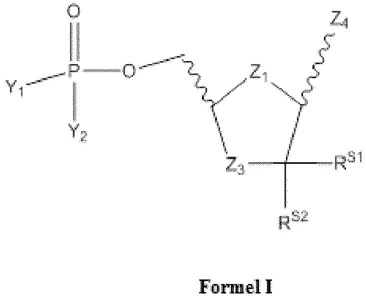 wobei:
Z₁ O ist, Z₃ CH-OH ist, Z₄ E ist, R^{S1} H ist, R^{S2} OH ist, Y₁ O-Ph ist und Y₂ Leu-Methylester ist;
oder
Z₁ O ist, Z₃ CH-OH ist, Z₄ E ist, R^{S1} H ist, R^{S2} OH ist, Y₁ O-Ph ist und Y₂ Val-Methylester ist;
oder
Z₁ O ist, Z₃ CH-OH ist, Z₄ E ist, R^{S1} H ist, R^{S2} OH ist, Y₁ O-Ph ist und Y₂ Ala-Isopropylester ist;
oder
Z₁ O ist, Z₃ CH-OH ist, Z₄ E ist, R^{S1} H ist, R^{S2} OH ist, Y₁ O-Ph ist und Y₂ Ala-Methylester ist;
oder
Z₁ O ist, Z₃ CH-OH ist, Z₄ E ist, R^{S1} F ist, R^{S2} F ist, Y₁ O-Ph ist und Y₂ Leu-Methylester ist;
oder
Z₁ O ist, Z₃ CH-OH ist, Z₄ E ist, R^{S1} F ist, R^{S2} F ist, Y₁ O-Ph ist und Y₂ Val-Methylester ist;
oder
Z₁ O ist, Z₃ CH-OH ist, Z₄ E ist, R^{S1} F ist, R^{S2} F ist, Y₁ O-Ph ist und Y₂ Ala-Isopropylester ist;
oder
Z₁ O ist, Z₃ CH-OH ist, Z₄ F ist, R^{S1} H ist, R^{S2} H ist, Y₁ O-Ph ist und Y₂ Val-Methylester ist;
wobei: 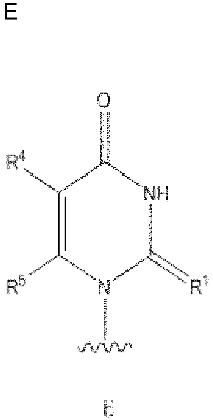 ist,
wobei R¹ = O, R⁴ = R⁵ = H; und 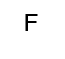 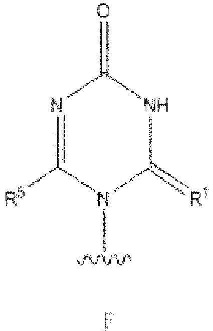 ist,
wobei R¹ = O, R⁵ = H.

## Revendications

1. - Procédé pour la préparation d'un composé de formule I ou un sel pharmaceutiquement acceptable de celui-ci selon la réaction suivante catalysée par un nucléoside désaminase, nucléoside désaminase étant une cytidine désaminase: 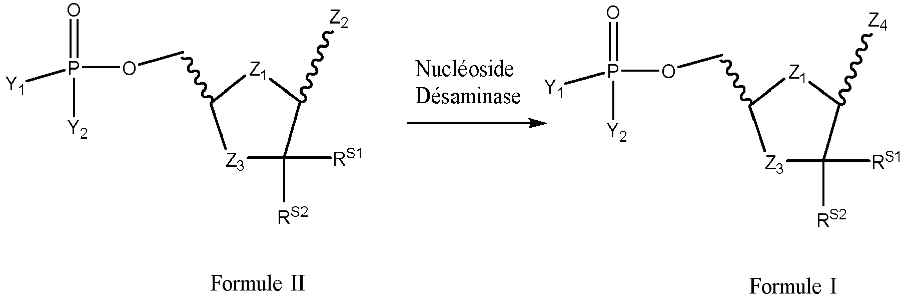 où
Z₁ est O;
Z₂ est sélectionné à partir de: 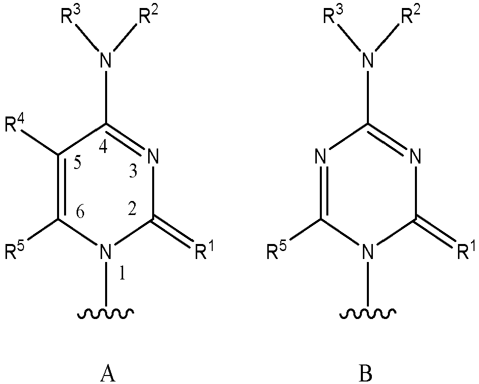
Z₃ est sélectionné, indépendamment de Z₁, à partir de O et C(R^{S3}R^{S4});
Z₄ est sélectionné à partir de: 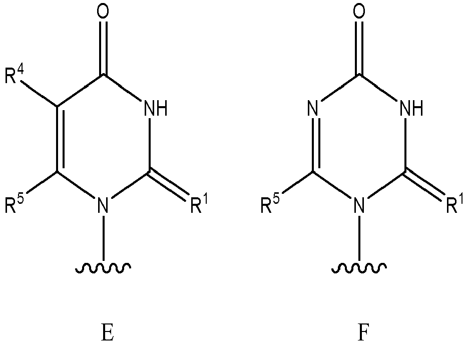 et
R¹ est sélectionné à partir de O, CH₂, alkyle, S et NH;
R² est hydrogène;
R³ est hydrogène;
R⁴ est sélectionné à partir d'hydrogène; OH; NH₂; SH; halogène, de préférence F, CI ou I; méthyle; une chaîne alkyle facultativement substituée; une chaîne alcényle facultativement substituée; une chaîne alcynyle facultativement substituée; trihaloalkyle; OR⁶; NR⁶R⁷; CN; COR⁶; CONR⁶R⁷; CO₂R⁶; C(S)OR⁶; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; SO₂NR⁶R⁷; un aryle facultativement substitué lié à C-5 par une chaîne alkyle, alcényle ou alcynyle facultativement substituée; et un hétérocycle facultativement substitué lié à C-5 par une chaîne alkyle, alcényle ou alcynyle facultativement substituée;
R⁵ est sélectionné à partir d'hydrogène; OH; NH₂; SH; halogène, de préférence F, CI ou I; méthyle; une chaîne alkyle facultativement substituée; une chaîne alcényle facultativement substituée; une chaîne alcynyle facultativement substituée; trihaloalkyle; OR⁶; NR⁶R⁷; CN; COR⁶; CONR⁶R⁷; CO₂R⁶; C(S)OR⁶; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; SO₂NR⁶R⁷; un aryle facultativement substitué lié à C-6 par une chaîne alkyle, alcényle ou alcynyle facultativement substituée; et un hétérocycle facultativement substitué lié à C-6 par une chaîne alkyle, alcényle ou alcynyle facultativement substituée;
R⁶ et R⁷ sont sélectionnés, indépendamment l'un de l'autre, à partir d'hydrogène; une chaîne alkyle facultativement substituée; une chaîne alcényle facultativement substituée; une chaîne alcynyle facultativement substituée; un hétérocycle facultativement substitué; et un aryle facultativement substitué, de préférence phényle ou naphtyle;
R^{S1} est sélectionné, indépendamment de R^{S2}, à partir d'hydrogène; halogène, de préférence F; méthyle; OH; NH₂; SH; N₃; une chaîne alkyle facultativement substituée; une chaîne alcényle facultativement substituée; une chaîne alcynyle facultativement substituée; trihaloalkyle, OR⁶; NR⁶R⁷; CN; COR⁶; CONR⁶R⁷; CO₂R⁶; C(S)OR⁶; OCONR⁶R⁷; OCOR⁶; OCO₂R⁶; OSO₂R⁶; OC(S)OR⁶; O-Cétal; O-Si-alkyle; O-Si-aryle; NHCONR⁶R⁷; NHCOR⁶; NR⁶CO₂R⁷; NHCO₂R⁶; NHC(S)OR⁶; SO₂NR⁶R⁷; un aryle facultativement substitué lié à C-2' par une chaîne alkyle, alcényle ou alcynyle facultativement substituée; et un hétérocycle facultativement substitué lié à C-2' par une chaîne alkyle, alcényle ou alcynyle facultativement substituée;
R^{S2} est sélectionné, indépendamment de R^{S1}, à partir d'hydrogène; halogène, de préférence F; méthyle; OH; NH₂; SH; N₃; une chaîne alkyle facultativement substituée; une chaîne alcényle facultativement substituée; une chaîne alcynyle facultativement substituée; trihaloalkyle; OR⁶; NR⁶R⁷; CN; COR⁶; CONR⁶R⁷; CO₂R⁶; C(S)OR⁶; OCONR⁶R⁷; OCOR⁶; OCO₂R⁶; OSO₂R⁶; OC(S)OR⁶; O-Cétal; O-Si-alkyle; O-Si-aryle; NHCONR⁶R⁷; NHCOR⁶; NR⁶CO₂R⁷; NHCO₂R⁶; NHC(S)OR⁶; SO₂NR⁶R⁷; un aryle facultativement substitué lié à C-2' par une chaîne alkyle, alcényle ou alcynyle facultativement substituée; et un hétérocycle facultativement substitué lié à C-2' par une chaîne alkyle, alcényle ou alcynyle facultativement substituée;
R^{S3} est sélectionné, indépendamment de R^{S4}, à partir d'hydrogène; OH; halogène, de préférence F; méthyle; CN; NH₂; SH; C≡CH; N₃; une chaîne alkyle facultativement substituée; une chaîne alcényle facultativement substituée; une chaîne alcynyle facultativement substituée, un aryle facultativement substitué; un hétérocycle facultativement substitué; OR⁶; OCONR⁶R⁷; OCOR⁶; OCO₂R⁶; OSO₂R⁶; OC(S)OR⁶; O-Cétal; O-Si-alkyle; et O-Si-aryle;
R^{S4} est sélectionné, indépendamment de R^{S3}, à partir d'hydrogène; OH; halogène, de préférence F; méthyle; CN; NH₂; SH; C≡CH; N₃; une chaîne alkyle facultativement substituée; une chaîne alcényle facultativement substituée; une chaîne alcynyle facultativement substituée; un aryle facultativement substitué; un hétérocycle facultativement substitué; OR⁶; OCONR⁶R⁷; OCOR⁶; OCO₂R⁶; OSO₂R⁶; OC(S)OR⁶; O-Cétal; O-Si-alkyle; et O-Si-aryle;
Y₁ est sélectionné, indépendamment de Y₂, à partir de OR⁸; NR⁶R⁷; COR⁶; CONR⁶R⁷; CO₂R⁶; C(S)OR⁶; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; SO₂NR⁶R⁷; une chaîne alkyle facultativement substituée; une chaîne alcényle facultativement substituée; une chaîne alcynyle facultativement substituée; une chaîne cycloalkyle facultativement substituée facultativement liée à P par les atomes O ou N; une chaîne cycloalcényle facultativement substituée facultativement liée à P par les atomes O ou N; une chaîne cycloalcynyle facultativement substituée facultativement liée à P par les atomes O ou N; un aryle facultativement substitué facultativement lié à P par les atomes O ou N; un hétérocycle facultativement substitué facultativement lié à P par les atomes O ou N; un éther d'une chaîne alkyle facultativement substituée; un éther d'une chaîne alcényle facultativement substituée; un éther d'une chaîne alcynyle facultativement substituée; un éther d'un aryle facultativement substitué, de préférence *O*-phényle ou *O*-naphtyle; un éther d'un hétérocycle facultativement substitué; et un acide aminé, de préférence alanine, valine, leucine ou isoleucine, soit sous forme libre ou protégé par un groupe fonctionnel approprié;
Y₂ est sélectionné, indépendamment de Y₁, à partir de OR⁸; NR⁶R⁷; COR⁶; CONR⁶R⁷; CO₂R⁶; C(S)OR⁶; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; SO₂NR⁶R⁷; une chaîne alkyle facultativement substituée; une chaîne alcényle facultativement substituée; une chaîne alcynyle facultativement substituée; une chaîne cycloalkyle facultativement substituée facultativement liée à P par les atomes O ou N; une chaîne cycloalcényle facultativement substituée facultativement liée à P par les atomes O ou N; une chaîne cycloalcynyle facultativement substituée facultativement liée à P par les atomes O ou N; un aryle facultativement substitué facultativement lié à P par les atomes O ou N; un hétérocycle facultativement substitué facultativement lié à P par les atomes O ou N; un éther d'une chaîne alkyle facultativement substituée; un éther d'une chaîne alcényle facultativement substituée; un éther d'une chaîne alcynyle facultativement substituée; un éther d'un aryle facultativement substitué, de préférence *O*-phényle ou *O*-naphtyle; un éther d'un hétérocycle facultativement substitué; et un acide aminé, de préférence alanine, valine, leucine ou isoleucine, soit sous forme libre ou protégé par un groupe fonctionnel approprié;
R⁸ est sélectionné à partir d'une chaîne alkyle facultativement substituée; une chaîne alcényle facultativement substituée; une chaîne alcynyle facultativement substituée, une chaîne cycloalkyle facultativement substituée facultativement liée à P par les atomes O ou N; une chaîne cycloalcényle facultativement substituée facultativement liée à P par les atomes O ou N; une chaîne cycloalcynyle facultativement substituée facultativement liée à P par les atomes O ou N; un aryle facultativement substitué facultativement lié à P par les atomes O ou N; un hétérocycle facultativement substitué facultativement lié à P par les atomes O ou N; aryle, de préférence phényle ou naphtyle;
où quand Z₂ est A, Z₄ est E; et quand Z₂ est B, Z₄ est F.

2. Procédé pour la préparation d'un composé de formule I, selon la revendication 1, où
Z₁ est O;
Z₂ est sélectionné à partir de 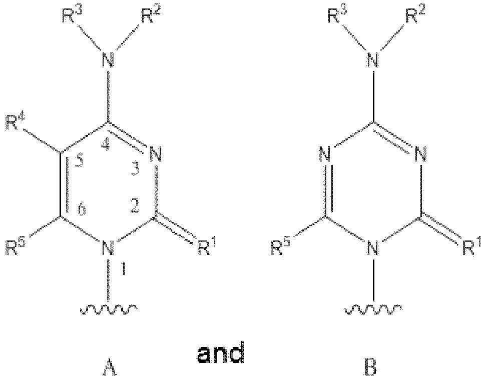
Z₃ est sélectionné, indépendamment de Z₁, à partir de O et C(R^{S3}R^{S4}), plus préférablement C(R^{S3}R^{S4});
Z₄ est sélectionné à partir de: 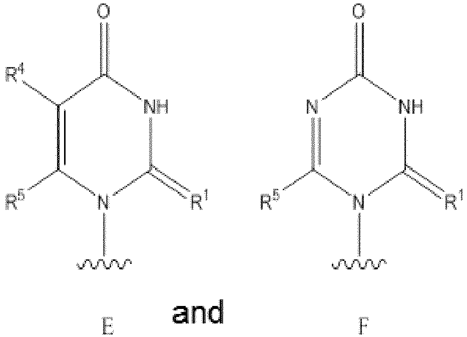
R¹ est O;
R² est H;
R³ est H;
R⁴ est sélectionné à partir de H; OH; halogène, de préférence F, Cl ou I, plus préférablement F; méthyle; trihaloalkyle; OR⁶; COR⁶; CONR⁶R⁷; CO₂R⁶; OCONR⁶R⁷; OCOR⁶; et OCO₂R⁶;
R⁵ est sélectionné à partir de H; OH; halogène, préférablement F, CI ou I, plus préférablement F; méthyle; trihaloalkyle; OR⁶; COR⁶; CONR⁶R⁷; CO₂R⁶; OCONR⁶R⁷; OCOR⁶; et OCO₂R⁶;
R^{S1} est sélectionné, indépendamment de R^{S2}, à partir d'hydrogène; halogène, de préférence F, méthyle; OH; OR⁶; NR⁶R⁷; OCONR⁶R⁷; OCOR⁶; OCO₂R⁶; OSO₂R⁶; OC(S)OR⁶; O-Cétal; O-Si-alkyle; et O-Si-aryle;
R^{S2} est sélectionné, indépendamment de R^{S1}, à partir d'hydrogène; halogène, de préférence F, méthyle; OH; OR⁶; NR⁶R⁷; OCONR⁶R⁷; OCOR⁶; OCO₂R⁶; OSO₂R⁶; OC(S)OR⁶; O-Cétal; O-Si-alkyle; et O-Si-aryle;
R^{S3} est sélectionné, indépendamment de R^{S4}, à partir d'hydrogène; méthyle; OH; OR⁶; OCONR⁶R⁷; OCOR⁶; OCO₂R⁶; OSO₂R⁶; OC(S)OR⁶; O-Cétal; O-Si-alkyle; O-Si-aryle; et halogène, de préférence F;
R^{S4} est sélectionné, indépendamment de R^{S4}, à partir d'hydrogène; méthyle; OH; OR⁶; OCONR⁶R⁷; OCOR⁶; OCO₂R⁶; OSO₂R⁶; OC(S)OR⁶; O-Cétal; O-Si-alkyle; O-Si-aryle; et halogène, de préférence F;
Y₁ est sélectionné, indépendamment de Y₂, à partir de OR⁸; NR⁶R⁷; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; une chaîne alkyle facultativement substituée; une chaîne alcényle facultativement substituée; une chaîne alcynyle facultativement substituée; une chaîne cycloalkyle facultativement substituée facultativement liée à P par les atomes O ou N; une chaîne cycloalcényle facultativement substituée facultativement liée à P par les atomes O ou N; une chaîne cycloalcynyle facultativement substituée facultativement liée à P par les atomes O ou N; un aryle facultativement substitué facultativement lié à P par les atomes O ou N; un hétérocycle facultativement substitué facultativement lié à P par les atomes O ou N; un éther d'une chaîne alkyle facultativement substituée; un éther d'une chaîne alcényle facultativement substituée; un éther d'une chaîne alcynyle facultativement substituée; un éther d'un aryle facultativement substitué, de préférence *O*-phényle ou *O*-naphtyle; Un éther d'un hétérocycle facultativement substitué; et un acide aminé, de préférence alanine, valine, leucine ou isoleucine, soit sous forme libre ou protégé par un groupe fonctionnel approprié;
Y₂ est sélectionné, indépendamment de Y₁, à partir de OR⁸; NR⁶R⁷; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; une chaîne alkyle facultativement substituée; une chaîne alcényle facultativement substituée; une chaîne alcynyle facultativement substituée; une chaîne cycloalkyle facultativement substituée facultativement liée à P par les atomes O ou N; une chaîne cycloalcényle facultativement substituée facultativement liée à P par les atomes O ou N; une chaîne cycloalcynyle facultativement substituée facultativement liée à P par les atomes O ou N; un aryle facultativement substitué facultativement lié à P par les atomes O ou N; un hétérocycle facultativement substitué facultativement lié à P par les atomes O ou N; un éther d'une chaîne alkyle facultativement substituée; un éther d'une chaîne alcényle facultativement substituée; un éther d'une chaîne alcynyle facultativement substituée; un éther d'un aryle facultativement substitué, de préférence *O*-phényle ou *O*-naphtyle; un éther d'un hétérocycle facultativement substitué; et un acide aminé, de préférence alanine, valine, leucine ou isoleucine, soit sous forme libre ou protégé par un groupe fonctionnel approprié;
R⁸ à partir d'une chaîne alkyle facultativement substituée; une chaîne alcényle facultativement substituée; une chaîne alcynyle facultativement substituée; une chaîne cycloalkyle facultativement substituée facultativement liée à P par les atomes O ou N; une chaîne cycloalcényle facultativement substituée facultativement liée à P par les atomes O ou N; une chaîne cycloalcynyle facultativement substituée facultativement liée à P par les atomes O ou N; un aryle facultativement substitué facultativement lié à P par les atomes O ou N; un hétérocycle facultativement substitué facultativement lié à P par les atomes O ou N; et aryle, de préférence phényle et naphtyle ;
où quand Z₂ est A, Z₄ est E; et quand Z₂ est B, Z₄ est F.

3. Procédé pour la préparation d'un composé de formule I, selon la revendication 1, où
Z₁ est O;
Z₂ est sélectionné à partir de 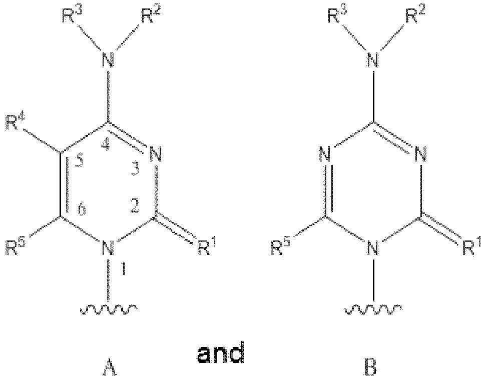
Z₃ est C(R^{S3}R^{S4});
Z₄ est sélectionné à partir de 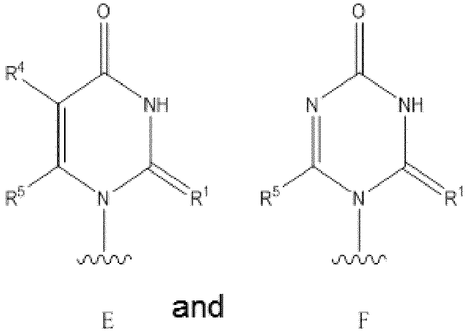
R¹ est O;
R² est H;
R³ est H;
R⁴ est sélectionné à partir de H; OH; halogène, de préférence F; méthyle et trihaloalkyle;
R⁵ est sélectionné à partir de H; OH; halogène; OR⁶; COR⁶; CONR⁶R⁷; CO₂R⁶; OCONR⁶R⁷; OCOR⁶; et OCO₂R⁶;
R^{S1} est sélectionné, indépendamment de R^{S2}, à partir d'hydrogène; halogène, de préférence F; méthyle; OH; OR⁶; OCONR⁶R⁷; OCOR⁶; OSO₂R⁶; O-Cétal; O-Si-alkyle; et O-Si-aryle; et OCO₂R⁶;
R^{S2} est sélectionné, indépendamment de R^{S1}, à partir d'hydrogène; halogène, de préférence F; méthyle; OH; OR⁶; OCONR⁶R⁷; OCOR⁶; OSO₂R⁶; O-Cétal; O-Si-alkyle; et O-Si-aryle; et OCO₂R⁶;
R^{S3} est sélectionné, indépendamment de R^{S4}, à partir d'hydrogène; méthyle; OH; OR⁶; OCONR⁶R⁷; OCOR⁶; OCO₂R⁶; OSO₂R⁶; OC(S)OR⁶; O-Cétal; O-Si-alkyle; O-Si-aryle et halogène, de préférence F;
R^{S4} est sélectionné, indépendamment de R^{S3}, à partir d'hydrogène; méthyle; OH; OR⁶; OCONR⁶R⁷; OCOR⁶; OCO₂R⁶; OSO₂R⁶; OC(S)OR⁶; O-Cétal; O-Si-alkyle; O-Si-aryle; et halogène, de préférence F;
Y₁ est sélectionné, indépendamment de Y₂, à partir de OR⁸; NR⁶R⁷; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; une chaîne alkyle facultativement substituée; une chaîne alcényle facultativement substituée; une chaîne alcynyle facultativement substituée; une chaîne cycloalkyle facultativement substituée facultativement liée à P par les atomes O ou N; une chaîne cycloalcényle facultativement substituée facultativement liée à P par les atomes O ou N; une chaîne cycloalcynyle facultativement substituée facultativement liée à P par les atomes O ou N; un aryle facultativement substitué facultativement lié à P par les atomes O ou N; un hétérocycle facultativement substitué facultativement lié à P par les atomes O ou N; un éther d'une chaîne alkyle facultativement substituée; un éther d'une chaîne alcényle facultativement substituée; un éther d'une chaîne alcynyle facultativement substituée; un éther d'un aryle facultativement substitué, de préférence *O*-phényle ou *O*-naphtyle; un éther d'un hétérocycle facultativement substitué; et un acide aminé, de préférence alanine, valine, leucine ou isoleucine, soit sous forme libre ou protégé par un groupe fonctionnel approprié;
Y₂ est sélectionné, indépendamment de Y₁, à partir de OR⁸; NR⁶R⁷; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; une chaîne alkyle facultativement substituée; une chaîne alcényle facultativement substituée; une chaîne alcynyle facultativement substituée; une chaîne cycloalkyle facultativement substituée facultativement liée à P par les atomes O ou N; une chaîne cycloalcényle facultativement substituée facultativement liée à P par les atomes O ou N; une chaîne cycloalcynyle facultativement substituée facultativement liée à P par les atomes O ou N; un aryle facultativement substitué facultativement lié à P par les atomes O ou N; un hétérocycle facultativement substitué facultativement lié à P par les atomes O ou N; un éther d'une chaîne alkyle facultativement substituée; un éther d'une chaîne alcényle facultativement substituée; Un éther d'une chaîne alcynyle facultativement substituée; un éther d'un aryle facultativement substitué, de préférence *O*-phényle ou *O*-naphtyle; un éther d'un hétérocycle facultativement substitué; et un acide aminé, de préférence alanine, valine, leucine ou isoleucine, soit sous forme libre ou protégé par un groupe fonctionnel approprié;
R⁸ est sélectionné à partir d'une chaîne alkyle facultativement substituée; une chaîne alcényle facultativement substituée; une chaîne alcynyle facultativement substituée; une chaîne cycloalkyle facultativement substituée facultativement liée à P par les atomes O ou N; Une chaîne cycloalcényle facultativement substituée facultativement liée à P par les atomes O ou N; une chaîne cycloalcynyle facultativement substituée facultativement liée à P par les atomes O ou N; un aryle facultativement substitué facultativement lié à P par les atomes O ou N; un hétérocycle facultativement substitué facultativement lié à P par les atomes O ou N; et aryle, de préférence phényle et naphtyle;
où quand Z₂ est A, Z₄ est E; et quand Z₂ est B, Z₄ est F.

4. Procédé pour la préparation d'un composé de formule I, selon la revendication 1, où
Z₁ est O;
Z₂ est sélectionné à partir 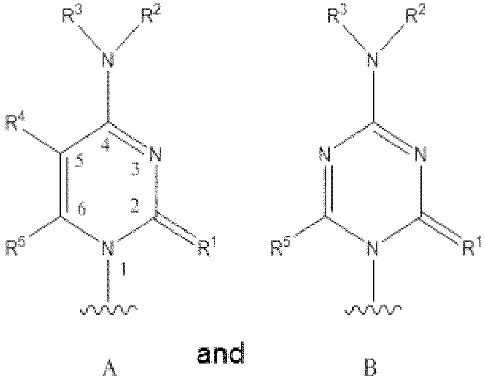
Z₃ est C(R^{S3}R^{S4}) où R^{S3} est H oR OH et où R^{S4} est, indépendamment de R^{S3}, H oR OH;
Z₄ est sélectionné à partir 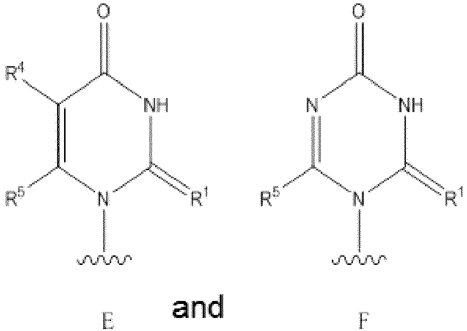
R¹ est O;
R² est H;
R³ est H;
R⁴ est H; méthyle oR halogène, de préférence F;
R⁵ est H;
R^{S1} est sélectionné, indépendamment de R^{S2}, à partir d'hydrogène; halogène, de préférence F; méthyle; et OH;
R^{S2} est sélectionné, indépendamment de R^{S1}, à partir d'hydrogène; halogène, de préférence F; méthyle; et OH;
Y₁ est sélectionné, indépendamment de Y₂, à partir OR⁸; un éther d'un aryle facultativement substitué, de préférence *O*-phényle ou *O*-naphtyle; un éther d'un hétérocycle facultativement substitué; et un acide aminé, de préférence alanine, valine, leucine ou isoleucine, soit sous forme libre ou protégé par un groupe fonctionnel approprié;
Y₂ est sélectionné, indépendamment de Y₁, à partir de OR⁸; NR⁶R⁷; NHCONR⁶R⁷; NHCOR⁶; NR⁶CO₂R⁷; NHCO₂R⁶; NHC(S)OR⁶; une chaîne cycloalkyle facultativement substituée facultativement liée à P par les atomes O ou N; une chaîne cycloalcényle facultativement substituée facultativement liée à P par les atomes O ou N; une chaîne cycloalcynyle facultativement substituée facultativement liée à P par les atomes O ou N; un aryle facultativement substitué facultativement lié à P par les atomes O ou N; un hétérocycle facultativement substitué facultativement lié à P par les atomes O ou N; un éther d'une chaîne alkyle facultativement substituée; un éther d'une chaîne alcényle facultativement substituée; un éther d'une chaîne alcynyle facultativement substituée; un éther d'un aryle facultativement substitué, de préférence *O*-phényle ou *O*-naphtyle; un éther d'un hétérocycle facultativement substitué; et un acide aminé, de préférence alanine, valine, leucine ou isoleucine, soit sous forme libre ou protégé par un groupe fonctionnel approprié;
R⁸ est sélectionné à partir d'une chaîne alkyle facultativement substituée; une chaîne alcényle facultativement substituée; une chaîne alcynyle facultativement substituée; une chaîne cycloalkyle facultativement substituée facultativement liée à P par les atomes O ou N; une chaîne cycloalcényle facultativement substituée facultativement liée à P par les atomes O ou N; une chaîne cycloalcynyle facultativement substituée facultativement liée à P par les atomes O ou N; un aryle facultativement substitué facultativement lié à P par les atomes O ou N; un hétérocycle facultativement substitué facultativement lié à P par les atomes O ou N; et aryle, de préférence phényle et naphtyle;
où quand Z₂ est A, Z₄ est E; et quand Z₂ est B, Z₄ est F.

5. Procédé pour la préparation d'un composé de formule I, selon la revendication 4, où
R⁴ est H;
Y₁ est sélectionné, indépendamment de Y₂, à partir OR⁸; un éther d'un aryle facultativement substitué, de préférence *O*-phényle ou *O*-naphtyle; un éther d'un hétérocycle facultativement substitué; et un acide aminé, de préférence alanine, valine, leucine ou isoleucine, soit sous forme libre ou protégé par un groupe fonctionnel approprié;
Y₂ est sélectionné, indépendamment de Y₁, à partir d'un éther d'un aryle facultativement substitué, de préférence *O*-phényle ou *O*-naphtyle; et un acide aminé, de préférence alanine, valine, leucine ou isoleucine, soit sous forme libre ou protégé par un groupe fonctionnel approprié;

6. Procédé pour la préparation d'un composé de formule I, selon la revendication 4, où
Y₁ est sélectionné, indépendamment de Y₂, à partir d'un éther d'un aryle facultativement substitué, de préférence *O*-phényle; et un acide aminé, de préférence alanine, valine, leucine ou isoleucine, soit sous forme libre ou protégé par un groupe fonctionnel approprié; et
Y₂ est sélectionné, indépendamment de Y₁, à partir d'un éther d'un aryle facultativement substitué, de préférence *O*-phényle; et un acide aminé, de préférence alanine, valine, leucine ou isoleucine, soit sous forme libre ou protégé par un groupe fonctionnel approprié;

7. Procédé, selon n'importe quelles revendications précédentes, où ledit procédé est réalisé à une température variant de 18 à 100 °C.

8. Procédé, selon n'importe quelles revendications précédentes, où le pH moyen varie de 3 à 12.

9. Procédé, selon n'importe quelles revendications précédentes, où la concentration d'un composé de formule I ou d'un sel pharmaceutiquement acceptable varie de 0.1 mM à 500 M.

10. Procédé, selon n'importe quelles revendications précédentes, où la quantité d'enzyme ayant une activité de cytidine désaminase varie de 0.001 à 10000 mg/ml, de préférence de 0.001 à 1000 mg/ml.

11. Procédé, selon n'importe quelles revendications précédentes, où la quantité d'enzyme ayant une activité de cytidine désaminase varie de 0.001 à 10000 AR/micromole de substrat, de préférence de 0.001 à 100 AR/micromole de substrat.

12. Procédé, selon n'importe quelles revendications précédentes, où le milieu de réaction est aqueux facultativement contenant aussi jusqu'à 50%, de préférence jusqu'à 30% et plus préférablement jusqu'à 15% de dissolvent organique approprié.

13. Procédé, selon la revendication 12, où ledit dissolvent organique est sélectionné à partir de méthanol, éthanol, propanol, isopropanol, t-butanol, n-butanol, acétate d'éthyle, acétate d'isopropyle, acétate de butyle, dichlorométhane, toluène, tétrahydrofurane, 2-méthyltetrahydrofurane, acétonitrile, acétone, éther de cyclopentyle et de méthyle, méthyle éthyle cétone, méthyle isobutyle cétone, diméthylamide, diméthylformamide et diméthylsulfoxide.

14. Composé de formule II: 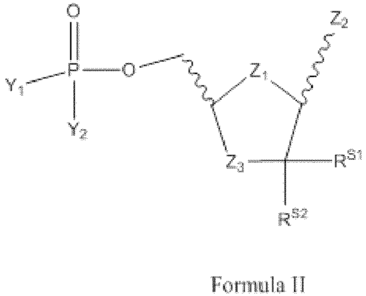 où:
Z₁ est O, Z₂ est A, Z₃ est CH-OPG, R^{S1} est H, R^{S2} est OPG, Y₁ est O-Ph et Y₂ est Leu-ester méthylique;
ou
Z₁ est O, Z₂ est A, Z₃ est CHOH, R^{S1} est H, R^{S2} est OH, Y₁ est O-Ph et Y₂ est Leu-ester méthylique;
ou
Z₁ est O, Z₂ est A, Z₃ est CH-OPG, R^{S1} est H, R^{S2} est OPG, Y₁ est O-Ph et Y₂ est Val- ester méthylique;
ou
Z₁ est O, Z₂ est A, Z₃ est CHOH, R^{S1} est H, R^{S2} est OH, Y₁ est O-Ph et Y₂ est Val- ester méthylique;
ou
Z₁ est O, Z₂ est A, Z₃ est CH-OPG, R^{S1} est H, R^{S2} est OPG, Y₁ est O-Ph et Y₂ est Ala-ester de isopropyle;
ou
Z₁ est O, Z₂ est A, Z₃ est CHOH, R^{S1} est H, R^{S2} est OH, Y₁ est O-Ph et Y₂ est Ala-ester de isopropyle;
ou
Z₁ est O, Z₂ est A, Z₃ est CHOH, R^{S1} est H, R^{S2} est OH, Y₁ est O-Ph et Y₂ est Ala-méthyl ester méthylique;
ou
Z₁ est O, Z₂ est A, Z₃ est CHOH, R^{S1} est F, R^{S2} est F, Y₁ est O-Ph et Y₂ est Leu- ester méthylique;
ou
Z₁ est O, Z₂ est A, Z₃ est CHOH, R^{S1} est F, R^{S2} est F, Y₁ est O-Ph et Y₂ est Val- ester méthylique;
ou
Z₁ est O, Z₂ est B, Z₃ est CHOH, R^{S1} est H, R^{S2} est H, Y₁ est O-Ph et Y₂ est Val- ester méthylique;
ou
Z₁ est O, Z₂ est B, Z₃ est CHOPOY₁Y₂, R^{S1} est H, R^{S2} est H, Y₁ est O-Ph et Y₂ est Val- ester méthylique;
où:
A est 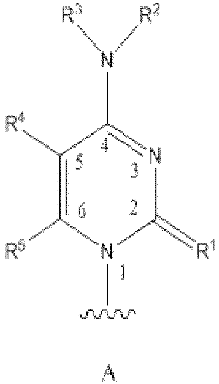
étant R¹ = O, R² = R³ = R⁴ = R⁵ = H;
B est 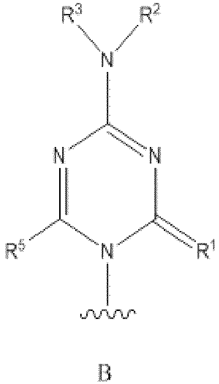
étant R¹ = O, R² = R³ = R⁵ = H;
PG est un groupe protecteur, de préférence un acétal ou cétal cyclique.

15. Composé de formule I: 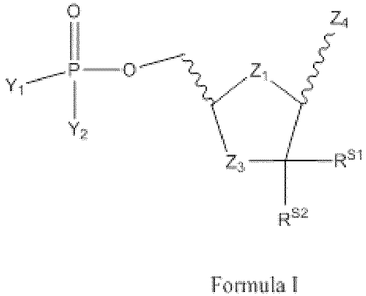 où:
Z₁ est O, Z₃ est CH-OH, Z₄ est E, R^{S1} est H, R^{S2} est OH, Y₁ est O-Ph et Y₂ est Leu- ester méthylique;
ou
Z₁ est O, Z₃ est CH-OH, Z₄ est E, R^{S1} est H, R^{S2} est OH, Y₁ est O-Ph et Y₂ est Val- ester méthylique;
ou
Z₁ est O, Z₃ est CH-OH, Z₄ est E, R^{S1} est H, R^{S2} est OH, Y₁ est O-Ph et Y₂ est Ala-ester de isopropyle;
ou
Z₁ est O, Z₃ est CH-OH, Z₄ est E, R^{S1} est H, R^{S2} est OH, Y₁ est O-Ph et Y₂ est Ala- ester méthylique;
ou
Z₁ est O, Z₃ est CH-OH, Z₄ est E, R^{S1} est F, R^{S2} est F, Y₁ est O-Ph et Y₂ est Leu- ester méthylique;
ou
Z₁ est O, Z₃ est CH-OH, Z₄ est E, R^{S1} est F, R^{S2} est F, Y₁ est O-Ph et Y₂ est Val- ester méthylique;
ou
Z₁ est O, Z₃ est CH-OH, Z₄ est E, R^{S1} est F, R^{S2} est F, Y₁ est O-Ph et Y₂ est Ala-ester de isopropyle;
ou
Z₁ est O, Z₃ est CH-OH, Z₄ est F, R^{S1} est H, R^{S2} est H, Y₁ est O-Ph et Y₂ est Val- ester méthylique;
où:
E est 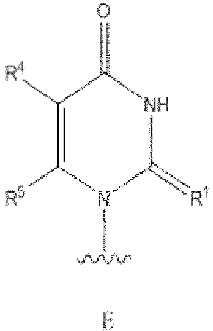
étant R¹ = O, R⁴ = R⁵ = H; et
F est 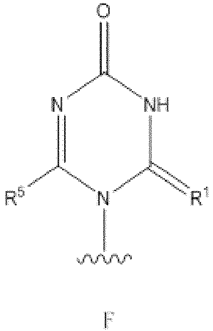
étant R¹ = O, R⁵ = H.
